(19) Europäisches Patentamt
European Patent Office
Office européen des brevets

(11) Publication number: **0 641 792 A1**

# (12) EUROPEAN PATENT APPLICATION

(21) Application number: **94113804.2**

(22) Date of filing: **02.09.94**

(51) Int. Cl.[6]: **C07D 339/04**, C07D 495/04, A61K 31/385

(30) Priority: **02.09.93 JP 218482/93**

(43) Date of publication of application: **08.03.95 Bulletin 95/10**

(84) Designated Contracting States: **BE CH DE ES FR GB IT LI NL SE**

(71) Applicant: **MITSUI TOATSU CHEMICALS, Inc.**
**2-5 Kasumigaseki 3-chome**
**Chiyoda-Ku**
**Tokyo 100 (JP)**

(72) Inventor: **Mita, Naruyoshi**
**2142, Togo**
**Mobara-shi,**
**Chiba-ken (JP)**
Inventor: **Ando, Tomoyuki**
**2142, Togo**
**Mobara-shi,**
**Chiba-ken (JP)**
Inventor: **Kanematsu, Akihito**
**2142, Togo**
**Mobara-shi,**
**Chiba-ken (JP)**
Inventor: **Sakai, Kazuya**
**2142, Togo**
**Mobara-shi,**
**Chiba-ken (JP)**
Inventor: **Mori, Mitsuhiro**
**2142, Togo**
**Mobara-shi,**
**Chiba-ken (JP)**
Inventor: **Sugawara, Tatsuo**
**2142, Togo**
**Mobara-shi,**
**Chiba-ken (JP)**
Inventor: **Yuasa, Shu**
**2141-640-41, Togo**
**Mobara-shi,**
**Chiba-ken (JP)**
Inventor: **Kawai, Kohichi**
**2142, Togo**
**Mobara-shi,**
**Chiba-ken (JP)**

(74) Representative: **Zumstein, Fritz, Dr. et al**
**Dr. F. Zumstein**
**Dipl.-Ing. F. Klingseisen**
**Bräuhausstrasse 4**
**D-80331 München (DE)**

## (54) 1,2-Dithiole derivatives and therapeutic agents containing them.

(57) Novel 1,2-dithiole-3-thione derivatives represented by general formulas (1) and (11); processes for production thereof; and therapeutic or preventive agents for diseases associated with peroxylipid, each containing said derivative as the active ingredient.

EP 0 641 792 A1

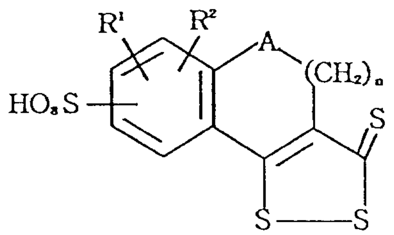

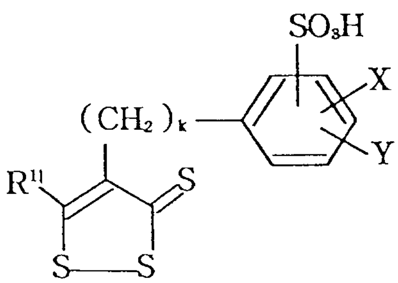

(11)

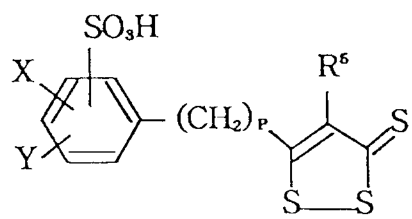

## Background of the Invention

### Field of the Invention

The present invention relates to novel 1,2-dithiole-3-thione derivatives; processes for production thereof; and therapeutic or preventive agents for diseases associated with peroxylipid, each containing said derivative as the active ingredient.

### Description of the Related Art

It has recently been made clear that peroxylipid has close connection with various diseases. These diseases include, for example, arteriosclerosis, lung diseases, digestive diseases, hepatic diseases, heart diseases, cerebrovascular disturbance, inflammation, autoimmune diseases, shock, disseminated intravascular coagulation, dermal diseases, ocular diseases, diabetes, cancer, renal diseases, iron hypersensitivity, pancreatitis, and toxicities of antibiotics and anticancer drugs. The manifestation and aggravation of said diseases is thought to be associated with the lipid peroxidation by active oxygens or free radicals.

With respect to heart diseases, ischemic heart diseases such as myocardial infarction and the like are on the rapid rise in recent years. For the therapy thereof, reperfusion therapy has come to be used widely, and favorable results have been obtained. Meanwhile, it is reported that even when the reperfusion therapy enables the restart of blood flow, the therapy invites new injuries, i.e. reperfusion injuries such as malignant arrhythmia (e.g. ventricular fibrillation), hemorrhagic infarction, no-reflow phenomenon and the like. These reperfusion injuries are thought to be associated with active oxygens and peroxylipid. It is also thought that the reperfusion injuries arise in heart operation or organ transplant, and attention is being paid to the injuries.

Therapeutic agents for ischemic heart diseases, having a 1,2-dithiole-3-thione skeleton are described in Japanese Patent Application Kokai (Laid-Open) No. 301868/1993. The agents have had a problem in water solubility when used as an injection.

The object of the present invention is to provide a novel drug which is capable of inhibiting the formation of peroxylipid and accordingly is useful for the therapy or prevention of diseases associated with peroxylipid and which is further highly water-soluble and accordingly can be administered intravenously when said diseases need urgent treatments.

## Summary of the Invention

The present inventors made an extensive study with an aim of developing a drug effective for the therapy or prevention of the above-mentioned diseases and, as a result, found out that the novel compounds or their salts of the present invention have an excellent effect and, when used as an injection, sufficient water solubility and are useful as a pharmaceutical agent. The finding has led to the completion of the present invention.

The first aspect of the present invention lies in a compound represented by general formula (1)

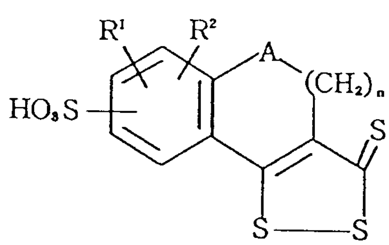

(1)

(wherein A is a methylene group or an oxygen atom; $R^1$ and $R^2$ are independently a hydrogen atom, a hydroxy group, a halogen atom, a lower alkyl group or a lower alkoxy group; and n is an integer of 0-3 when A is a methylene group, and an integer of 1-3 when A is an oxygen atom), or a salt thereof; a therapeutic or preventive agent for diseases associated with peroxylipid, which contains said compound or salt as an active ingredient; and a process for producing said compound.

Description is made on the present compound represented by general formula (1).

The halogen atom can be exemplified by fluorine atom, chlorine atom, bromine atom and iodine atom.

The lower alkyl group is typically an alkyl group of 1-4 carbon atoms, etc. and can be exemplified by methyl group, ethyl group, n-propyl group, isopropyl group, n-butyl group, isobutyl group, sec-butyl group and tert-butyl group.

The lower alkoxy group is typically an alkoxy group of 1-4 carbon atoms and can be exemplified by methoxy group, ethoxy group, n-propoxy group, isopropoxy group, allyloxy group, n-butoxy group, isobutoxy group, sec-butoxy group and tert-butoxy group.

The salt of the compound of general formula (1) can be any pharmacologically acceptable salt and can be exemplified by inorganic (e.g. lithium, sodium, potassium, calcium and aluminum) salts and salts with organic amines (e.g. ammonia, trimethylamine, triethylamine, piperidine and morpholine) or with amino acids.

Specific examples of the compound of general formula (1) are shown below in Tables 1-12. Needless to say, the compound of general formula (1) is not restricted to those shown therein.

Table-1

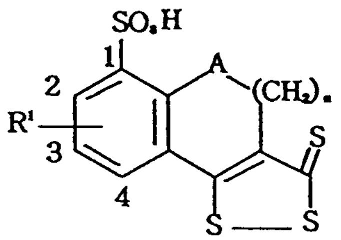

| Compound | A | n | $R^1$ |
|---|---|---|---|
| 1 | $CH_2$ | 0 | H |
| 2 | $CH_2$ | 0 | 2-OH |
| 3 | $CH_2$ | 0 | 3-OH |
| 4 | $CH_2$ | 0 | 4-OH |
| 5 | $CH_2$ | 0 | 2-F |
| 6 | $CH_2$ | 0 | 3-F |
| 7 | $CH_2$ | 0 | 4-F |
| 8 | $CH_2$ | 0 | 2-Cl |
| 9 | $CH_2$ | 0 | 3-Cl |
| 10 | $CH_2$ | 0 | 4-Cl |
| 11 | $CH_2$ | 0 | 2-Br |
| 12 | $CH_2$ | 0 | 3-Br |
| 13 | $CH_2$ | 0 | 4-Br |
| 14 | $CH_2$ | 0 | 2-I |

| | | | |
|---|---|---|---|
| 15 | $CH_2$ | 0 | 3-I |
| 16 | $CH_2$ | 0 | 4-I |
| 17 | $CH_2$ | 0 | $2-CH_3$ |
| 18 | $CH_2$ | 0 | $3-CH_3$ |
| 19 | $CH_2$ | 0 | $4-CH_3$ |
| 20 | $CH_2$ | 0 | $2-CH_3CH_2$ |
| 21 | $CH_2$ | 0 | $3-CH_3CH_2$ |
| 22 | $CH_2$ | 0 | $4-CH_3CH_2$ |
| 23 | $CH_2$ | 0 | $2-CH_3CH_2CH_2$ |
| 24 | $CH_2$ | 0 | $3-CH_3CH_2CH_2$ |
| 25 | $CH_2$ | 0 | $4-CH_3CH_2CH_2$ |
| 26 | $CH_2$ | 0 | $2-(CH_3)_2CH$ |
| 27 | $CH_2$ | 0 | $3-(CH_3)_2CH$ |
| 28 | $CH_2$ | 0 | $4-(CH_3)_2CH$ |
| 29 | $CH_2$ | 0 | $2-CH_3(CH_2)_2CH_2$ |
| 30 | $CH_2$ | 0 | $3-CH_3(CH_2)_2CH_2$ |
| 31 | $CH_2$ | 0 | $4-CH_3(CH_2)_2CH_2$ |
| 32 | $CH_2$ | 0 | $2-(CH_3)_2CHCH_2$ |
| 33 | $CH_2$ | 0 | $3-(CH_3)_2CHCH_2$ |
| 34 | $CH_2$ | 0 | $4-(CH_3)_2CHCH_2$ |
| 35 | $CH_2$ | 0 | $2-CH_3CH_2CH(CH_3)$ |
| 36 | $CH_2$ | 0 | $3-CH_3CH_2CH(CH_3)$ |
| 37 | $CH_2$ | 0 | $4-CH_3CH_2CH(CH_3)$ |
| 38 | $CH_2$ | 0 | $2-(CH_3)_3C$ |
| 39 | $CH_2$ | 0 | $3-(CH_3)_3C$ |
| 40 | $CH_2$ | 0 | $4-(CH_3)_3C$ |
| 41 | $CH_2$ | 0 | $2-CH_3O$ |
| 42 | $CH_2$. | 0 | $3-CH_3O$ |
| 43 | $CH_2$ | 0 | $4-CH_3O$ |

| | | | |
|---|---|---|---|
| 44 | $CH_2$ | 0 | 2 − $CH_3CH_2O$ |
| 45 | $CH_2$ | 0 | 3 − $CH_3CH_2O$ |
| 46 | $CH_2$ | 0 | 4 − $CH_3CH_2O$ |
| 47 | $CH_2$ | 0 | 2 − $CH_3CH_2CH_2O$ |
| 48 | $CH_2$ | 0 | 3 − $CH_3CH_2CH_2O$ |
| 49 | $CH_2$ | 0 | 4 − $CH_3CH_2CH_2O$ |
| 50 | $CH_2$ | 0 | 2 − $(CH_3)_2CHO$ |
| 51 | $CH_2$ | 0 | 3 − $(CH_3)_2CHO$ |
| 52 | $CH_2$ | 0 | 4 − $(CH_3)_2CHO$ |
| 53 | $CH_2$ | 0 | 2 − $CH_3(CH_2)_2CH_2O$ |
| 54 | $CH_2$ | 0 | 3 − $CH_3(CH_2)_2CH_2O$ |
| 55 | $CH_2$ | 0 | 4 − $CH_3(CH_2)_2CH_2O$ |
| 56 | $CH_2$ | 0 | 2 − $(CH_3)_2CHCH_2O$ |
| 57 | $CH_2$ | 0 | 3 − $(CH_3)_2CHCH_2O$ |
| 58 | $CH_2$ | 0 | 4 − $(CH_3)_2CHCH_2O$ |
| 59 | $CH_2$ | 0 | 2 − $CH_3CH_2CH(CH_3)O$ |
| 60 | $CH_2$ | 0 | 3 − $CH_3CH_2CH(CH_3)O$ |
| 61 | $CH_2$ | 0 | 4 − $CH_3CH_2CH(CH_3)O$ |
| 62 | $CH_2$ | 0 | 2 − $(CH_3)_3CO$ |
| 63 | $CH_2$ | 0 | 3 − $(CH_3)_3CO$ |
| 64 | $CH_2$ | 0 | 4 − $(CH_3)_3CO$ |
| 65 | $CH_2$ | 1 | H |
| 66 | $CH_2$ | 1 | 2 − OH |
| 67 | $CH_2$ | 1 | 3 − OH |
| 68 | $CH_2$ | 1 | 4 − OH |
| 69 | $CH_2$ | 1 | 2 − F |
| 70 | $CH_2$ | 1 | 3 − F |
| 71 | $CH_2$ | 1 | 4 − F |
| 72 | $CH_2$ | 1 | 2 − Cl |

| | | | |
|---|---|---|---|
| 73 | $CH_2$ | 1 | 3-Cl |
| 74 | $CH_2$ | 1 | 4-Cl |
| 75 | $CH_2$ | 1 | 2-Br |
| 76 | $CH_2$ | 1 | 3-Br |
| 77 | $CH_2$ | 1 | 4-Br |
| 78 | $CH_2$ | 1 | 2-I |
| 79 | $CH_2$ | 1 | 3-I |
| 80 | $CH_2$ | 1 | 4-I |
| 81 | $CH_2$ | 1 | $2-CH_3$ |
| 82 | $CH_2$ | 1 | $3-CH_3$ |
| 83 | $CH_2$ | 1 | $4-CH_3$ |
| 84 | $CH_2$ | 1 | $2-CH_3CH_2$ |
| 85 | $CH_2$ | 1 | $3-CH_3CH_2$ |
| 86 | $CH_2$ | 1 | $4-CH_3CH_2$ |
| 87 | $CH_2$ | 1 | $2-CH_3CH_2CH_2$ |
| 88 | $CH_2$ | 1 | $3-CH_3CH_2CH_2$ |
| 89 | $CH_2$ | 1 | $4-CH_3CH_2CH_2$ |
| 90 | $CH_2$ | 1 | $2-(CH_3)_2CH$ |
| 91 | $CH_2$ | 1 | $3-(CH_3)_2CH$ |
| 92 | $CH_2$ | 1 | $4-(CH_3)_2CH$ |
| 93 | $CH_2$ | 1 | $2-CH_3(CH_2)_2CH_2$ |
| 94 | $CH_2$ | 1 | $3-CH_3(CH_2)_2CH_2$ |
| 95 | $CH_2$ | 1 | $4-CH_3(CH_2)_2CH_2$ |
| 96 | $CH_2$ | 1 | $2-(CH_3)_2CHCH_2$ |
| 97 | $CH_2$ | 1 | $3-(CH_3)_2CHCH_2$ |
| 98 | $CH_2$ | 1 | $4-(CH_3)_2CHCH_2$ |
| 99 | $CH_2$ | 1 | $2-CH_3CH_2CH(CH_3)$ |
| 100 | $CH_2$ | 1 | $3-CH_3CH_2CH(CH_3)$ |
| 101 | $CH_2$ | 1 | $4-CH_3CH_2CH(CH_3)$ |

| | | | |
|---|---|---|---|
| 102 | $CH_2$ | 1 | 2-$(CH_3)_3C$ |
| 103 | $CH_2$ | 1 | 3-$(CH_3)_3C$ |
| 104 | $CH_2$ | 1 | 4-$(CH_3)_3C$ |
| 105 | $CH_2$ | 1 | 2-$CH_3O$ |
| 106 | $CH_2$ | 1 | 3-$CH_3O$ |
| 107 | $CH_2$ | 1 | 4-$CH_3O$ |
| 108 | $CH_2$ | 1 | 2-$CH_3CH_2O$ |
| 109 | $CH_2$ | 1 | 3-$CH_3CH_2O$ |
| 110 | $CH_2$ | 1 | 4-$CH_3CH_2O$ |
| 111 | $CH_2$ | 1 | 2-$CH_3CH_2CH_2O$ |
| 112 | $CH_2$ | 1 | 3-$CH_3CH_2CH_2O$ |
| 113 | $CH_2$ | 1 | 4-$CH_3CH_2CH_2O$ |
| 114 | $CH_2$ | 1 | 2-$(CH_3)_2CHO$ |
| 115 | $CH_2$ | 1 | 3-$(CH_3)_2CHO$ |
| 116 | $CH_2$ | 1 | 4-$(CH_3)_2CHO$ |
| 117 | $CH_2$ | 1 | 2-$CH_3(CH_2)_2CH_2O$ |
| 118 | $CH_2$ | 1 | 3-$CH_3(CH_2)_2CH_2O$ |
| 119 | $CH_2$ | 1 | 4-$CH_3(CH_2)_2CH_2O$ |
| 120 | $CH_2$ | 1 | 2-$(CH_3)_2CHCH_2O$ |
| 121 | $CH_2$ | 1 | 3-$(CH_3)_2CHCH_2O$ |
| 122 | $CH_2$ | 1 | 4-$(CH_3)_2CHCH_2O$ |
| 123 | $CH_2$ | 1 | 2-$CH_3CH_2CH(CH_3)O$ |
| 124 | $CH_2$ | 1 | 3-$CH_3CH_2CH(CH_3)O$ |
| 125 | $CH_2$ | 1 | 4-$CH_3CH_2CH(CH_3)O$ |
| 126 | $CH_2$ | 1 | 2-$(CH_3)_3CO$ |
| 127 | $CH_2$ | 1 | 3-$(CH_3)_3CO$ |
| 128 | $CH_2$ | 1 | 4-$(CH_3)_3CO$ |
| 129 | $CH_2$ | 2 | H |
| 130 | $CH_2$ | 2 | 2-OH |

| | | | |
|---|---|---|---|
| 131 | $CH_2$ | 2 | 3-OH |
| 132 | $CH_2$ | 2 | 4-OH |
| 133 | $CH_2$ | 2 | 2-F |
| 134 | $CH_2$ | 2 | 3-F |
| 135 | $CH_2$ | 2 | 4-F |
| 136 | $CH_2$ | 2 | 2-Cl |
| 137 | $CH_2$ | 2 | 3-Cl |
| 138 | $CH_2$ | 2 | 4-Cl |
| 139 | $CH_2$ | 2 | 2-Br |
| 140 | $CH_2$ | 2 | 3-Br |
| 141 | $CH_2$ | 2 | 4-Br |
| 142 | $CH_2$ | 2 | 2-I |
| 143 | $CH_2$ | 2 | 3-I |
| 144 | $CH_2$ | 2 | 4-I |
| 145 | $CH_2$ | 2 | $2-CH_3$ |
| 146 | $CH_2$ | 2 | $3-CH_3$ |
| 147 | $CH_2$ | 2 | $4-CH_3$ |
| 148 | $CH_2$ | 2 | $2-CH_3CH_2$ |
| 149 | $CH_2$ | 2 | $3-CH_3CH_2$ |
| 150 | $CH_2$ | 2 | $4-CH_3CH_2$ |
| 151 | $CH_2$ | 2 | $2-CH_3CH_2CH_2$ |
| 152 | $CH_2$ | 2 | $3-CH_3CH_2CH_2$ |
| 153 | $CH_2$ | 2 | $4-CH_3CH_2CH_2$ |
| 154 | $CH_2$ | 2 | $2-(CH_3)_2CH$ |
| 155 | $CH_2$ | 2 | $3-(CH_3)_2CH$ |
| 156 | $CH_2$ | 2 | $4-(CH_3)_2CH$ |
| 157 | $CH_2$ | 2 | $2-CH_3(CH_2)_2CH_2$ |
| 158 | $CH_2$ | 2 | $3-CH_3(CH_2)_2CH_2$ |
| 159 | $CH_2$ | 2 | $4-CH_3(CH_2)_2CH_2$ |

| | | | |
|---|---|---|---|
| 160 | $CH_2$ | 2 | 2-$(CH_3)_2CHCH_2$ |
| 161 | $CH_2$ | 2 | 3-$(CH_3)_2CHCH_2$ |
| 162 | $CH_2$ | 2 | 4-$(CH_3)_2CHCH_2$ |
| 163 | $CH_2$ | 2 | 2-$CH_3CH_2CH(CH_3)$ |
| 164 | $CH_2$ | 2 | 3-$CH_3CH_2CH(CH_3)$ |
| 165 | $CH_2$ | 2 | 4-$CH_3CH_2CH(CH_3)$ |
| 166 | $CH_2$ | 2 | 2-$(CH_3)_3C$ |
| 167 | $CH_2$ | 2 | 3-$(CH_3)_3C$ |
| 168 | $CH_2$ | 2 | 4-$(CH_3)_3C$ |
| 169 | $CH_2$ | 2 | 2-$CH_3O$ |
| 170 | $CH_2$ | 2 | 3-$CH_3O$ |
| 171 | $CH_2$ | 2 | 4-$CH_3O$ |
| 172 | $CH_2$ | 2 | 2-$CH_3CH_2O$ |
| 173 | $CH_2$ | 2 | 3-$CH_3CH_2O$ |
| 174 | $CH_2$ | 2 | 4-$CH_3CH_2O$ |
| 175 | $CH_2$ | 2 | 2-$CH_3CH_2CH_2O$ |
| 176 | $CH_2$ | 2 | 3-$CH_3CH_2CH_2O$ |
| 177 | $CH_2$ | 2 | 4-$CH_3CH_2CH_2O$ |
| 178 | $CH_2$ | 2 | 2-$(CH_3)_2CHO$ |
| 179 | $CH_2$ | 2 | 3-$(CH_3)_2CHO$ |
| 180 | $CH_2$ | 2 | 4-$(CH_3)_2CHO$ |
| 181 | $CH_2$ | 2 | 2-$CH_3(CH_2)_2CH_2O$ |
| 182 | $CH_2$ | 2 | 3-$CH_3(CH_2)_2CH_2O$ |
| 183 | $CH_2$ | 2 | 4-$CH_3(CH_2)_2CH_2O$ |
| 184 | $CH_2$ | 2 | 2-$(CH_3)_2CHCH_2O$ |
| 185 | $CH_2$ | 2 | 3-$(CH_3)_2CHCH_2O$ |
| 186 | $CH_2$ | 2 | 4-$(CH_3)_2CHCH_2O$ |
| 187 | $CH_2$ | 2 | 2-$CH_3CH_2CH(CH_3)O$ |
| 188 | $CH_2$ | 2 | 3-$CH_3CH_2CH(CH_3)O$ |

| | | | |
|---|---|---|---|
| 189 | $CH_2$ | 2 | $4-CH_3CH_2CH(CH_3)O$ |
| 190 | $CH_2$ | 2 | $2-(CH_3)_3CO$ |
| 191 | $CH_2$ | 2 | $3-(CH_3)_3CO$ |
| 192 | $CH_2$ | 2 | $4-(CH_3)_3CO$ |
| 193 | $CH_2$ | 3 | H |
| 194 | $CH_2$ | 3 | 2−OH |
| 195 | $CH_2$ | 3 | 3−OH |
| 196 | $CH_2$ | 3 | 4−OH |
| 197 | $CH_2$ | 3 | 2−F |
| 198 | $CH_2$ | 3 | 3−F |
| 199 | $CH_2$ | 3 | 4−F |
| 200 | $CH_2$ | 3 | 2−Cl |
| 201 | $CH_2$ | 3 | 3−Cl |
| 202 | $CH_2$ | 3 | 4−Cl |
| 203 | $CH_2$ | 3 | 2−Br |
| 204 | $CH_2$ | 3 | 3−Br |
| 205 | $CH_2$ | 3 | 4−Br |
| 206 | $CH_2$ | 3 | 2−1 |
| 207 | $CH_2$ | 3 | 3−I |
| 208 | $CH_2$ | 3 | 4−I |
| 209 | $CH_2$ | 3 | $2-CH_3$ |
| 210 | $CH_2$ | 3 | $3-CH_3$ |
| 211 | $CH_2$ | 3 | $4-CH_3$ |
| 212 | $CH_2$ | 3 | $2-CH_3CH_2$ |
| 213 | $CH_2$ | 3 | $3-CH_3CH_2$ |
| 214 | $CH_2$ | 3 | $4-CH_3CH_2$ |
| 215 | $CH_2$ | 3 | $2-CH_3CH_2CH_2$ |
| 216 | $CH_2$ | 3 | $3-CH_3CH_2CH_2$ |
| 217 | $CH_2$ | 3 | $4-CH_3CH_2CH_2$ |

| | | | |
|---|---|---|---|
| 218 | $CH_2$ | 3 | 2-$(CH_3)_2CH$ |
| 219 | $CH_2$ | 3 | 3-$(CH_3)_2CH$ |
| 220 | $CH_2$ | 3 | 4-$(CH_3)_2CH$ |
| 221 | $CH_2$ | 3 | 2-$CH_3(CH_2)_2CH_2$ |
| 222 | $CH_2$ | 3 | 3-$CH_3(CH_2)_2CH_2$ |
| 223 | $CH_2$ | 3 | 4-$CH_3(CH_2)_2CH_2$ |
| 224 | $CH_2$ | 3 | 2-$(CH_3)_2CHCH_2$ |
| 225 | $CH_2$ | 3 | 3-$(CH_3)_2CHCH_2$ |
| 226 | $CH_2$ | 3 | 4-$(CH_3)_2CHCH_2$ |
| 227 | $CH_2$ | 3 | 2-$CH_3CH_2CH(CH_3)$ |
| 228 | $CH_2$ | 3 | 3-$CH_3CH_2CH(CH_3)$ |
| 229 | $CH_2$ | 3 | 4-$CH_3CH_2CH(CH_3)$ |
| 230 | $CH_2$ | 3 | 2-$(CH_3)_3C$ |
| 231 | $CH_2$ | 3 | 3-$(CH_3)_3C$ |
| 232 | $CH_2$ | 3 | 4-$(CH_3)_3C$ |
| 233 | $CH_2$ | 3 | 2-$CH_3O$ |
| 234 | $CH_2$ | 3 | 3-$CH_3O$ |
| 235 | $CH_2$ | 3 | 4-$CH_3O$ |
| 236 | $CH_2$ | 3 | 2-$CH_3CH_2O$ |
| 237 | $CH_2$ | 3 | 3-$CH_3CH_2O$ |
| 238 | $CH_2$ | 3 | 4-$CH_3CH_2O$ |
| 239 | $CH_2$ | 3 | 2-$CH_3CH_2CH_2O$ |
| 240 | $CH_2$ | 3 | 3-$CH_3CH_2CH_2O$ |
| 241 | $CH_2$ | 3 | 4-$CH_3CH_2CH_2O$ |
| 242 | $CH_2$ | 3 | 2-$(CH_3)_2CHO$ |
| 243 | $CH_2$ | 3 | 3-$(CH_3)_2CHO$ |
| 244 | $CH_2$ | 3 | 4-$(CH_3)_2CHO$ |
| 245 | $CH_2$ | 3 | 2-$CH_3(CH_2)_2CH_2O$ |
| 246 | $CH_2$ | 3 | 3-$CH_3(CH_2)_2CH_2O$ |

| | | | |
|---|---|---|---|
| 247 | $CH_2$ | 3 | 4-$CH_3(CH_2)_2CH_2O$ |
| 248 | $CH_2$ | 3 | 2-$(CH_3)_2CHCH_2O$ |
| 249 | $CH_2$ | 3 | 3-$(CH_3)_2CHCH_2O$ |
| 250 | $CH_2$ | 3 | 4-$(CH_3)_2CHCH_2O$ |
| 251 | $CH_2$ | 3 | 2-$CH_3CH_2CH(CH_3)O$ |
| 252 | $CH_2$ | 3 | 3-$CH_3CH_2CH(CH_3)O$ |
| 253 | $CH_2$ | 3 | 4-$CH_3CH_2CH(CH_3)O$ |
| 254 | $CH_2$ | 3 | 2-$(CH_3)_3CO$ |
| 255 | $CH_2$ | 3 | 3-$(CH_3)_3CO$ |
| 256 | $CH_2$ | 3 | 4-$(CH_3)_3CO$ |

Table-2

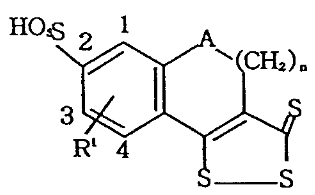

| Compound | A | n | $R^1$ |
|---|---|---|---|
| 1 | $CH_2$ | 0 | H |
| 2 | $CH_2$ | 0 | 1-OH |
| 3 | $CH_2$ | 0 | 3-OH |
| 4 | $CH_2$ | 0 | 4-OH |

| | | | |
|---|---|---|---|
| 5 | $CH_2$ | 0 | 1−F |
| 6 | $CH_2$ | 0 | 3−F |
| 7 | $CH_2$ | 0 | 4−F |
| 8 | $CH_2$ | 0 | 1−Cl |
| 9 | $CH_2$ | 0 | 3−Cl |
| 10 | $CH_2$ | 0 | 4−Cl |
| 11 | $CH_2$ | 0 | 1−Br |
| 12 | $CH_2$ | 0 | 3−Br |
| 13 | $CH_2$ | 0 | 4−Br |
| 14 | $CH_2$ | 0 | 1−I |
| 15 | $CH_2$ | 0 | 3−I |
| 16 | $CH_2$ | 0 | 4−I |
| 17 | $CH_2$ | 0 | $1-CH_3$ |
| 18 | $CH_2$ | 0 | $3-CH_3$ |
| 19 | $CH_2$ | 0 | $4-CH_3$ |
| 20 | $CH_2$ | 0 | $1-CH_3CH_2$ |
| 21 | $CH_2$ | 0 | $3-CH_3CH_2$ |
| 22 | $CH_2$ | 0 | $4-CH_3CH_2$ |
| 23 | $CH_2$ | 0 | $1-CH_3CH_2CH_2$ |
| 24 | $CH_2$ | 0 | $3-CH_3CH_2CH_2$ |
| 25 | $CH_2$ | 0 | $4-CH_3CH_2CH_2$ |
| 26 | $CH_2$ | 0 | $1-(CH_3)_2CH$ |
| 27 | $CH_2$ | 0 | $3-(CH_3)_2CH$ |
| 28 | $CH_2$ | 0 | $4-(CH_3)_2CH$ |
| 29 | $CH_2$ | 0 | $1-CH_3(CH_2)_2CH_2$ |
| 30 | $CH_2$ | 0 | $3-CH_3(CH_2)_2CH_2$ |
| 31 | $CH_2$ | 0 | $4-CH_3(CH_2)_2CH_2$ |
| 32 | $CH_2$ | 0 | $1-(CH_3)_2CHCH_2$ |
| 33 | $CH_2$ | 0 | $3-(CH_3)_2CHCH_2$ |

| | | | |
|---|---|---|---|
| 34 | $CH_2$ | 0 | 4-$(CH_3)_2CHCH_2$ |
| 35 | $CH_2$ | 0 | 1-$CH_3CH_2CH(CH_3)$ |
| 36 | $CH_2$ | 0 | 3-$CH_3CH_2CH(CH_3)$ |
| 37 | $CH_2$ | 0 | 4-$CH_3CH_2CH(CH_3)$ |
| 38 | $CH_2$ | 0 | 1-$(CH_3)_3C$ |
| 39 | $CH_2$ | 0 | 3-$(CH_3)_3C$ |
| 40 | $CH_2$ | 0 | 4-$(CH_3)_3C$ |
| 41 | $CH_2$ | 0 | 1-$CH_3O$ |
| 42 | $CH_2$ | 0 | 3-$CH_3O$ |
| 43 | $CH_2$ | 0 | 4-$CH_3O$ |
| 44 | $CH_2$ | 0 | 1-$CH_3CH_2O$ |
| 45 | $CH_2$ | 0 | 3-$CH_3CH_2O$ |
| 46 | $CH_2$ | 0 | 4-$CH_3CH_2O$ |
| 47 | $CH_2$ | 0 | 1-$CH_3CH_2CH_2O$ |
| 48 | $CH_2$ | 0 | 3-$CH_3CH_2CH_2O$ |
| 49 | $CH_2$ | 0 | 4-$CH_3CH_2CH_2O$ |
| 50 | $CH_2$ | 0 | 1-$(CH_3)_2CHO$ |
| 51 | $CH_2$ | 0 | 3-$(CH_3)_2CHO$ |
| 52 | $CH_2$ | 0 | 4-$(CH_3)_2CHO$ |
| 53 | $CH_2$ | 0 | 1-$CH_3(CH_2)_2CH_2O$ |
| 54 | $CH_2$ | 0 | 3-$CH_3(CH_2)_2CH_2O$ |
| 55 | $CH_2$ | 0 | 4-$CH_3(CH_2)_2CH_2O$ |
| 56 | $CH_2$ | 0 | 1-$(CH_3)_2CHCH_2O$ |
| 57 | $CH_2$ | 0 | 3-$(CH_3)_2CHCH_2O$ |
| 58 | $CH_2$ | 0 | 4-$(CH_3)_2CHCH_2O$ |
| 59 | $CH_2$ | 0 | 1-$CH_3CH_2CH(CH_3)O$ |
| 60 | $CH_2$ | 0 | 3-$CH_3CH_2CH(CH_3)O$ |
| 61 | $CH_2$ | 0 | 4-$CH_3CH_2CH(CH_3)O$ |
| 62 | $CH_2$ | 0 | 1-$(CH_3)_3CO$ |

| | | | |
|---|---|---|---|
| 63 | $CH_2$ | 0 | 3-$(CH_3)_3CO$ |
| 64 | $CH_2$ | 0 | 4-$(CH_3)_3CO$ |
| 65 | $CH_2$ | 1 | H |
| 66 | $CH_2$ | 1 | 1-OH |
| 67 | $CH_2$ | 1 | 3-OH |
| 68 | $CH_2$ | 1 | 4-OH |
| 69 | $CH_2$ | 1 | 1-F |
| 70 | $CH_2$ | 1 | 3-F |
| 71 | $CH_2$ | 1 | 4-F |
| 72 | $CH_2$ | 1 | 1-Cl |
| 73 | $CH_2$ | 1 | 3-Cl |
| 74 | $CH_2$ | 1 | 4-Cl |
| 75 | $CH_2$ | 1 | 1-Br |
| 76 | $CH_2$ | 1 | 3-Br |
| 77 | $CH_2$ | 1 | 4-Br |
| 78 | $CH_2$ | 1 | 1-I |
| 79 | $CH_2$ | 1 | 3-I |
| 80 | $CH_2$ | 1 | 4-I |
| 81 | $CH_2$ | 1 | 1-$CH_3$ |
| 82 | $CH_2$ | 1 | 3-$CH_3$ |
| 83 | $CH_2$ | 1 | 4-$CH_3$ |
| 84 | $CH_2$ | 1 | 1-$CH_3CH_2$ |
| 85 | $CH_2$ | 1 | 3-$CH_3CH_2$ |
| 86 | $CH_2$ | 1 | 4-$CH_3CH_2$ |
| 87 | $CH_2$ | 1 | 1-$CH_3CH_2CH_2$ |
| 88 | $CH_2$ | 1 | 3-$CH_3CH_2CH_2$ |
| 89 | $CH_2$ | 1 | 4-$CH_3CH_2CH_2$ |
| 90 | $CH_2$ | 1 | 1-$(CH_3)_2CH$ |
| 91 | $CH_2$ | 1 | 3-$(CH_3)_2CH$ |

| | | | |
|---|---|---|---|
| 92 | $CH_2$ | 1 | $4-(CH_3)_2CH$ |
| 93 | $CH_2$ | 1 | $1-CH_3(CH_2)_2CH_2$ |
| 94 | $CH_2$ | 1 | $3-CH_3(CH_2)_2CH_2$ |
| 95 | $CH_2$ | 1 | $4-CH_3(CH_2)_2CH_2$ |
| 96 | $CH_2$ | 1 | $1-(CH_3)_2CHCH_2$ |
| 97 | $CH_2$ | 1 | $3-(CH_3)_2CHCH_2$ |
| 98 | $CH_2$ | 1 | $4-(CH_3)_2CHCH_2$ |
| 99 | $CH_2$ | 1 | $1-CH_3CH_2CH(CH_3)$ |
| 100 | $CH_2$ | 1 | $3-CH_3CH_2CH(CH_3)$ |
| 101 | $CH_2$ | 1 | $4-CH_3CH_2CH(CH_3)$ |
| 102 | $CH_2$ | 1 | $1-(CH_3)_3C$ |
| 103 | $CH_2$ | 1 | $3-(CH_3)_3C$ |
| 104 | $CH_2$ | 1 | $4-(CH_3)_3C$ |
| 105 | $CH_2$ | 1 | $1-CH_3O$ |
| 106 | $CH_2$ | 1 | $3-CH_3O$ |
| 107 | $CH_2$ | 1 | $4-CH_3O$ |
| 108 | $CH_2$ | 1 | $1-CH_3CH_2O$ |
| 109 | $CH_2$ | 1 | $3-CH_3CH_2O$ |
| 110 | $CH_2$ | 1 | $4-CH_3CH_2O$ |
| 111 | $CH_2$ | 1 | $1-CH_3CH_2CH_2O$ |
| 112 | $CH_2$ | 1 | $3-CH_3CH_2CH_2O$ |
| 113 | $CH_2$ | 1 | $4-CH_3CH_2CH_2O$ |
| 114 | $CH_2$ | 1 | $1-(CH_3)_2CHO$ |
| 115 | $CH_2$ | 1 | $3-(CH_3)_2CHO$ |
| 116 | $CH_2$ | 1 | $4-(CH_3)_2CHO$ |
| 117 | $CH_2$ | 1 | $1-CH_3(CH_2)_2CH_2O$ |
| 118 | $CH_2$ | 1 | $3-CH_3(CH_2)_2CH_2O$ |
| 119 | $CH_2$ | 1 | $4-CH_3(CH_2)_2CH_2O$ |
| 120 | $CH_2$ | 1 | $1-(CH_3)_2CHCH_2O$ |

| | | | |
|---|---|---|---|
| 121 | $CH_2$ | 1 | 3-$(CH_3)_2CHCH_2O$ |
| 122 | $CH_2$ | 1 | 4-$(CH_3)_2CHCH_2O$ |
| 123 | $CH_2$ | 1 | 1-$CH_3CH_2CH(CH_3)O$ |
| 124 | $CH_2$ | 1 | 3-$CH_3CH_2CH(CH_3)O$ |
| 125 | $CH_2$ | 1 | 4-$CH_3CH_2CH(CH_3)O$ |
| 126 | $CH_2$ | 1 | 1-$(CH_3)_3CO$ |
| 127 | $CH_2$ | 1 | 3-$(CH_3)_3CO$ |
| 128 | $CH_2$ | 1 | 4-$(CH_3)_3CO$ |
| 129 | $CH_2$ | 2 | H |
| 130 | $CH_2$ | 2 | 1-OH |
| 131 | $CH_2$ | 2 | 3-OH |
| 132 | $CH_2$ | 2 | 4-OH |
| 133 | $CH_2$ | 2 | 1-F |
| 134 | $CH_2$ | 2 | 3-F |
| 135 | $CH_2$ | 2 | 4-F |
| 136 | $CH_2$ | 2 | 1-Cl |
| 137 | $CH_2$ | 2 | 3-Cl |
| 138 | $CH_2$ | 2 | 4-Cl |
| 139 | $CH_2$ | 2 | 1-Br |
| 140 | $CH_2$ | 2 | 3-Br |
| 141 | $CH_2$ | 2 | 4-Br |
| 142 | $CH_2$ | 2 | 1-I |
| 143 | $CH_2$ | 2 | 3-I |
| 144 | $CH_2$ | 2 | 4-I |
| 145 | $CH_2$ | 2 | 1-$CH_3$ |
| 146 | $CH_2$ | 2 | 3-$CH_3$ |
| 147 | $CH_2$ | 2 | 4-$CH_3$ |
| 148 | $CH_2$ | 2 | 1-$CH_3CH_2$ |
| 149 | $CH_2$ | 2 | 3-$CH_3CH_2$ |

| | | | |
|---|---|---|---|
| 150 | $CH_2$ | 2 | 4 − $CH_3CH_2$ |
| 151 | $CH_2$ | 2 | 1 − $CH_3CH_2CH_2$ |
| 152 | $CH_2$ | 2 | 3 − $CH_3CH_2CH_2$ |
| 153 | $CH_2$ | 2 | 4 − $CH_3CH_2CH_2$ |
| 154 | $CH_2$ | 2 | 1 − $(CH_3)_2CH$ |
| 155 | $CH_2$ | 2 | 3 − $(CH_3)_2CH$ |
| 156 | $CH_2$ | 2 | 4 − $(CH_3)_2CH$ |
| 157 | $CH_2$ | 2 | 1 − $CH_3(CH_2)_2CH_2$ |
| 158 | $CH_2$ | 2 | 3 − $CH_3(CH_2)_2CH_2$ |
| 159 | $CH_2$ | 2 | 4 − $CH_3(CH_2)_2CH_2$ |
| 160 | $CH_2$ | 2 | 1 − $(CH_3)_2CHCH_2$ |
| 161 | $CH_2$ | 2 | 3 − $(CH_3)_2CHCH_2$ |
| 162 | $CH_2$ | 2 | 4 − $(CH_3)_2CHCH_2$ |
| 163 | $CH_2$ | 2 | 1 − $CH_3CH_2CH(CH_3)$ |
| 164 | $CH_2$ | 2 | 3 − $CH_3CH_2CH(CH_3)$ |
| 165 | $CH_2$ | 2 | 4 − $CH_3CH_2CH(CH_3)$ |
| 166 | $CH_2$ | 2 | 1 − $(CH_3)_3C$ |
| 167 | $CH_2$ | 2 | 3 − $(CH_3)_3C$ |
| 168 | $CH_2$ | 2 | 4 − $(CH_3)_3C$ |
| 169 | $CH_2$ | 2 | 1 − $CH_3O$ |
| 170 | $CH_2$ | 2 | 3 − $CH_3O$ |
| 171 | $CH_2$ | 2 | 4 − $CH_3O$ |
| 172 | $CH_2$ | 2 | 1 − $CH_3CH_2O$ |
| 173 | $CH_2$ | 2 | 3 − $CH_3CH_2O$ |
| 174 | $CH_2$ | 2 | 4 − $CH_3CH_2O$ |
| 175 | $CH_2$ | 2 | 1 − $CH_3CH_2CH_2O$ |
| 176 | $CH_2$ | 2 | 3 − $CH_3CH_2CH_2O$ |
| 177 | $CH_2$ | 2 | 4 − $CH_3CH_2CH_2O$ |
| 178 | $CH_2$ | 2 | 1 − $(CH_3)_2CHO$ |

| | | | |
|---|---|---|---|
| 179 | $CH_2$ | 2 | 3-$(CH_3)_2CHO$ |
| 180 | $CH_2$ | 2 | 4-$(CH_3)_2CHO$ |
| 181 | $CH_2$ | 2 | 1-$CH_3(CH_2)_2CH_2O$ |
| 182 | $CH_2$ | 2 | 3-$CH_3(CH_2)_2CH_2O$ |
| 183 | $CH_2$ | 2 | 4-$CH_3(CH_2)_2CH_2O$ |
| 184 | $CH_2$ | 2 | 1-$(CH_3)_2CHCH_2O$ |
| 185 | $CH_2$ | 2 | 3-$(CH_3)_2CHCH_2O$ |
| 186 | $CH_2$ | 2 | 4-$(CH_3)_2CHCH_2O$ |
| 187 | $CH_2$ | 2 | 1-$CH_3CH_2CH(CH_3)O$ |
| 188 | $CH_2$ | 2 | 3-$CH_3CH_2CH(CH_3)O$ |
| 189 | $CH_2$ | 2 | 4-$CH_3CH_2CH(CH_3)O$ |
| 190 | $CH_2$ | 2 | 1-$(CH_3)_3CO$ |
| 191 | $CH_2$ | 2 | 3-$(CH_3)_3CO$ |
| 192 | $CH_2$ | 2 | 4-$(CH_3)_3CO$ |
| 193 | $CH_2$ | 3 | H |
| 194 | $CH_2$ | 3 | 1-OH |
| 195 | $CH_2$ | 3 | 3-OH |
| 196 | $CH_2$ | 3 | 4-OH |
| 197 | $CH_2$ | 3 | 1-F |
| 198 | $CH_2$ | 3 | 3-F |
| 199 | $CH_2$ | 3 | 4-F |
| 200 | $CH_2$ | 3 | 1-Cl |
| 201 | $CH_2$ | 3 | 3-Cl |
| 202 | $CH_2$ | 3 | 4-Cl |
| 203 | $CH_2$ | 3 | 1-Br |
| 204 | $CH_2$ | 3 | 3-Br |
| 205 | $CH_2$ | 3 | 4-Br |
| 206 | $CH_2$ | 3 | 1-I |
| 207 | $CH_2$ | 3 | 3-I |

| | | | |
|---|---|---|---|
| 208 | $CH_2$ | 3 | 4-I |
| 209 | $CH_2$ | 3 | 1-$CH_3$ |
| 210 | $CH_2$ | 3 | 3-$CH_3$ |
| 211 | $CH_2$ | 3 | 4-$CH_3$ |
| 212 | $CH_2$ | 3 | 1-$CH_3CH_2$ |
| 213 | $CH_2$ | 3 | 3-$CH_3CH_2$ |
| 214 | $CH_2$ | 3 | 4-$CH_3CH_2$ |
| 215 | $CH_2$ | 3 | 1-$CH_3CH_2CH_2$ |
| 216 | $CH_2$ | 3 | 3-$CH_3CH_2CH_2$ |
| 217 | $CH_2$ | 3 | 4-$CH_3CH_2CH_2$ |
| 218 | $CH_2$ | 3 | 1-$(CH_3)_2CH$ |
| 219 | $CH_2$ | 3 | 3-$(CH_3)_2CH$ |
| 220 | $CH_2$ | 3 | 4-$(CH_3)_2CH$ |
| 221 | $CH_2$ | 3 | 1-$CH_3(CH_2)_2CH_2$ |
| 222 | $CH_2$ | 3 | 3-$CH_3(CH_2)_2CH_2$ |
| 223 | $CH_2$ | 3 | 4-$CH_3(CH_2)_2CH_2$ |
| 224 | $CH_2$ | 3 | 1-$(CH_3)_2CHCH_2$ |
| 225 | $CH_2$ | 3 | 3-$(CH_3)_2CHCH_2$ |
| 226 | $CH_2$ | 3 | 4-$(CH_3)_2CHCH_2$ |
| 227 | $CH_2$ | 3 | 1-$CH_3CH_2CH(CH_3)$ |
| 228 | $CH_2$ | 3 | 3-$CH_3CH_2CH(CH_3)$ |
| 229 | $CH_2$ | 3 | 4-$CH_3CH_2CH(CH_3)$ |
| 230 | $CH_2$ | 3 | 1-$(CH_3)_3C$ |
| 231 | $CH_2$ | 3 | 3-$(CH_3)_3C$ |
| 232 | $CH_2$ | 3 | 4-$(CH_3)_3C$ |
| 233 | $CH_2$ | 3 | 1-$CH_3O$ |
| 234 | $CH_2$ | 3 | 3-$CH_3O$ |
| 235 | $CH_2$ | 3 | 4-$CH_3O$ |
| 236 | $CH_2$ | 3 | 1-$CH_3CH_2O$ |

| | | | |
|---|---|---|---|
| 237 | $CH_2$ | 3 | 3 − $CH_3CH_2O$ |
| 238 | $CH_2$ | 3 | 4 − $CH_3CH_2O$ |
| 239 | $CH_2$ | 3 | 1 − $CH_3CH_2CH_2O$ |
| 240 | $CH_2$ | 3 | 3 − $CH_3CH_2CH_2O$ |
| 241 | $CH_2$ | 3 | 4 − $CH_3CH_2CH_2O$ |
| 242 | $CH_2$ | 3 | 1 − $(CH_3)_2CHO$ |
| 243 | $CH_2$ | 3 | 3 − $(CH_3)_2CHO$ |
| 244 | $CH_2$ | 3 | 4 − $(CH_3)_2CHO$ |
| 245 | $CH_2$ | 3 | 1 − $CH_3(CH_2)_2CH_2O$ |
| 246 | $CH_2$ | 3 | 3 − $CH_3(CH_2)_2CH_2O$ |
| 247 | $CH_2$ | 3 | 4 − $CH_3(CH_2)_2CH_2O$ |
| 248 | $CH_2$ | 3 | 1 − $(CH_3)_2CHCH_2O$ |
| 249 | $CH_2$ | 3 | 3 − $(CH_3)_2CHCH_2O$ |
| 250 | $CH_2$ | 3 | 4 − $(CH_3)_2CHCH_2O$ |
| 251 | $CH_2$ | 3 | 1 − $CH_3CH_2CH(CH_3)O$ |
| 252 | $CH_2$ | 3 | 3 − $CH_3CH_2CH(CH_3)O$ |
| 253 | $CH_2$ | 3 | 4 − $CH_3CH_2CH(CH_3)O$ |
| 254 | $CH_2$ | 3 | 1 − $(CH_3)_3CO$ |
| 255 | $CH_2$ | 3 | 3 − $(CH_3)_3CO$ |
| 256 | $CH_2$ | 3 | 4 − $(CH_3)_3CO$ |

Table-3

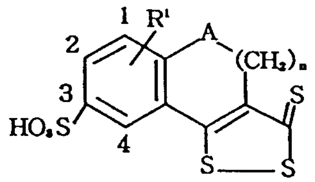

| Compound | A | n | $R^1$ |
|---|---|---|---|
| 1 | $CH_2$ | 0 | H |
| 2 | $CH_2$ | 0 | 1-OH |
| 3 | $CH_2$ | 0 | 2-OH |
| 4 | $CH_2$ | 0 | 4-OH |
| 5 | $CH_2$ | 0 | 1-F |
| 6 | $CH_2$ | 0 | 2-F |
| 7 | $CH_2$ | 0 | 4-F |
| 8 | $CH_2$ | 0 | 1-Cl |
| 9 | $CH_2$ | 0 | 2-Cl |
| 10 | $CH_2$ | 0 | 4-Cl |
| 11 | $CH_2$ | 0 | 1-Br |
| 12 | $CH_2$ | 0 | 2-Br |
| 13 | $CH_2$ | 0 | 4-Br |
| 14 | $CH_2$ | 0 | 1-I |
| 15 | $CH_2$ | 0 | 2-I |
| 16 | $CH_2$ | 0 | 4-I |
| 17 | $CH_2$ | 0 | 1-$CH_3$ |
| 18 | $CH_2$ | 0 | 2-$CH_3$ |
| 19 | $CH_2$ | 0 | 4-$CH_3$ |
| 20 | $CH_2$ | 0 | 1-$CH_3CH_2$ |

| | | | |
|---|---|---|---|
| 21 | $CH_2$ | 0 | $2-CH_3CH_2$ |
| 22 | $CH_2$ | 0 | $4-CH_3CH_2$ |
| 23 | $CH_2$ | 0 | $1-CH_3CH_2CH_2$ |
| 24 | $CH_2$ | 0 | $2-CH_3CH_2CH_2$ |
| 25 | $CH_2$ | 0 | $4-CH_3CH_2CH_2$ |
| 26 | $CH_2$ | 0 | $1-(CH_3)_2CH$ |
| 27 | $CH_2$ | 0 | $2-(CH_3)_2CH$ |
| 28 | $CH_2$ | 0 | $4-(CH_3)_2CH$ |
| 29 | $CH_2$ | 0 | $1-CH_3(CH_2)_2CH_2$ |
| 30 | $CH_2$ | 0 | $2-CH_3(CH_2)_2CH_2$ |
| 31 | $CH_2$ | 0 | $4-CH_3(CH_2)_2CH_2$ |
| 32 | $CH_2$ | 0 | $1-(CH_3)_2CHCH_2$ |
| 33 | $CH_2$ | 0 | $2-(CH_3)_2CHCH_2$ |
| 34 | $CH_2$ | 0 | $4-(CH_3)_2CHCH_2$ |
| 35 | $CH_2$ | 0 | $1-CH_3CH_2CH(CH_3)$ |
| 36 | $CH_2$ | 0 | $2-CH_3CH_2CH(CH_3)$ |
| 37 | $CH_2$ | 0 | $4-CH_3CH_2CH(CH_3)$ |
| 38 | $CH_2$ | 0 | $1-(CH_3)_3C$ |
| 39 | $CH_2$ | 0 | $2-(CH_3)_3C$ |
| 40 | $CH_2$ | 0 | $4-(CH_3)_3C$ |
| 41 | $CH_2$ | 0 | $1-CH_3O$ |
| 42 | $CH_2$ | 0 | $2-CH_3O$ |
| 43 | $CH_2$ | 0 | $4-CH_3O$ |
| 44 | $CH_2$ | 0 | $1-CH_3CH_2O$ |
| 45 | $CH_2$ | 0 | $2-CH_3CH_2O$ |
| 46 | $CH_2$ | 0 | $4-CH_3CH_2O$ |
| 47 | $CH_2$ | 0 | $1-CH_3CH_2CH_2O$ |
| 48 | $CH_2$ | 0 | $2-CH_3CH_2CH_2O$ |
| 49 | $CH_2$ | 0 | $4-CH_3CH_2CH_2O$ |

| | | | |
|---|---|---|---|
| 50 | $CH_2$ | 0 | 1−$(CH_3)_2CHO$ |
| 51 | $CH_2$ | 0 | 2−$(CH_3)_2CHO$ |
| 52 | $CH_2$ | 0 | 4−$(CH_3)_2CHO$ |
| 53 | $CH_2$ | 0 | 1−$CH_3(CH_2)_2CH_2O$ |
| 54 | $CH_2$ | 0 | 2−$CH_3(CH_2)_2CH_2O$ |
| 55 | $CH_2$ | 0 | 4−$CH_3(CH_2)_2CH_2O$ |
| 56 | $CH_2$ | 0 | 1−$(CH_3)_2CHCH_2O$ |
| 57 | $CH_2$ | 0 | 2−$(CH_3)_2CHCH_2O$ |
| 58 | $CH_2$ | 0 | 4−$(CH_3)_2CHCH_2O$ |
| 59 | $CH_2$ | 0 | 1−$CH_3CH_2CH(CH_3)O$ |
| 60 | $CH_2$ | 0 | 2−$CH_3CH_2CH(CH_3)O$ |
| 61 | $CH_2$ | 0 | 4−$CH_3CH_2CH(CH_3)O$ |
| 62 | $CH_2$ | 0 | 1−$(CH_3)_3CO$ |
| 63 | $CH_2$ | 0 | 2−$(CH_3)_3CO$ |
| 64 | $CH_2$ | 0 | 4−$(CH_3)_3CO$ |
| 65 | $CH_2$ | 1 | H |
| 66 | $CH_2$ | 1 | 1−OH |
| 67 | $CH_2$ | 1 | 2−OH |
| 68 | $CH_2$ | 1 | 4−OH |
| 69 | $CH_2$ | 1 | 1−F |
| 70 | $CH_2$ | 1 | 2−F |
| 71 | $CH_2$ | 1 | 4−F |
| 72 | $CH_2$ | 1 | 1−Cl |
| 73 | $CH_2$ | 1 | 2−Cl |
| 74 | $CH_2$ | 1 | 4−Cl |
| 75 | $CH_2$ | 1 | 1−Br |
| 76 | $CH_2$ | 1 | 2−Br |
| 77 | $CH_2$ | 1 | 4−Br |
| 78 | $CH_2$ | 1 | 1−I |

| | | | |
|---|---|---|---|
| 79 | $CH_2$ | 1 | 2-I |
| 80 | $CH_2$ | 1 | 4-I |
| 81 | $CH_2$ | 1 | $1-CH_3$ |
| 82 | $CH_2$ | 1 | $2-CH_3$ |
| 83 | $CH_2$ | 1 | $4-CH_3$ |
| 84 | $CH_2$ | 1 | $1-CH_3CH_2$ |
| 85 | $CH_2$ | 1 | $2-CH_3CH_2$ |
| 86 | $CH_2$ | 1 | $4-CH_3CH_2$ |
| 87 | $CH_2$ | 1 | $1-CH_3CH_2CH_2$ |
| 88 | $CH_2$ | 1 | $2-CH_3CH_2CH_2$ |
| 89 | $CH_2$ | 1 | $4-CH_3CH_2CH_2$ |
| 90 | $CH_2$ | 1 | $1-(CH_3)_2CH$ |
| 91 | $CH_2$ | 1 | $2-(CH_3)_2CH$ |
| 92 | $CH_2$ | 1 | $4-(CH_3)_2CH$ |
| 93 | $CH_2$ | 1 | $1-CH_3(CH_2)_2CH_2$ |
| 94 | $CH_2$ | 1 | $2-CH_3(CH_2)_2CH_2$ |
| 95 | $CH_2$ | 1 | $4-CH_3(CH_2)_2CH_2$ |
| 96 | $CH_2$ | 1 | $1-(CH_3)_2CHCH_2$ |
| 97 | $CH_2$ | 1 | $2-(CH_3)_2CHCH_2$ |
| 98 | $CH_2$ | 1 | $4-(CH_3)_2CHCH_2$ |
| 99 | $CH_2$ | 1 | $1-CH_3CH_2CH(CH_3)$ |
| 100 | $CH_2$ | 1 | $2-CH_3CH_2CH(CH_3)$ |
| 101 | $CH_2$ | 1 | $4-CH_3CH_2CH(CH_3)$ |
| 102 | $CH_2$ | 1 | $1-(CH_3)_3C$ |
| 103 | $CH_2$ | 1 | $2-(CH_3)_3C$ |
| 104 | $CH_2$ | 1 | $4-(CH_3)_3C$ |
| 105 | $CH_2$ | 1 | $1-CH_3O$ |
| 106 | $CH_2$ | 1 | $2-CH_3O$ |
| 107 | $CH_2$ | 1 | $4-CH_3O$ |

| | | | |
|---|---|---|---|
| 108 | $CH_2$ | 1 | $1-CH_3CH_2O$ |
| 109 | $CH_2$ | 1 | $2-CH_3CH_2O$ |
| 110 | $CH_2$ | 1 | $4-CH_3CH_2O$ |
| 111 | $CH_2$ | 1 | $1-CH_3CH_2CH_2O$ |
| 112 | $CH_2$ | 1 | $2-CH_3CH_2CH_2O$ |
| 113 | $CH_2$ | 1 | $4-CH_3CH_2CH_2O$ |
| 114 | $CH_2$ | 1 | $1-(CH_3)_2CHO$ |
| 115 | $CH_2$ | 1 | $2-(CH_3)_2CHO$ |
| 116 | $CH_2$ | 1 | $4-(CH_3)_2CHO$ |
| 117 | $CH_2$ | 1 | $1-CH_3(CH_2)_2CH_2O$ |
| 118 | $CH_2$ | 1 | $2-CH_3(CH_2)_2CH_2O$ |
| 119 | $CH_2$ | 1 | $4-CH_3(CH_2)_2CH_2O$ |
| 120 | $CH_2$ | 1 | $1-(CH_3)_2CHCH_2O$ |
| 121 | $CH_2$ | 1 | $2-(CH_3)_2CHCH_2O$ |
| 122 | $CH_2$ | 1 | $4-(CH_3)_2CHCH_2O$ |
| 123 | $CH_2$ | 1 | $1-CH_3CH_2CH(CH_3)O$ |
| 124 | $CH_2$ | 1 | $2-CH_3CH_2CH(CH_3)O$ |
| 125 | $CH_2$ | 1 | $4-CH_3CH_2CH(CH_3)O$ |
| 126 | $CH_2$ | 1 | $1-(CH_3)_3CO$ |
| 127 | $CH_2$ | 1 | $2-(CH_3)_3CO$ |
| 128 | $CH_2$ | 1 | $4-(CH_3)_3CO$ |
| 129 | $CH_2$ | 2 | H |
| 130 | $CH_2$ | 2 | 1-OH |
| 131 | $CH_2$ | 2 | 2-OH |
| 132 | $CH_2$ | 2 | 4-OH |
| 133 | $CH_2$ | 2 | 1-F |
| 134 | $CH_2$ | 2 | 2-F |
| 135 | $CH_2$ | 2 | 4-F |
| 136 | $CH_2$ | 2 | 1-Cl |

| | | | |
|---|---|---|---|
| 137 | $CH_2$ | 2 | 2-Cl |
| 138 | $CH_2$ | 2 | 4-Cl |
| 139 | $CH_2$ | 2 | 1-Br |
| 140 | $CH_2$ | 2 | 2-Br |
| 141 | $CH_2$ | 2 | 4-Br |
| 142 | $CH_2$ | 2 | I-I |
| 143 | $CH_2$ | 2 | 2-I |
| 144 | $CH_2$ | 2 | 4-I |
| 145 | $CH_2$ | 2 | $1-CH_3$ |
| 146 | $CH_2$ | 2 | $2-CH_3$ |
| 147 | $CH_2$ | 2 | $4-CH_3$ |
| 148 | $CH_2$ | 2 | $1-CH_3CH_2$ |
| 149 | $CH_2$ | 2 | $2-CH_3CH_2$ |
| 150 | $CH_2$ | 2 | $4-CH_3CH_2$ |
| 151 | $CH_2$ | 2 | $1-CH_3CH_2CH_2$ |
| 152 | $CH_2$ | 2 | $2-CH_3CH_2CH_2$ |
| 153 | $CH_2$ | 2 | $4-CH_3CH_2CH_2$ |
| 154 | $CH_2$ | 2 | $1-(CH_3)_2CH$ |
| 155 | $CH_2$ | 2 | $2-(CH_3)_2CH$ |
| 156 | $CH_2$ | 2 | $4-(CH_3)_2CH$ |
| 157 | $CH_2$ | 2 | $1-CH_3(CH_2)_2CH_2$ |
| 158 | $CH_2$ | 2 | $2-CH_3(CH_2)_2CH_2$ |
| 159 | $CH_2$ | 2 | $4-CH_3(CH_2)_2CH_2$ |
| 160 | $CH_2$ | 2 | $1-(CH_3)_2CHCH_2$ |
| 161 | $CH_2$ | 2 | $2-(CH_3)_2CHCH_2$ |
| 162 | $CH_2$ | 2 | $4-(CH_3)_2CHCH_2$ |
| 163 | $CH_2$ | 2 | $1-CH_3CH_2CH(CH_3)$ |
| 164 | $CH_2$ | 2 | $2-CH_3CH_2CH(CH_3)$ |
| 165 | $CH_2$ | 2 | $4-CH_3CH_2CH(CH_3)$ |

| | | | |
|---|---|---|---|
| 166 | $CH_2$ | 2 | 1-$(CH_3)_3C$ |
| 167 | $CH_2$ | 2 | 2-$(CH_3)_3C$ |
| 168 | $CH_2$ | 2 | 4-$(CH_3)_3C$ |
| 169 | $CH_2$ | 2 | 1-$CH_3O$ |
| 170 | $CH_2$ | 2 | 2-$CH_3O$ |
| 171 | $CH_2$ | 2 | 4-$CH_3O$ |
| 172 | $CH_2$ | 2 | 1-$CH_3CH_2O$ |
| 173 | $CH_2$ | 2 | 2-$CH_3CH_2O$ |
| 174 | $CH_2$ | 2 | 4-$CH_3CH_2O$ |
| 175 | $CH_2$ | 2 | 1-$CH_3CH_2CH_2O$ |
| 176 | $CH_2$ | 2 | 2-$CH_3CH_2CH_2O$ |
| 177 | $CH_2$ | 2 | 4-$CH_3CH_2CH_2O$ |
| 178 | $CH_2$ | 2 | 1-$(CH_3)_2CHO$ |
| 179 | $CH_2$ | 2 | 2-$(CH_3)_2CHO$ |
| 180 | $CH_2$ | 2 | 4-$(CH_3)_2CHO$ |
| 181 | $CH_2$ | 2 | 1-$CH_3(CH_2)_2CH_2O$ |
| 182 | $CH_2$ | 2 | 2-$CH_3(CH_2)_2CH_2O$ |
| 183 | $CH_2$ | 2 | 4-$CH_3(CH_2)_2CH_2O$ |
| 184 | $CH_2$ | 2 | 1-$(CH_3)_2CHCH_2O$ |
| 185 | $CH_2$ | 2 | 2-$(CH_3)_2CHCH_2O$ |
| 186 | $CH_2$ | 2 | 4-$(CH_3)_2CHCH_2O$ |
| 187 | $CH_2$ | 2 | 1-$CH_3CH_2CH(CH_3)O$ |
| 188 | $CH_2$ | 2 | 2-$CH_3CH_2CH(CH_3)O$ |
| 189 | $CH_2$ | 2 | 4-$CH_3CH_2CH(CH_3)O$ |
| 190 | $CH_2$ | 2 | 1-$(CH_3)_3CO$ |
| 191 | $CH_2$ | 2 | 2-$(CH_3)_3CO$ |
| 192 | $CH_2$ | 2 | 4-$(CH_3)_3CO$ |
| 193 | $CH_2$ | 3 | H |
| 194 | $CH_2$ | 3 | 1-OH |

| | | | |
|---|---|---|---|
| 195 | $CH_2$ | 3 | 2-OH |
| 196 | $CH_2$ | 3 | 4-OH |
| 197 | $CH_2$ | 3 | 1-F |
| 198 | $CH_2$ | 3 | 2-F |
| 199 | $CH_2$ | 3 | 4-F |
| 200 | $CH_2$ | 3 | 1-Cl |
| 201 | $CH_2$ | 3 | 2-Cl |
| 202 | $CH_2$ | 3 | 4-Cl |
| 203 | $CH_2$ | 3 | 1-Br |
| 204 | $CH_2$ | 3 | 2-Br |
| 205 | $CH_2$ | 3 | 4-Br |
| 206 | $CH_2$ | 3 | 1-I |
| 207 | $CH_2$ | 3 | 2-I |
| 208 | $CH_2$ | 3 | 4-I |
| 209 | $CH_2$ | 3 | $1-CH_3$ |
| 210 | $CH_2$ | 3 | $2-CH_3$ |
| 211 | $CH_2$ | 3 | $4-CH_3$ |
| 212 | $CH_2$ | 3 | $1-CH_3CH_2$ |
| 213 | $CH_2$ | 3 | $2-CH_3CH_2$ |
| 214 | $CH_2$ | 3 | $4-CH_3CH_2$ |
| 215 | $CH_2$ | 3 | $1-CH_3CH_2CH_2$ |
| 216 | $CH_2$ | 3 | $2-CH_3CH_2CH_2$ |
| 217 | $CH_2$ | 3 | $4-CH_3CH_2CH_2$ |
| 218 | $CH_2$ | 3 | $1-(CH_3)_2CH$ |
| 219 | $CH_2$ | 3 | $2-(CH_3)_2CH$ |
| 220 | $CH_2$ | 3 | $4-(CH_3)_2CH$ |
| 221 | $CH_2$ | 3 | $1-CH_3(CH_2)_2CH_2$ |
| 222 | $CH_2$ | 3 | $2-CH_3(CH_2)_2CH_2$ |
| 223 | $CH_2$ | 3 | $4-CH_3(CH_2)_2CH_2$ |

| | | | |
|---|---|---|---|
| 224 | $CH_2$ | 3 | 1-$(CH_3)_2CHCH_2$ |
| 225 | $CH_2$ | 3 | 2-$(CH_3)_2CHCH_2$ |
| 226 | $CH_2$ | 3 | 4-$(CH_3)_2CHCH_2$ |
| 227 | $CH_2$ | 3 | 1-$CH_3CH_2CH(CH_3)$ |
| 228 | $CH_2$ | 3 | 2-$CH_3CH_2CH(CH_3)$ |
| 229 | $CH_2$ | 3 | 4-$CH_3CH_2CH(CH_3)$ |
| 230 | $CH_2$ | 3 | 1-$(CH_3)_3C$ |
| 231 | $CH_2$ | 3 | 2-$(CH_3)_3C$ |
| 232 | $CH_2$ | 3 | 4-$(CH_3)_3C$ |
| 233 | $CH_2$ | 3 | 1-$CH_3O$ |
| 234 | $CH_2$ | 3 | 2-$CH_3O$ |
| 235 | $CH_2$ | 3 | 4-$CH_3O$ |
| 236 | $CH_2$ | 3 | 1-$CH_3CH_2O$ |
| 237 | $CH_2$ | 3 | 2-$CH_3CH_2O$ |
| 238 | $CH_2$ | 3 | 4-$CH_3CH_2O$ |
| 239 | $CH_2$ | 3 | 1-$CH_3CH_2CH_2O$ |
| 240 | $CH_2$ | 3 | 2-$CH_3CH_2CH_2O$ |
| 241 | $CH_2$ | 3 | 4-$CH_3CH_2CH_2O$ |
| 242 | $CH_2$ | 3 | 1-$(CH_3)_2CHO$ |
| 243 | $CH_2$ | 3 | 2-$(CH_3)_2CHO$ |
| 244 | $CH_2$ | 3 | 4-$(CH_3)_2CHO$ |
| 245 | $CH_2$ | 3 | 1-$CH_3(CH_2)_2CH_2O$ |
| 246 | $CH_2$ | 3 | 2-$CH_3(CH_2)_2CH_2O$ |
| 247 | $CH_2$ | 3 | 4-$CH_3(CH_2)_2CH_2O$ |
| 248 | $CH_2$ | 3 | 1-$(CH_3)_2CHCH_2O$ |
| 249 | $CH_2$ | 3 | 2-$(CH_3)_2CHCH_2O$ |
| 250 | $CH_2$ | 3 | 4-$(CH_3)_2CHCH_2O$ |
| 251 | $CH_2$ | . 3 | 1-$CH_3CH_2CH(CH_3)O$ |
| 252 | $CH_2$ | 3 | 2-$CH_3CH_2CH(CH_3)O$ |

| | | | |
|---|---|---|---|
| 253 | $CH_2$ | 3 | $4-CH_3CH_2CH(CH_3)O$ |
| 254 | $CH_2$ | 3 | $1-(CH_3)_3CO$ |
| 255 | $CH_2$ | 3 | $2-(CH_3)_3CO$ |
| 256 | $CH_2$ | 3 | $4-(CH_3)_3CO$ |

Table-4

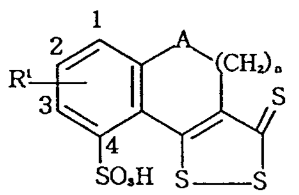

| Compound | A | n | $R^1$ |
|---|---|---|---|
| 1 | $CH_2$ | 0 | H |
| 2 | $CH_2$ | 0 | 1-OH |
| 3 | $CH_2$ | 0 | 2-OH |
| 4 | $CH_2$ | 0 | 3-OH |
| 5 | $CH_2$ | 0 | 1-F |
| 6 | $CH_2$ | 0 | 2-F |
| 7 | $CH_2$ | 0 | 3-F |
| 8 | $CH_2$ | 0 | 1-Cl |
| 9 | $CH_2$ | 0 | 2-Cl |
| 10 | $CH_2$ | 0 | 3-Cl |

| | | | |
|---|---|---|---|
| 11 | $CH_2$ | 0 | 1-Br |
| 12 | $CH_2$ | 0 | 2-Br |
| 13 | $CH_2$ | 0 | 3-Br |
| 14 | $CH_2$ | 0 | 1-I |
| 15 | $CH_2$ | 0 | 2-I |
| 16 | $CH_2$ | 0 | 3-I |
| 17 | $CH_2$ | 0 | $1-CH_3$ |
| 18 | $CH_2$ | 0 | $2-CH_3$ |
| 19 | $CH_2$ | 0 | $3-CH_3$ |
| 20 | $CH_2$ | 0 | $1-CH_3CH_2$ |
| 21 | $CH_2$ | 0 | $2-CH_3CH_2$ |
| 22 | $CH_2$ | 0 | $3-CH_3CH_2$ |
| 23 | $CH_2$ | 0 | $1-CH_3CH_2CH_2$ |
| 24 | $CH_2$ | 0 | $2-CH_3CH_2CH_2$ |
| 25 | $CH_2$ | 0 | $3-CH_3CH_2CH_2$ |
| 26 | $CH_2$ | 0 | $1-(CH_3)_2CH$ |
| 27 | $CH_2$ | 0 | $2-(CH_3)_2CH$ |
| 28 | $CH_2$ | 0 | $3-(CH_3)_2CH$ |
| 29 | $CH_2$ | 0 | $1-CH_3(CH_2)_2CH_2$ |
| 30 | $CH_2$ | 0 | $2-CH_3(CH_2)_2CH_2$ |
| 31 | $CH_2$ | 0 | $3-CH_3(CH_2)_2CH_2$ |
| 32 | $CH_2$ | 0 | $1-(CH_3)_2CHCH_2$ |
| 33 | $CH_2$ | 0 | $2-(CH_3)_2CHCH_2$ |
| 34 | $CH_2$ | 0 | $3-(CH_3)_2CHCH_2$ |
| 35 | $CH_2$ | 0 | $1-CH_3CH_2CH(CH_3)$ |
| 36 | $CH_2$ | 0 | $2-CH_3CH_2CH(CH_3)$ |
| 37 | $CH_2$ | 0 | $3-CH_3CH_2CH(CH_3)$ |
| 38 | $CH_2$ | 0 | $1-(CH_3)_3C$ |
| 39 | $CH_2$ | 0 | $2-(CH_3)_3C$ |

| | | | |
|---|---|---|---|
| 40 | $CH_2$ | 0 | $3-(CH_3)_3C$ |
| 41 | $CH_2$ | 0 | $1-CH_3O$ |
| 42 | $CH_2$ | 0 | $2-CH_3O$ |
| 43 | $CH_2$ | 0 | $3-CH_3O$ |
| 44 | $CH_2$ | 0 | $1-CH_3CH_2O$ |
| 45 | $CH_2$ | 0 | $2-CH_3CH_2O$ |
| 46 | $CH_2$ | 0 | $3-CH_3CH_2O$ |
| 47 | $CH_2$ | 0 | $1-CH_3CH_2CH_2O$ |
| 48 | $CH_2$ | 0 | $2-CH_3CH_2CH_2O$ |
| 49 | $CH_2$ | 0 | $3-CH_3CH_2CH_2O$ |
| 50 | $CH_2$ | 0 | $1-(CH_3)_2CHO$ |
| 51 | $CH_2$ | 0 | $2-(CH_3)_2CHO$ |
| 52 | $CH_2$ | 0 | $3-(CH_3)_2CHO$ |
| 53 | $CH_2$ | 0 | $1-CH_3(CH_2)_2CH_2O$ |
| 54 | $CH_2$ | 0 | $2-CH_3(CH_2)_2CH_2O$ |
| 55 | $CH_2$ | 0 | $3-CH_3(CH_2)_2CH_2O$ |
| 56 | $CH_2$ | 0 | $1-(CH_3)_2CHCH_2O$ |
| 57 | $CH_2$ | 0 | $2-(CH_3)_2CHCH_2O$ |
| 58 | $CH_2$ | 0 | $3-(CH_3)_2CHCH_2O$ |
| 59 | $CH_2$ | 0 | $1-CH_3CH_2CH(CH_3)O$ |
| 60 | $CH_2$ | 0 | $2-CH_3CH_2CH(CH_3)O$ |
| 61 | $CH_2$ | 0 | $3-CH_3CH_2CH(CH_3)O$ |
| 62 | $CH_2$ | 0 | $1-(CH_3)_3CO$ |
| 63 | $CH_2$ | 0 | $2-(CH_3)_3CO$ |
| 64 | $CH_2$ | 0 | $3-(CH_3)_3CO$ |
| 65 | $CH_2$ | 1 | H |
| 66 | $CH_2$ | 1 | $1-OH$ |
| 67 | $CH_2$ | 1 | $2-OH$ |
| 68 | $CH_2$ | 1 | $3-OH$ |

| | | | |
|---|---|---|---|
| 69 | $CH_2$ | 1 | 1−F |
| 70 | $CH_2$ | 1 | 2−F |
| 71 | $CH_2$ | 1 | 3−F |
| 72 | $CH_2$ | 1 | 1−Cl |
| 73 | $CH_2$ | 1 | 2−Cl |
| 74 | $CH_2$ | 1 | 3−Cl |
| 75 | $CH_2$ | 1 | 1−Br |
| 76 | $CH_2$ | 1 | 2−Br |
| 77 | $CH_2$ | 1 | 3−Br |
| 78 | $CH_2$ | 1 | 1−I |
| 79 | $CH_2$ | 1 | 2−1 |
| 80 | $CH_2$ | 1 | 3−I |
| 81 | $CH_2$ | 1 | 1−$CH_3$ |
| 82 | $CH_2$ | 1 | 2−$CH_3$ |
| 83 | $CH_2$ | 1 | 3−$CH_3$ |
| 84 | $CH_2$ | 1 | 1−$CH_3CH_2$ |
| 85 | $CH_2$ | 1 | 2−$CH_3CH_2$ |
| 86 | $CH_2$ | 1 | 3−$CH_3CH_2$ |
| 87 | $CH_2$ | 1 | 1−$CH_3CH_2CH_2$ |
| 88 | $CH_2$ | 1 | 2−$CH_3CH_2CH_2$ |
| 89 | $CH_2$ | 1 | 3−$CH_3CH_2CH_2$ |
| 90 | $CH_2$ | 1 | 1−$(CH_3)_2CH$ |
| 91 | $CH_2$ | 1 | 2−$(CH_3)_2CH$ |
| 92 | $CH_2$ | 1 | 3−$(CH_3)_2CH$ |
| 93 | $CH_2$ | 1 | 1−$CH_3(CH_2)_2CH_2$ |
| 94 | $CH_2$ | 1 | 2−$CH_3(CH_2)_2CH_2$ |
| 95 | $CH_2$ | 1 | 3−$CH_3(CH_2)_2CH_2$ |
| 96 | $CH_2$ | 1 | 1−$(CH_3)_2CHCH_2$ |
| 97 | $CH_2$ | 1 | 2−$(CH_3)_2CHCH_2$ |

| | | | |
|---|---|---|---|
| 98 | $CH_2$ | 1 | $3-(CH_3)_2CHCH_2$ |
| 99 | $CH_2$ | 1 | $1-CH_3CH_2CH(CH_3)$ |
| 100 | $CH_2$ | 1 | $2-CH_3CH_2CH(CH_3)$ |
| 101 | $CH_2$ | 1 | $3-CH_3CH_2CH(CH_3)$ |
| 102 | $CH_2$ | 1 | $1-(CH_3)_3C$ |
| 103 | $CH_2$ | 1 | $2-(CH_3)_3C$ |
| 104 | $CH_2$ | 1 | $3-(CH_3)_3C$ |
| 105 | $CH_2$ | 1 | $1-CH_3O$ |
| 106 | $CH_2$ | 1 | $2-CH_3O$ |
| 107 | $CH_2$ | 1 | $3-CH_3O$ |
| 108 | $CH_2$ | 1 | $1-CH_3CH_2O$ |
| 109 | $CH_2$ | 1 | $2-CH_3CH_2O$ |
| 110 | $CH_2$ | 1 | $3-CH_3CH_2O$ |
| 111 | $CH_2$ | 1 | $1-CH_3CH_2CH_2O$ |
| 112 | $CH_2$ | 1 | $2-CH_3CH_2CH_2O$ |
| 113 | $CH_2$ | 1 | $3-CH_3CH_2CH_2O$ |
| 114 | $CH_2$ | 1 | $1-(CH_3)_2CHO$ |
| 115 | $CH_2$ | 1 | $2-(CH_3)_2CHO$ |
| 116 | $CH_2$ | 1 | $3-(CH_3)_2CHO$ |
| 117 | $CH_2$ | 1 | $1-CH_3(CH_2)_2CH_2O$ |
| 118 | $CH_2$ | 1 | $2-CH_3(CH_2)_2CH_2O$ |
| 119 | $CH_2$ | 1 | $3-CH_3(CH_2)_2CH_2O$ |
| 120 | $CH_2$ | 1 | $1-(CH_3)_2CHCH_2O$ |
| 121 | $CH_2$ | 1 | $2-(CH_3)_2CHCH_2O$ |
| 122 | $CH_2$ | 1 | $3-(CH_3)_2CHCH_2O$ |
| 123 | $CH_2$ | 1 | $1-CH_3CH_2CH(CH_3)O$ |
| 124 | $CH_2$ | 1 | $2-CH_3CH_2CH(CH_3)O$ |
| 125 | $CH_2$ | 1 | $3-CH_3CH_2CH(CH_3)O$ |
| 126 | $CH_2$ | 1 | $1-(CH_3)_3CO$ |

| | | | |
|---|---|---|---|
| 127 | $CH_2$ | 1 | 2－$(CH_3)_3CO$ |
| 128 | $CH_2$ | 1 | 3－$(CH_3)_3CO$ |
| 129 | $CH_2$ | 2 | H |
| 130 | $CH_2$ | 2 | 1－OH |
| 131 | $CH_2$ | 2 | 2－OH |
| 132 | $CH_2$ | 2 | 3－OH |
| 133 | $CH_2$ | 2 | 1－F |
| 134 | $CH_2$ | 2 | 2－F |
| 135 | $CH_2$ | 2 | 3－F |
| 136 | $CH_2$ | 2 | 1－Cl |
| 137 | $CH_2$ | 2 | 2－Cl |
| 138 | $CH_2$ | 2 | 3－Cl |
| 139 | $CH_2$ | 2 | 1－Br |
| 140 | $CH_2$ | 2 | 2－Br |
| 141 | $CH_2$ | 2 | 3－Br |
| 142 | $CH_2$ | 2 | 1－I |
| 143 | $CH_2$ | 2 | 2－I |
| 144 | $CH_2$ | 2 | 3－I |
| 145 | $CH_2$ | 2 | 1－$CH_3$ |
| 146 | $CH_2$ | 2 | 2－$CH_3$ |
| 147 | $CH_2$ | 2 | 3－$CH_3$ |
| 148 | $CH_2$ | 2 | 1－$CH_3CH_2$ |
| 149 | $CH_2$ | 2 | 2－$CH_3CH_2$ |
| 150 | $CH_2$ | 2 | 3－$CH_3CH_2$ |
| 151 | $CH_2$ | 2 | 1－$CH_3CH_2CH_2$ |
| 152 | $CH_2$ | 2 | 2－$CH_3CH_2CH_2$ |
| 153 | $CH_2$ | 2 | 3－$CH_3CH_2CH_2$ |
| 154 | $CH_2$ | 2 | 1－$(CH_3)_2CH$ |
| 155 | $CH_2$ | 2 | 2－$(CH_3)_2CH$ |

| | | | |
|---|---|---|---|
| 156 | $CH_2$ | 2 | 3-$(CH_3)_2CH$ |
| 157 | $CH_2$ | 2 | 1-$CH_3(CH_2)_2CH_2$ |
| 158 | $CH_2$ | 2 | 2-$CH_3(CH_2)_2CH_2$ |
| 159 | $CH_2$ | 2 | 3-$CH_3(CH_2)_2CH_2$ |
| 160 | $CH_2$ | 2 | 1-$(CH_3)_2CHCH_2$ |
| 161 | $CH_2$ | 2 | 2-$(CH_3)_2CHCH_2$ |
| 162 | $CH_2$ | 2 | 3-$(CH_3)_2CHCH_2$ |
| 163 | $CH_2$ | 2 | 1-$CH_3CH_2CH(CH_3)$ |
| 164 | $CH_2$ | 2 | 2-$CH_3CH_2CH(CH_3)$ |
| 165 | $CH_2$ | 2 | 3-$CH_3CH_2CH(CH_3)$ |
| 166 | $CH_2$ | 2 | 1-$(CH_3)_3C$ |
| 167 | $CH_2$ | 2 | 2-$(CH_3)_3C$ |
| 168 | $CH_2$ | 2 | 3-$(CH_3)_3C$ |
| 169 | $CH_2$ | 2 | 1-$CH_3O$ |
| 170 | $CH_2$ | 2 | 2-$CH_3O$ |
| 171 | $CH_2$ | 2 | 3-$CH_3O$ |
| 172 | $CH_2$ | 2 | 1-$CH_3CH_2O$ |
| 173 | $CH_2$ | 2 | 2-$CH_3CH_2O$ |
| 174 | $CH_2$ | 2 | 3-$CH_3CH_2O$ |
| 175 | $CH_2$ | 2 | 1-$CH_3CH_2CH_2O$ |
| 176 | $CH_2$ | 2 | 2-$CH_3CH_2CH_2O$ |
| 177 | $CH_2$ | 2 | 3-$CH_3CH_2CH_2O$ |
| 178 | $CH_2$ | 2 | 1-$(CH_3)_2CHO$ |
| 179 | $CH_2$ | 2 | 2-$(CH_3)_2CHO$ |
| 180 | $CH_2$ | 2 | 3-$(CH_3)_2CHO$ |
| 181 | $CH_2$ | 2 | 1-$CH_3(CH_2)_2CH_2O$ |
| 182 | $CH_2$ | 2 | 2-$CH_3(CH_2)_2CH_2O$ |
| 183 | $CH_2$ | 2 | 3-$CH_3(CH_2)_2CH_2O$ |
| 184 | $CH_2$ | 2 | 1-$(CH_3)_2CHCH_2O$ |

| | | | |
|---|---|---|---|
| 185 | $CH_2$ | 2 | 2-$(CH_3)_2CHCH_2O$ |
| 186 | $CH_2$ | 2 | 3-$(CH_3)_2CHCH_2O$ |
| 187 | $CH_2$ | 2 | 1-$CH_3CH_2CH(CH_3)O$ |
| 188 | $CH_2$ | 2 | 2-$CH_3CH_2CH(CH_3)O$ |
| 189 | $CH_2$ | 2 | 3-$CH_3CH_2CH(CH_3)O$ |
| 190 | $CH_2$ | 2 | 1-$(CH_3)_3CO$ |
| 191 | $CH_2$ | 2 | 2-$(CH_3)_3CO$ |
| 192 | $CH_2$ | 2 | 3-$(CH_3)_3CO$ |
| 193 | $CH_2$ | 3 | H |
| 194 | $CH_2$ | 3 | 1-OH |
| 195 | $CH_2$ | 3 | 2-OH |
| 196 | $CH_2$ | 3 | 3-OH |
| 197 | $CH_2$ | 3 | 1-F |
| 198 | $CH_2$ | 3 | 2-F |
| 199 | $CH_2$ | 3 | 3-F |
| 200 | $CH_2$ | 3 | 1-Cl |
| 201 | $CH_2$ | 3 | 2-Cl |
| 202 | $CH_2$ | 3 | 3-Cl |
| 203 | $CH_2$ | 3 | 1-Br |
| 204 | $CH_2$ | 3 | 2-Br |
| 205 | $CH_2$ | 3 | 3-Br |
| 206 | $CH_2$ | 3 | 1-I |
| 207 | $CH_2$ | 3 | 2-I |
| 208 | $CH_2$ | 3 | 3-I |
| 209 | $CH_2$ | 3 | 1-$CH_3$ |
| 210 | $CH_2$ | 3 | 2-$CH_3$ |
| 211 | $CH_2$ | 3 | 3-$CH_3$ |
| 212 | $CH_2$ | 3 | 1-$CH_3CH_2$ |
| 213 | $CH_2$ | 3 | 2-$CH_3CH_2$ |

| | | | |
|---|---|---|---|
| 214 | $CH_2$ | 3 | $3-CH_3CH_2$ |
| 215 | $CH_2$ | 3 | $1-CH_3CH_2CH_2$ |
| 216 | $CH_2$ | 3 | $2-CH_3CH_2CH_2$ |
| 217 | $CH_2$ | 3 | $3-CH_3CH_2CH_2$ |
| 218 | $CH_2$ | 3 | $1-(CH_3)_2CH$ |
| 219 | $CH_2$ | 3 | $2-(CH_3)_2CH$ |
| 220 | $CH_2$ | 3 | $3-(CH_3)_2CH$ |
| 221 | $CH_2$ | 3 | $1-CH_3(CH_2)_2CH_2$ |
| 222 | $CH_2$ | 3 | $2-CH_3(CH_2)_2CH_2$ |
| 223 | $CH_2$ | 3 | $3-CH_3(CH_2)_2CH_2$ |
| 224 | $CH_2$ | 3 | $1-(CH_3)_2CHCH_2$ |
| 225 | $CH_2$ | 3 | $2-(CH_3)_2CHCH_2$ |
| 226 | $CH_2$ | 3 | $3-(CH_3)_2CHCH_2$ |
| 227 | $CH_2$ | 3 | $1-CH_3CH_2CH(CH_3)$ |
| 228 | $CH_2$ | 3 | $2-CH_3CH_2CH(CH_3)$ |
| 229 | $CH_2$ | 3 | $3-CH_3CH_2CH(CH_3)$ |
| 230 | $CH_2$ | 3 | $1-(CH_3)_3C$ |
| 231 | $CH_2$ | 3 | $2-(CH_3)_3C$ |
| 232 | $CH_2$ | 3 | $3-(CH_3)_3C$ |
| 233 | $CH_2$ | 3 | $1-CH_3O$ |
| 234 | $CH_2$ | 3 | $2-CH_3O$ |
| 235 | $CH_2$ | 3 | $3-CH_3O$ |
| 236 | $CH_2$ | 3 | $1-CH_3CH_2O$ |
| 237 | $CH_2$ | 3 | $2-CH_3CH_2O$ |
| 238 | $CH_2$ | 3 | $3-CH_3CH_2O$ |
| 239 | $CH_2$ | 3 | $1-CH_3CH_2CH_2O$ |
| 240 | $CH_2$ | 3 | $2-CH_3CH_2CH_2O$ |
| 241 | $CH_2$ | 3 | $3-CH_3CH_2CH_2O$ |
| 242 | $CH_2$ | 3 | $1-(CH_3)_2CHO$ |

| | | | |
|---|---|---|---|
| 243 | $CH_2$ | 3 | 2-$(CH_3)_2CHO$ |
| 244 | $CH_2$ | 3 | 3-$(CH_3)_2CHO$ |
| 245 | $CH_2$ | 3 | 1-$CH_3(CH_2)_2CH_2O$ |
| 246 | $CH_2$ | 3 | 2-$CH_3(CH_2)_2CH_2O$ |
| 247 | $CH_2$ | 3 | 3-$CH_3(CH_2)_2CH_2O$ |
| 248 | $CH_2$ | 3 | 1-$(CH_3)_2CHCH_2O$ |
| 249 | $CH_2$ | 3 | 2-$(CH_3)_2CHCH_2O$ |
| 250 | $CH_2$ | 3 | 3-$(CH_3)_2CHCH_2O$ |
| 251 | $CH_2$ | 3 | 1-$CH_3CH_2CH(CH_3)O$ |
| 252 | $CH_2$ | 3 | 2-$CH_3CH_2CH(CH_3)O$ |
| 253 | $CH_2$ | 3 | 3-$CH_3CH_2CH(CH_3)O$ |
| 254 | $CH_2$ | 3 | 1-$(CH_3)_3CO$ |
| 255 | $CH_2$ | 3 | 2-$(CH_3)_3CO$ |
| 256 | $CH_2$ | 3 | 3-$(CH_3)_3CO$ |

Table-5

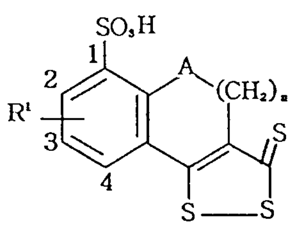

| Compound | A | n | $R^1$ |
|---|---|---|---|

| | | | |
|---|---|---|---|
| 1 | O | 1 | H |
| 2 | O | 1 | 2-OH |
| 3 | O | 1 | 3-OH |
| 4 | O | 1 | 4-OH |
| 5 | O | 1 | 2-F |
| 6 | O | 1 | 3-F |
| 7 | O | 1 | 4-F |
| 8 | O | 1 | 2-Cl |
| 9 | O | 1 | 3-Cl |
| 10 | O | 1 | 4-Cl |
| 11 | O | 1 | 2-Br |
| 12 | O | 1 | 3-Br |
| 13 | O | 1 | 4-Br |
| 14 | O | 1 | 2-I |
| 15 | O | 1 | 3-I |
| 16 | O | 1 | 4-I |
| 17 | O | 1 | $2-CH_3$ |
| 18 | O | 1 | $3-CH_3$ |
| 19 | O | 1 | $4-CH_3$ |
| 20 | O | 1 | $2-CH_3CH_2$ |
| 21 | O | 1 | $3-CH_3CH_2$ |
| 22 | O | 1 | $4-CH_3CH_2$ |
| 23 | O | 1 | $2-CH_3CH_2CH_2$ |
| 24 | O | 1 | $3-CH_3CH_2CH_2$ |
| 25 | O | 1 | $4-CH_3CH_2CH_2$ |
| 26 | O | 1 | $2-(CH_3)_2CH$ |
| 27 | O | 1 | $3-(CH_3)_2CH$ |
| 28 | O | 1 | $4-(CH_3)_2CH$ |
| 29 | O | 1 | $2-CH_3(CH_2)_2CH_2$ |

| | | | |
|---|---|---|---|
| 30 | O | 1 | 3-$CH_3(CH_2)_2CH_2$ |
| 31 | O | 1 | 4-$CH_3(CH_2)_2CH_2$ |
| 32 | O | 1 | 2-$(CH_3)_2CHCH_2$ |
| 33 | O | 1 | 3-$(CH_3)_2CHCH_2$ |
| 34 | O | 1 | 4-$(CH_3)_2CHCH_2$ |
| 35 | O | 1 | 2-$CH_3CH_2CH(CH_3)$ |
| 36 | O | 1 | 3-$CH_3CH_2CH(CH_3)$ |
| 37 | O | 1 | 4-$CH_3CH_2CH(CH_3)$ |
| 38 | O | 1 | 2-$(CH_3)_3C$ |
| 39 | O | 1 | 3-$(CH_3)_3C$ |
| 40 | O | 1 | 4-$(CH_3)_3C$ |
| 41 | O | 1 | 2-$CH_3O$ |
| 42 | O | 1 | 3-$CH_3O$ |
| 43 | O | 1 | 4-$CH_3O$ |
| 44 | O | 1 | 2-$CH_3CH_2O$ |
| 45 | O | 1 | 3-$CH_3CH_2O$ |
| 46 | O | 1 | 4-$CH_3CH_2O$ |
| 47 | O | 1 | 2-$CH_3CH_2CH_2O$ |
| 48 | O | 1 | 3-$CH_3CH_2CH_2O$ |
| 49 | O | 1 | 4-$CH_3CH_2CH_2O$ |
| 50 | O | 1 | 2-$(CH_3)_2CHO$ |
| 51 | O | 1 | 3-$(CH_3)_2CHO$ |
| 52 | O | 1 | 4-$(CH_3)_2CHO$ |
| 53 | O | 1 | 2-$CH_3(CH_2)_2CH_2O$ |
| 54 | O | 1 | 3-$CH_3(CH_2)_2CH_2O$ |
| 55 | O | 1 | 4-$CH_3(CH_2)_2CH_2O$ |
| 56 | O | 1 | 2-$(CH_3)_2CHCH_2O$ |
| 57 | O | 1 | 3-$(CH_3)_2CHCH_2O$ |
| 58 | O | 1 | 4-$(CH_3)_2CHCH_2O$ |

| | | | |
|---|---|---|---|
| 59 | O | 1 | $2-CH_3CH_2CH(CH_3)O$ |
| 60 | O | 1 | $3-CH_3CH_2CH(CH_3)O$ |
| 61 | O | 1 | $4-CH_3CH_2CH(CH_3)O$ |
| 62 | O | 1 | $2-(CH_3)_3CO$ |
| 63 | O | 1 | $3-(CH_3)_3CO$ |
| 64 | O | 1 | $4-(CH_3)_3CO$ |
| 65 | O | 2 | H |
| 66 | O | 2 | 2-OH |
| 67 | O | 2 | 3-OH |
| 68 | O | 2 | 4-OH |
| 69 | O | 2 | 2-F |
| 70 | O | 2 | 3-F |
| 71 | O | 2 | 4-F |
| 72 | O | 2 | 2-Cl |
| 73 | O | 2 | 3-Cl |
| 74 | O | 2 | 4-Cl |
| 75 | O | 2 | 2-Br |
| 76 | O | 2 | 3-Br |
| 77 | O | 2 | 4-Br |
| 78 | O | 2 | 2-I |
| 79 | O | 2 | 3-I |
| 80 | O | 2 | 4-I |
| 81 | O | 2 | $2-CH_3$ |
| 82 | O | 2 | $3-CH_3$ |
| 83 | O | 2 | $4-CH_3$ |
| 84 | O | 2 | $2-CH_3CH_2$ |
| 85 | O | 2 | $3-CH_3CH_2$ |
| 86 | O | 2 | $4-CH_3CH_2$ |
| 87 | O | 2 | $2-CH_3CH_2CH_2$ |

| | | | |
|---|---|---|---|
| 88 | O | 2 | 3-$CH_3CH_2CH_2$ |
| 89 | O | 2 | 4-$CH_3CH_2CH_2$ |
| 90 | O | 2 | 2-$(CH_3)_2CH$ |
| 91 | O | 2 | 3-$(CH_3)_2CH$ |
| 92 | O | 2 | 4-$(CH_3)_2CH$ |
| 93 | O | 2 | 2-$CH_3(CH_2)_2CH_2$ |
| 94 | O | 2 | 3-$CH_3(CH_2)_2CH_2$ |
| 95 | O | 2 | 4-$CH_3(CH_2)_2CH_2$ |
| 96 | O | 2 | 2-$(CH_3)_2CHCH_2$ |
| 97 | O | 2 | 3-$(CH_3)_2CHCH_2$ |
| 98 | O | 2 | 4-$(CH_3)_2CHCH_2$ |
| 99 | O | 2 | 2-$CH_3CH_2CH(CH_3)$ |
| 100 | O | 2 | 3-$CH_3CH_2CH(CH_3)$ |
| 101 | O | 2 | 4-$CH_3CH_2CH(CH_3)$ |
| 102 | O | 2 | 2-$(CH_3)_3C$ |
| 103 | O | 2 | 3-$(CH_3)_3C$ |
| 104 | O | 2 | 4-$(CH_3)_3C$ |
| 105 | O | 2 | 2-$CH_3O$ |
| 106 | O | 2 | 3-$CH_3O$ |
| 107 | O | 2 | 4-$CH_3O$ |
| 108 | O | 2 | 2-$CH_3CH_2O$ |
| 109 | O | 2 | 3-$CH_3CH_2O$ |
| 110 | O | 2 | 4-$CH_3CH_2O$ |
| 111 | O | 2 | 2-$CH_3CH_2CH_2O$ |
| 112 | O | 2 | 3-$CH_3CH_2CH_2O$ |
| 113 | O | 2 | 4-$CH_3CH_2CH_2O$ |
| 114 | O | 2 | 2-$(CH_3)_2CHO$ |
| 115 | O | 2 | 3-$(CH_3)_2CHO$ |
| 116 | O | 2 | 4-$(CH_3)_2CHO$ |

| | | | |
|---|---|---|---|
| 117 | O | 2 | 2-$CH_3(CH_2)_2CH_2O$ |
| 118 | O | 2 | 3-$CH_3(CH_2)_2CH_2O$ |
| 119 | O | 2 | 4-$CH_3(CH_2)_2CH_2O$ |
| 120 | O | 2 | 2-$(CH_3)_2CHCH_2O$ |
| 121 | O | 2 | 3-$(CH_3)_2CHCH_2O$ |
| 122 | O | 2 | 4-$(CH_3)_2CHCH_2O$ |
| 123 | O | 2 | 2-$CH_3CH_2CH(CH_3)O$ |
| 124 | O | 2 | 3-$CH_3CH_2CH(CH_3)O$ |
| 125 | O | 2 | 4-$CH_3CH_2CH(CH_3)O$ |
| 126 | O | 2 | 2-$(CH_3)_3CO$ |
| 127 | O | 2 | 3-$(CH_3)_3CO$ |
| 128 | O | 2 | 4-$(CH_3)_3CO$ |
| 129 | O | 3 | H |
| 130 | O | 3 | 2-OH |
| 131 | O | 3 | 3-OH |
| 132 | O | 3 | 4-OH |
| 133 | O | 3 | 2-F |
| 134 | O | 3 | 3-F |
| 135 | O | 3 | 4-F |
| 136 | O | 3 | 2-Cl |
| 137 | O | 3 | 3-Cl |
| 138 | O | 3 | 4-Cl |
| 139 | O | 3 | 2-Br |
| 140 | O | 3 | 3-Br |
| 141 | O | 3 | 4-Br |
| 142 | O | 3 | 2-I |
| 143 | O | 3 | 3-I |
| 144 | O | 3 | 4-I |
| 145 | O | 3 | 2-$CH_3$ |

| | | | |
|---|---|---|---|
| 146 | O | 3 | $3-CH_3$ |
| 147 | O | 3 | $4-CH_3$ |
| 148 | O | 3 | $2-CH_3CH_2$ |
| 149 | O | 3 | $3-CH_3CH_2$ |
| 150 | O | 3 | $4-CH_3CH_2$ |
| 151 | O | 3 | $2-CH_3CH_2CH_2$ |
| 152 | O | 3 | $3-CH_3CH_2CH_2$ |
| 153 | O | 3 | $4-CH_3CH_2CH_2$ |
| 154 | O | 3 | $2-(CH_3)_2CH$ |
| 155 | O | 3 | $3-(CH_3)_2CH$ |
| 156 | O | 3 | $4-(CH_3)_2CH$ |
| 157 | O | 3 | $2-CH_3(CH_2)_2CH_2$ |
| 158 | O | 3 | $3-CH_3(CH_2)_2CH_2$ |
| 159 | O | 3 | $4-CH_3(CH_2)_2CH_2$ |
| 160 | O | 3 | $2-(CH_3)_2CHCH_2$ |
| 161 | O | 3 | $3-(CH_3)_2CHCH_2$ |
| 162 | O | 3 | $4-(CH_3)_2CHCH_2$ |
| 163 | O | 3 | $2-CH_3CH_2CH(CH_3)$ |
| 164 | O | 3 | $3-CH_3CH_2CH(CH_3)$ |
| 165 | O | 3 | $4-CH_3CH_2CH(CH_3)$ |
| 166 | O | 3 | $2-(CH_3)_3C$ |
| 167 | O | 3 | $3-(CH_3)_3C$ |
| 168 | O | 3 | $4-(CH_3)_3C$ |
| 169 | O | 3 | $2-CH_3O$ |
| 170 | O | 3 | $3-CH_3O$ |
| 171 | O | 3 | $4-CH_3O$ |
| 172 | O | 3 | $2-CH_3CH_2O$ |
| 173 | O | 3 | $3-CH_3CH_2O$ |
| 174 | O | 3 | $4-CH_3CH_2O$ |

| | | | |
|---|---|---|---|
| 175 | O | 3 | $2-CH_3CH_2CH_2O$ |
| 176 | O | 3 | $3-CH_3CH_2CH_2O$ |
| 177 | O | 3 | $4-CH_3CH_2CH_2O$ |
| 178 | O | 3 | $2-(CH_3)_2CHO$ |
| 179 | O | 3 | $3-(CH_3)_2CHO$ |
| 180 | O | 3 | $4-(CH_3)_2CHO$ |
| 181 | O | 3 | $2-CH_3(CH_2)_2CH_2O$ |
| 182 | O | 3 | $3-CH_3(CH_2)_2CH_2O$ |
| 183 | O | 3 | $4-CH_3(CH_2)_2CH_2O$ |
| 184 | O | 3 | $2-(CH_3)_2CHCH_2O$ |
| 185 | O | 3 | $3-(CH_3)_2CHCH_2O$ |
| 186 | O | 3 | $4-(CH_3)_2CHCH_2O$ |
| 187 | O | 3 | $2-CH_3CH_2CH(CH_3)O$ |
| 188 | O | 3 | $3-CH_3CH_2CH(CH_3)O$ |
| 189 | O | 3 | $4-CH_3CH_2CH(CH_3)O$ |
| 190 | O | 3 | $2-(CH_3)_3CO$ |
| 191 | O | 3 | $3-(CH_3)_3CO$ |
| 192 | O | 3 | $4-(CH_3)_3CO$ |

Table-6

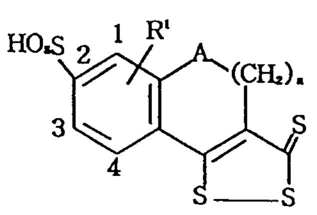

| Compound | A | n | $R^1$ |
|---|---|---|---|
| 1 | O | 1 | H |
| 2 | O | 1 | 1-OH |
| 3 | O | 1 | 3-OH |
| 4 | O | 1 | 4-OH |
| 5 | O | 1 | 1-F |
| 6 | O | 1 | 3-F |
| 7 | O | 1 | 4-F |
| 8 | O | 1 | 1-Cl |
| 9 | O | 1 | 3-Cl |
| 10 | O | 1 | 4-Cl |
| 11 | O | 1 | 1-Br |
| 12 | O | 1 | 3-Br |
| 13 | O | 1 | 4-Br |
| 14 | O | 1 | 1-I |
| 15 | O | 1 | 3-I |
| 16 | O | 1 | 4-I |
| 17 | O | 1 | 1-$CH_3$ |
| 18 | O | 1 | 3-$CH_3$ |
| 19 | O | 1 | 4-$CH_3$ |
| 20 | O | 1 | 1-$CH_3CH_2$ |
| 21 | O | 1 | 3-$CH_3CH_2$ |
| 22 | O | 1 | 4-$CH_3CH_2$ |
| 23 | O | 1 | 1-$CH_3CH_2CH_2$ |
| 24 | O | 1 | 3-$CH_3CH_2CH_2$ |
| 25 | O | 1 | 4-$CH_3CH_2CH_2$ |

| | | | |
|---|---|---|---|
| 26 | O | 1 | 1-$(CH_3)_2CH$ |
| 27 | O | 1 | 3-$(CH_3)_2CH$ |
| 28 | O | 1 | 4-$(CH_3)_2CH$ |
| 29 | O | 1 | 1-$CH_3(CH_2)_2CH_2$ |
| 30 | O | 1 | 3-$CH_3(CH_2)_2CH_2$ |
| 31 | O | 1 | 4-$CH_3(CH_2)_2CH_2$ |
| 32 | O | 1 | 1-$(CH_3)_2CHCH_2$ |
| 33 | O | 1 | 3-$(CH_3)_2CHCH_2$ |
| 34 | O | 1 | 4-$(CH_3)_2CHCH_2$ |
| 35 | O | 1 | 1-$CH_3CH_2CH(CH_3)$ |
| 36 | O | 1 | 3-$CH_3CH_2CH(CH_3)$ |
| 37 | O | 1 | 4-$CH_3CH_2CH(CH_3)$ |
| 38 | O | 1 | 1-$(CH_3)_3C$ |
| 39 | O | 1 | 3-$(CH_3)_3C$ |
| 40 | O | 1 | 4-$(CH_3)_3C$ |
| 41 | O | 1 | 1-$CH_3O$ |
| 42 | O | 1 | 3-$CH_3O$ |
| 43 | O | 1 | 4-$CH_3O$ |
| 44 | O | 1 | 1-$CH_3CH_2O$ |
| 45 | O | 1 | 3-$CH_3CH_2O$ |
| 46 | O | 1 | 4-$CH_3CH_2O$ |
| 47 | O | 1 | 1-$CH_3CH_2CH_2O$ |
| 48 | O | 1 | 3-$CH_3CH_2CH_2O$ |
| 49 | O | 1 | 4-$CH_3CH_2CH_2O$ |
| 50 | O | 1 | 1-$(CH_3)_2CHO$ |
| 51 | O | 1 | 3-$(CH_3)_2CHO$ |
| 52 | O | 1 | 4-$(CH_3)_2CHO$ |
| 53 | O | 1 | 1-$CH_3(CH_2)_2CH_2O$ |
| 54 | O | 1 | 3-$CH_3(CH_2)_2CH_2O$ |

| | | | |
|---|---|---|---|
| 55 | O | 1 | $4-CH_3(CH_2)_2CH_2O$ |
| 56 | O | 1 | $1-(CH_3)_2CHCH_2O$ |
| 57 | O | 1 | $3-(CH_3)_2CHCH_2O$ |
| 58 | O | 1 | $4-(CH_3)_2CHCH_2O$ |
| 59 | O | 1 | $1-CH_3CH_2CH(CH_3)O$ |
| 60 | O | 1 | $3-CH_3CH_2CH(CH_3)O$ |
| 61 | O | 1 | $4-CH_3CH_2CH(CH_3)O$ |
| 62 | O | 1 | $1-(CH_3)_3CO$ |
| 63 | O | 1 | $3-(CH_3)_3CO$ |
| 64 | O | 1 | $4-(CH_3)_3CO$ |
| 65 | O | 2 | H |
| 66 | O | 2 | 1-OH |
| 67 | O | 2 | 3-OH |
| 68 | O | 2 | 4-OH |
| 69 | O | 2 | 1-F |
| 70 | O | 2 | 3-F |
| 71 | O | 2 | 4-F |
| 72 | O | 2 | 1-Cl |
| 73 | O | 2 | 3-Cl |
| 74 | O | 2 | 4-Cl |
| 75 | O | 2 | 1-Br |
| 76 | O | 2 | 3-Br |
| 77 | O | 2 | 4-Br |
| 78 | O | 2 | 1-I |
| 79 | O | 2 | 3-I |
| 80 | O | 2 | 4-I |
| 81 | O | 2 | $1-CH_3$ |
| 82 | O | 2 | $3-CH_3$ |
| 83 | O | 2 | $4-CH_3$ |

| | | | |
|---|---|---|---|
| 84 | O | 2 | $1-CH_3CH_2$ |
| 85 | O | 2 | $3-CH_3CH_2$ |
| 86 | O | 2 | $4-CH_3CH_2$ |
| 87 | O | 2 | $1-CH_3CH_2CH_2$ |
| 88 | O | 2 | $3-CH_3CH_2CH_2$ |
| 89 | O | 2 | $4-CH_3CH_2CH_2$ |
| 90 | O | 2 | $1-(CH_3)_2CH$ |
| 91 | O | 2 | $3-(CH_3)_2CH$ |
| 92 | O | 2 | $4-(CH_3)_2CH$ |
| 93 | O | 2 | $1-CH_3(CH_2)_2CH_2$ |
| 94 | O | 2 | $3-CH_3(CH_2)_2CH_2$ |
| 95 | O | 2 | $4-CH_3(CH_2)_2CH_2$ |
| 96 | O | 2 | $1-(CH_3)_2CHCH_2$ |
| 97 | O | 2 | $3-(CH_3)_2CHCH_2$ |
| 98 | O | 2 | $4-(CH_3)_2CHCH_2$ |
| 99 | O | 2 | $1-CH_3CH_2CH(CH_3)$ |
| 100 | O | 2 | $3-CH_3CH_2CH(CH_3)$ |
| 101 | O | 2 | $4-CH_3CH_2CH(CH_3)$ |
| 102 | O | 2 | $1-(CH_3)_3C$ |
| 103 | O | 2 | $3-(CH_3)_3C$ |
| 104 | O | 2 | $4-(CH_3)_3C$ |
| 105 | O | 2 | $1-CH_3O$ |
| 106 | O | 2 | $3-CH_3O$ |
| 107 | O | 2 | $4-CH_3O$ |
| 108 | O | 2 | $1-CH_3CH_2O$ |
| 109 | O | 2 | $3-CH_3CH_2O$ |
| 110 | O | 2 | $4-CH_3CH_2O$ |
| 111 | O | 2 | $1-CH_3CH_2CH_2O$ |
| 112 | O | 2 | $3-CH_3CH_2CH_2O$ |

| | | | |
|---|---|---|---|
| 113 | O | 2 | 4-$CH_3CH_2CH_2O$ |
| 114 | O | 2 | 1-$(CH_3)_2CHO$ |
| 115 | O | 2 | 3-$(CH_3)_2CHO$ |
| 116 | O | 2 | 4-$(CH_3)_2CHO$ |
| 117 | O | 2 | 1-$CH_3(CH_2)_2CH_2O$ |
| 118 | O | 2 | 3-$CH_3(CH_2)_2CH_2O$ |
| 119 | O | 2 | 4-$CH_3(CH_2)_2CH_2O$ |
| 120 | O | 2 | 1-$(CH_3)_2CHCH_2O$ |
| 121 | O | 2 | 3-$(CH_3)_2CHCH_2O$ |
| 122 | O | 2 | 4-$(CH_3)_2CHCH_2O$ |
| 123 | O | 2 | 1-$CH_3CH_2CH(CH_3)O$ |
| 124 | O | 2 | 3-$CH_3CH_2CH(CH_3)O$ |
| 125 | O | 2 | 4-$CH_3CH_2CH(CH_3)O$ |
| 126 | O | 2 | 1-$(CH_3)_3CO$ |
| 127 | O | 2 | 3-$(CH_3)_3CO$ |
| 128 | O | 2 | 4-$(CH_3)_3CO$ |
| 129 | O | 3 | H |
| 130 | O | 3 | 1-OH |
| 131 | O | 3 | 3-OH |
| 132 | O | 3 | 4-OH |
| 133 | O | 3 | 1-F |
| 134 | O | 3 | 3-F |
| 135 | O | 3 | 4-F |
| 136 | O | 3 | 1-Cl |
| 137 | O | 3 | 3-Cl |
| 138 | O | 3 | 4-Cl |
| 139 | O | 3 | 1-Br |
| 140 | O | 3 | 3-Br |
| 141 | O | 3 | 4-Br |

| | | | |
|---|---|---|---|
| 142 | O | 3 | 1-I |
| 143 | O | 3 | 3-I |
| 144 | O | 3 | 4-I |
| 145 | O | 3 | $1-CH_3$ |
| 146 | O | 3 | $3-CH_3$ |
| 147 | O | 3 | $4-CH_3$ |
| 148 | O | 3 | $1-CH_3CH_2$ |
| 149 | O | 3 | $3-CH_3CH_2$ |
| 150 | O | 3 | $4-CH_3CH_2$ |
| 151 | O | 3 | $I-CH_3CH_2CH_2$ |
| 152 | O | 3 | $3-CH_3CH_2CH_2$ |
| 153 | O | 3 | $4-CH_3CH_2CH_2$ |
| 154 | O | 3 | $1-(CH_3)_2CH$ |
| 155 | O | 3 | $3-(CH_3)_2CH$ |
| 156 | O | 3 | $4-(CH_3)_2CH$ |
| 157 | O | 3 | $1-CH_3(CH_2)_2CH_2$ |
| 158 | O | 3 | $3-CH_3(CH_2)_2CH_2$ |
| 159 | O | 3 | $4-CH_3(CH_2)_2CH_2$ |
| 160 | O | 3 | $1-(CH_3)_2CHCH_2$ |
| 161 | O | 3 | $3-(CH_3)_2CHCH_2$ |
| 162 | O | 3 | $4-(CH_3)_2CHCH_2$ |
| 163 | O | 3 | $1-CH_3CH_2CH(CH_3)$ |
| 164 | O | 3 | $3-CH_3CH_2CH(CH_3)$ |
| 165 | O | 3 | $4-CH_3CH_2CH(CH_3)$ |
| 166 | O | 3 | $1-(CH_3)_3C$ |
| 167 | O | 3 | $3-(CH_3)_3C$ |
| 168 | O | 3 | $4-(CH_3)_3C$ |
| 169 | O | 3 | $1-CH_3O$ |
| 170 | O | 3 | $3-CH_3O$ |

| | | | |
|---|---|---|---|
| 171 | O | 3 | 4 − $CH_3O$ |
| 172 | O | 3 | 1 − $CH_3CH_2O$ |
| 173 | O | 3 | 3 − $CH_3CH_2O$ |
| 174 | O | 3 | 4 − $CH_3CH_2O$ |
| 175 | O | 3 | 1 − $CH_3CH_2CH_2O$ |
| 176 | O | 3 | 3 − $CH_3CH_2CH_2O$ |
| 177 | O | 3 | 4 − $CH_3CH_2CH_2O$ |
| 178 | O | 3 | 1 − $(CH_3)_2CHO$ |
| 179 | O | 3 | 3 − $(CH_3)_2CHO$ |
| 180 | O | 3 | 4 − $(CH_3)_2CHO$ |
| 181 | O | 3 | 1 − $CH_3(CH_2)_2CH_2O$ |
| 182 | O | 3 | 3 − $CH_3(CH_2)_2CH_2O$ |
| 183 | O | 3 | 4 − $CH_3(CH_2)_2CH_2O$ |
| 184 | O | 3 | 1 − $(CH_3)_2CHCH_2O$ |
| 185 | O | 3 | 3 − $(CH_3)_2CHCH_2O$ |
| 186 | O | 3 | 4 − $(CH_3)_2CHCH_2O$ |
| 187 | O | 3 | 1 − $CH_3CH_2CH(CH_3)O$ |
| 188 | O | 3 | 3 − $CH_3CH_2CH(CH_3)O$ |
| 189 | O | 3 | 4 − $CH_3CH_2CH(CH_3)O$ |
| 190 | O | 3 | 1 − $(CH_3)_3CO$ |
| 191 | O | 3 | 3 − $(CH_3)_3CO$ |
| 192 | O | 3 | 4 − $(CH_3)_3CO$ |

Table-7

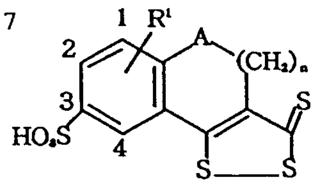

| Compound | A | n | $R^1$ |
|---|---|---|---|
| 1 | O | 1 | H |
| 2 | O | 1 | 1-OH |
| 3 | O | 1 | 2-OH |
| 4 | O | 1 | 4-OH |
| 5 | O | 1 | 1-F |
| 6 | O | 1 | 2-F |
| 7 | O | 1 | 4-F |
| 8 | O | 1 | 1-Cl |
| 9 | O | 1 | 2-Cl |
| 10 | O | 1 | 4-Cl |
| 11 | O | 1 | 1-Br |
| 12 | O | 1 | 2-Br |
| 13 | O | 1 | 4-Br |
| 14 | O | 1 | 1-I |
| 15 | O | 1 | 2-I |
| 16 | O | 1 | 4-I |
| 17 | O | 1 | 1-$CH_3$ |
| 18 | O | 1 | 2-$CH_3$ |
| 19 | O | 1 | 4-$CH_3$ |
| 20 | O | 1 | 1-$CH_3CH_2$ |

| | | | |
|---|---|---|---|
| 21 | O | 1 | 2 − $CH_3CH_2$ |
| 22 | O | 1 | 4 − $CH_3CH_2$ |
| 23 | O | 1 | 1 − $CH_3CH_2CH_2$ |
| 24 | O | 1 | 2 − $CH_3CH_2CH_2$ |
| 25 | O | 1 | 4 − $CH_3CH_2CH_2$ |
| 26 | O | 1 | 1 − $(CH_3)_2CH$ |
| 27 | O | 1 | 2 − $(CH_3)_2CH$ |
| 28 | O | 1 | 4 − $(CH_3)_2CH$ |
| 29 | O | 1 | 1 − $CH_3(CH_2)_2CH_2$ |
| 30 | O | 1 | 2 − $CH_3(CH_2)_2CH_2$ |
| 31 | O | 1 | 4 − $CH_3(CH_2)_2CH_2$ |
| 32 | O | 1 | 1 − $(CH_3)_2CHCH_2$ |
| 33 | O | 1 | 2 − $(CH_3)_2CHCH_2$ |
| 34 | O | 1 | 4 − $(CH_3)_2CHCH_2$ |
| 35 | O | 1 | 1 − $CH_3CH_2CH(CH_3)$ |
| 36 | O | 1 | 2 − $CH_3CH_2CH(CH_3)$ |
| 37 | O | 1 | 4 − $CH_3CH_2CH(CH_3)$ |
| 38 | O | 1 | 1 − $(CH_3)_3C$ |
| 39 | O | 1 | 2 − $(CH_3)_3C$ |
| 40 | O | 1 | 4 − $(CH_3)_3C$ |
| 41 | O | 1 | 1 − $CH_3O$ |
| 42 | O | 1 | 2 − $CH_3O$ |
| 43 | O | 1 | 4 − $CH_3O$ |
| 44 | O | 1 | 1 − $CH_3CH_2O$ |
| 45 | O | 1 | 2 − $CH_3CH_2O$ |
| 46 | O | 1 | 4 − $CH_3CH_2O$ |
| 47 | O | 1 | 1 − $CH_3CH_2CH_2O$ |
| 48 | O | 1 | 2 − $CH_3CH_2CH_2O$ |
| 49 | O | 1 | 4 − $CH_3CH_2CH_2O$ |

| | | | |
|---|---|---|---|
| 50 | O | 1 | 1−$(CH_3)_2CHO$ |
| 51 | O | 1 | 2−$(CH_3)_2CHO$ |
| 52 | O | 1 | 4−$(CH_3)_2CHO$ |
| 53 | O | 1 | 1−$CH_3(CH_2)_2CH_2O$ |
| 54 | O | 1 | 2−$CH_3(CH_2)_2CH_2O$ |
| 55 | O | 1 | 4−$CH_3(CH_2)_2CH_2O$ |
| 56 | O | 1 | 1−$(CH_3)_2CHCH_2O$ |
| 57 | O | 1 | 2−$(CH_3)_2CHCH_2O$ |
| 58 | O | 1 | 4−$(CH_3)_2CHCH_2O$ |
| 59 | O | 1 | 1−$CH_3CH_2CH(CH_3)O$ |
| 60 | O | 1 | 2−$CH_3CH_2CH(CH_3)O$ |
| 61 | O | 1 | 4−$CH_3CH_2CH(CH_3)O$ |
| 62 | O | 1 | 1−$(CH_3)_3CO$ |
| 63 | O | 1 | 2−$(CH_3)_3CO$ |
| 64 | O | 1 | 4−$(CH_3)_3CO$ |
| 65 | O | 2 | H |
| 66 | O | 2 | 1−OH |
| 67 | O | 2 | 2−OH |
| 68 | O | 2 | 4−OH |
| 69 | O | 2 | 1−F |
| 70 | O | 2 | 2−F |
| 71 | O | 2 | 4−F |
| 72 | O | 2 | 1−Cl |
| 73 | O | 2 | 2−Cl |
| 74 | O | 2 | 4−Cl |
| 75 | O | 2 | 1−Br |
| 76 | O | 2 | 2−Br |
| 77 | O | 2 | 4−Br |
| 78 | O | 2 | 1−I |

| | | | |
|---|---|---|---|
| 79 | O | 2 | 2 − I |
| 80 | O | 2 | 4 − I |
| 81 | O | 2 | 1 − $CH_3$ |
| 82 | O | 2 | 2 − $CH_3$ |
| 83 | O | 2 | 4 − $CH_3$ |
| 84 | O | 2 | 1 − $CH_3CH_2$ |
| 85 | O | 2 | 2 − $CH_3CH_2$ |
| 86 | O | 2 | 4 − $CH_3CH_2$ |
| 87 | O | 2 | 1 − $CH_3CH_2CH_2$ |
| 88 | O | 2 | 2 − $CH_3CH_2CH_2$ |
| 89 | O | 2 | 4 − $CH_3CH_2CH_2$ |
| 90 | O | 2 | 1 − $(CH_3)_2CH$ |
| 91 | O | 2 | 2 − $(CH_3)_2CH$ |
| 92 | O | 2 | 4 − $(CH_3)_2CH$ |
| 93 | O | 2 | 1 − $CH_3(CH_2)_2CH_2$ |
| 94 | O | 2 | 2 − $CH_3(CH_2)_2CH_2$ |
| 95 | O | 2 | 4 − $CH_3(CH_2)_2CH_2$ |
| 96 | O | 2 | 1 − $(CH_3)_2CHCH_2$ |
| 97 | O | 2 | 2 − $(CH_3)_2CHCH_2$ |
| 98 | O | 2 | 4 − $(CH_3)_2CHCH_2$ |
| 99 | O | 2 | 1 − $CH_3CH_2CH(CH_3)$ |
| 100 | O | 2 | 2 − $CH_3CH_2CH(CH_3)$ |
| 101 | O | 2 | 4 − $CH_3CH_2CH(CH_3)$ |
| 102 | O | 2 | 1 − $(CH_3)_3C$ |
| 103 | O | 2 | 2 − $(CH_3)_3C$ |
| 104 | O | 2 | 4 − $(CH_3)_3C$ |
| 105 | O | 2 | 1 − $CH_3O$ |
| 106 | O | 2 | 2 − $CH_3O$ |
| 107 | O | 2 | 4 − $CH_3O$ |

| | | | |
|---|---|---|---|
| 108 | O | 2 | 1-$CH_3CH_2O$ |
| 109 | O | 2 | 2-$CH_3CH_2O$ |
| 110 | O | 2 | 4-$CH_3CH_2O$ |
| 111 | O | 2 | 1-$CH_3CH_2CH_2O$ |
| 112 | O | 2 | 2-$CH_3CH_2CH_2O$ |
| 113 | O | 2 | 4-$CH_3CH_2CH_2O$ |
| 114 | O | 2 | 1-$(CH_3)_2CHO$ |
| 115 | O | 2 | 2-$(CH_3)_2CHO$ |
| 116 | O | 2 | 4-$(CH_3)_2CHO$ |
| 117 | O | 2 | 1-$CH_3(CH_2)_2CH_2O$ |
| 118 | O | 2 | 2-$CH_3(CH_2)_2CH_2O$ |
| 119 | O | 2 | 4-$CH_3(CH_2)_2CH_2O$ |
| 120 | O | 2 | 1-$(CH_3)_2CHCH_2O$ |
| 121 | O | 2 | 2-$(CH_3)_2CHCH_2O$ |
| 122 | O | 2 | 4-$(CH_3)_2CHCH_2O$ |
| 123 | O | 2 | 1-$CH_3CH_2CH(CH_3)O$ |
| 124 | O | 2 | 2-$CH_3CH_2CH(CH_3)O$ |
| 125 | O | 2 | 4-$CH_3CH_2CH(CH_3)O$ |
| 126 | O | 2 | 1-$(CH_3)_3CO$ |
| 127 | O | 2 | 2-$(CH_3)_3CO$ |
| 128 | O | 2 | 4-$(CH_3)_3CO$ |
| 129 | O | 3 | H |
| 130 | O | 3 | 1-OH |
| 131 | O | 3 | 2-OH |
| 132 | O | 3 | 4-OH |
| 133 | O | 3 | 1-F |
| 134 | O | 3 | 2-F |
| 135 | O | 3 | 4-F |
| 136 | O | 3 | 1-Cl |

| | | | |
|---|---|---|---|
| 137 | O | 3 | 2-Cl |
| 138 | O | 3 | 4-Cl |
| 139 | O | 3 | 1-Br |
| 140 | O | 3 | 2-Br |
| 141 | O | 3 | 4-Br |
| 142 | O | 3 | 1-I |
| 143 | O | 3 | 2-I |
| 144 | O | 3 | 4-I |
| 145 | O | 3 | 1-$CH_3$ |
| 146 | O | 3 | 2-$CH_3$ |
| 147 | O | 3 | 4-$CH_3$ |
| 148 | O | 3 | 1-$CH_3CH_2$ |
| 149 | O | 3 | 2-$CH_3CH_2$ |
| 150 | O | 3 | 4-$CH_3CH_2$ |
| 151 | O | 3 | 1-$CH_3CH_2CH_2$ |
| 152 | O | 3 | 2-$CH_3CH_2CH_2$ |
| 153 | O | 3 | 4-$CH_3CH_2CH_2$ |
| 154 | O | 3 | 1-$(CH_3)_2CH$ |
| 155 | O | 3 | 2-$(CH_3)_2CH$ |
| 156 | O | 3 | 4-$(CH_3)_2CH$ |
| 157 | O | 3 | 1-$CH_3(CH_2)_2CH_2$ |
| 158 | O | 3 | 2-$CH_3(CH_2)_2CH_2$ |
| 159 | O | 3 | 4-$CH_3(CH_2)_2CH_2$ |
| 160 | O | 3 | 1-$(CH_3)_2CHCH_2$ |
| 161 | O | 3 | 2-$(CH_3)_2CHCH_2$ |
| 162 | O | 3 | 4-$(CH_3)_2CHCH_2$ |
| 163 | O | 3 | 1-$CH_3CH_2CH(CH_3)$ |
| 164 | O | 3 | 2-$CH_3CH_2CH(CH_3)$ |
| 165 | O | 3 | 4-$CH_3CH_2CH(CH_3)$ |

| | | | |
|---|---|---|---|
| 166 | O | 3 | 1−$(CH_3)_3C$ |
| 167 | O | 3 | 2−$(CH_3)_3C$ |
| 168 | O | 3 | 4−$(CH_3)_3C$ |
| 169 | O | 3 | 1−$CH_3O$ |
| 170 | O | 3 | 2−$CH_3O$ |
| 171 | O | 3 | 4−$CH_3O$ |
| 172 | O | 3 | 1−$CH_3CH_2O$ |
| 173 | O | 3 | 2−$CH_3CH_2O$ |
| 174 | O | 3 | 4−$CH_3CH_2O$ |
| 175 | O | 3 | 1−$CH_3CH_2CH_2O$ |
| 176 | O | 3 | 2−$CH_3CH_2CH_2O$ |
| 177 | O | 3 | 4−$CH_3CH_2CH_2O$ |
| 178 | O | 3 | 1−$(CH_3)_2CHO$ |
| 179 | O | 3 | 2−$(CH_3)_2CHO$ |
| 180 | O | 3 | 4−$(CH_3)_2CHO$ |
| 181 | O | 3 | 1−$CH_3(CH_2)_2CH_2O$ |
| 182 | O | 3 | 2−$CH_3(CH_2)_2CH_2O$ |
| 183 | O | 3 | 4−$CH_3(CH_2)_2CH_2O$ |
| 184 | O | 3 | 1−$(CH_3)_2CHCH_2O$ |
| 185 | O | 3 | 2−$(CH_3)_2CHCH_2O$ |
| 186 | O | 3 | 4−$(CH_3)_2CHCH_2O$ |
| 187 | O | 3 | 1−$CH_3CH_2CH(CH_3)O$ |
| 188 | O | 3 | 2−$CH_3CH_2CH(CH_3)O$ |
| 189 | O | 3 | 4−$CH_3CH_2CH(CH_3)O$ |
| 190 | O | 3 | 1−$(CH_3)_3CO$ |
| 191 | O | 3 | 2−$(CH_3)_3CO$ |
| 192 | O | 3 | 4−$(CH_3)_3CO$ |

Table-8

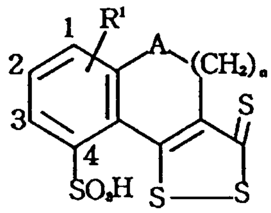

| Compound | A | n | $R^1$ |
|---|---|---|---|
| 1 | O | 1 | H |
| 2 | O | 1 | 1-OH |
| 3 | O | 1 | 2-OH |
| 4 | O | 1 | 3-OH |
| 5 | O | 1 | 1-F |
| 6 | O | 1 | 2-F |
| 7 | O | 1 | 3-F |
| 8 | O | 1 | 1-Cl |
| 9 | O | 1 | 2-Cl |
| 10 | O | 1 | 3-Cl |
| 11 | O | 1 | 1-Br |
| 12 | O | 1 | 2-Br |
| 13 | O | 1 | 3-Br |
| 14 | O | 1 | 1-I |
| 15 | O | 1 | 2-I |
| 16 | O | 1 | 3-I |

| | | | |
|---|---|---|---|
| 17 | O | 1 | 1 − $CH_3$ |
| 18 | O | 1 | 2 − $CH_3$ |
| 19 | O | 1 | 3 − $CH_3$ |
| 20 | O | 1 | 1 − $CH_3CH_2$ |
| 21 | O | 1 | 2 − $CH_3CH_2$ |
| 22 | O | 1 | 3 − $CH_3CH_2$ |
| 23 | O | 1 | 1 − $CH_3CH_2CH_2$ |
| 24 | O | 1 | 2 − $CH_3CH_2CH_2$ |
| 25 | O | 1 | 3 − $CH_3CH_2CH_2$ |
| 26 | O | 1 | 1 − $(CH_3)_2CH$ |
| 27 | O | 1 | 2 − $(CH_3)_2CH$ |
| 28 | O | 1 | 3 − $(CH_3)_2CH$ |
| 29 | O | 1 | 1 − $CH_3(CH_2)_2CH_2$ |
| 30 | O | 1 | 2 − $CH_3(CH_2)_2CH_2$ |
| 31 | O | 1 | 3 − $CH_3(CH_2)_2CH_2$ |
| 32 | O | 1 | 1 − $(CH_3)_2CHCH_2$ |
| 33 | O | 1 | 2 − $(CH_3)_2CHCH_2$ |
| 34 | O | 1 | 3 − $(CH_3)_2CHCH_2$ |
| 35 | O | 1 | 1 − $CH_3CH_2CH(CH_3)$ |
| 36 | O | 1 | 2 − $CH_3CH_2CH(CH_3)$ |
| 37 | O | 1 | 3 − $CH_3CH_2CH(CH_3)$ |
| 38 | O | 1 | 1 − $(CH_3)_3C$ |
| 39 | O | 1 | 2 − $(CH_3)_3C$ |
| 40 | O | 1 | 3 − $(CH_3)_3C$ |
| 41 | O | 1 | 1 − $CH_3O$ |
| 42 | O | 1 | 2 − $CH_3O$ |
| 43 | O | 1 | 3 − $CH_3O$ |
| 44 | O | 1 | 1 − $CH_3CH_2O$ |
| 45 | O | 1 | 2 − $CH_3CH_2O$ |

| | | | |
|---|---|---|---|
| 46 | O | 1 | 3−$CH_3CH_2O$ |
| 47 | O | 1 | 1−$CH_3CH_2CH_2O$ |
| 48 | O | 1 | 2−$CH_3CH_2CH_2O$ |
| 49 | O | 1 | 3−$CH_3CH_2CH_2O$ |
| 50 | O | 1 | 1−$(CH_3)_2CHO$ |
| 51 | O | 1 | 2−$(CH_3)_2CHO$ |
| 52 | O | 1 | 3−$(CH_3)_2CHO$ |
| 53 | O | 1 | 1−$CH_3(CH_2)_2CH_2O$ |
| 54 | O | 1 | 2−$CH_3(CH_2)_2CH_2O$ |
| 55 | O | 1 | 3−$CH_3(CH_2)_2CH_2O$ |
| 56 | O | 1 | 1−$(CH_3)_2CHCH_2O$ |
| 57 | O | 1 | 2−$(CH_3)_2CHCH_2O$ |
| 58 | O | 1 | 3−$(CH_3)_2CHCH_2O$ |
| 59 | O | 1 | 1−$CH_3CH_2CH(CH_3)O$ |
| 60 | O | 1 | 2−$CH_3CH_2CH(CH_3)O$ |
| 61 | O | 1 | 3−$CH_3CH_2CH(CH_3)O$ |
| 62 | O | 1 | 1−$(CH_3)_3CO$ |
| 63 | O | 1 | 2−$(CH_3)_3CO$ |
| 64 | O | 1 | 3−$(CH_3)_3CO$ |
| 65 | O | 2 | H |
| 66 | O | 2 | 1−OH |
| 67 | O | 2 | 2−OH |
| 68 | O | 2 | 3−OH |
| 69 | O | 2 | 1−F |
| 70 | O | 2 | 2−F |
| 71 | O | 2 | 3−F |
| 72 | O | 2 | 1−Cl |
| 73 | O | 2 | 2−Cl |
| 74 | O | 2 | 3−Cl |

| | | | |
|---|---|---|---|
| 75 | O | 2 | 1-Br |
| 76 | O | 2 | 2-Br |
| 77 | O | 2 | 3-Br |
| 78 | O | 2 | 1-I |
| 79 | O | 2 | 2-I |
| 80 | O | 2 | 3-I |
| 81 | O | 2 | 1-$CH_3$ |
| 82 | O | 2 | 2-$CH_3$ |
| 83 | O | 2 | 3-$CH_3$ |
| 84 | O | 2 | 1-$CH_3CH_2$ |
| 85 | O | 2 | 2-$CH_3CH_2$ |
| 86 | O | 2 | 3-$CH_3CH_2$ |
| 87 | O | 2 | 1-$CH_3CH_2CH_2$ |
| 88 | O | 2 | 2-$CH_3CH_2CH_2$ |
| 89 | O | 2 | 3-$CH_3CH_2CH_2$ |
| 90 | O | 2 | 1-$(CH_3)_2CH$ |
| 91 | O | 2 | 2-$(CH_3)_2CH$ |
| 92 | O | 2 | 3-$(CH_3)_2CH$ |
| 93 | O | 2 | 1-$CH_3(CH_2)_2CH_2$ |
| 94 | O | 2 | 2-$CH_3(CH_2)_2CH_2$ |
| 95 | O | 2 | 3-$CH_3(CH_2)_2CH_2$ |
| 96 | O | 2 | 1-$(CH_3)_2CHCH_2$ |
| 97 | O | 2 | 2-$(CH_3)_2CHCH_2$ |
| 98 | O | 2 | 3-$(CH_3)_2CHCH_2$ |
| 99 | O | 2 | 1-$CH_3CH_2CH(CH_3)$ |
| 100 | O | 2 | 2-$CH_3CH_2CH(CH_3)$ |
| 101 | O | 2 | 3-$CH_3CH_2CH(CH_3)$ |
| 102 | O | 2 | 1-$(CH_3)_3C$ |
| 103 | O | 2 | 2-$(CH_3)_3C$ |

| | | | |
|---|---|---|---|
| 104 | O | 2 | 3-$(CH_3)_3C$ |
| 105 | O | 2 | 1-$CH_3O$ |
| 106 | O | 2 | 2-$CH_3O$ |
| 107 | O | 2 | 3-$CH_3O$ |
| 108 | O | 2 | 1-$CH_3CH_2O$ |
| 109 | O | 2 | 2-$CH_3CH_2O$ |
| 110 | O | 2 | 3-$CH_3CH_2O$ |
| 111 | O | 2 | 1-$CH_3CH_2CH_2O$ |
| 112 | O | 2 | 2-$CH_3CH_2CH_2O$ |
| 113 | O | 2 | 3-$CH_3CH_2CH_2O$ |
| 114 | O | 2 | 1-$(CH_3)_2CHO$ |
| 115 | O | 2 | 2-$(CH_3)_2CHO$ |
| 116 | O | 2 | 3-$(CH_3)_2CHO$ |
| 117 | O | 2 | 1-$CH_3(CH_2)_2CH_2O$ |
| 118 | O | 2 | 2-$CH_3(CH_2)_2CH_2O$ |
| 119 | O | 2 | 3-$CH_3(CH_2)_2CH_2O$ |
| 120 | O | 2 | 1-$(CH_3)_2CHCH_2O$ |
| 121 | O | 2 | 2-$(CH_3)_2CHCH_2O$ |
| 122 | O | 2 | 3-$(CH_3)_2CHCH_2O$ |
| 123 | O | 2 | 1-$CH_3CH_2CH(CH_3)O$ |
| 124 | O | 2 | 2-$CH_3CH_2CH(CH_3)O$ |
| 125 | O | 2 | 3-$CH_3CH_2CH(CH_3)O$ |
| 126 | O | 2 | 1-$(CH_3)_3CO$ |
| 127 | O | 2 | 2-$(CH_3)_3CO$ |
| 128 | O | 2 | 3-$(CH_3)_3CO$ |
| 129 | O | 3 | H |
| 130 | O | 3 | 1-OH |
| 131 | O | 3 | 2-OH |
| 132 | O | 3 | 3-OH |

| | | | |
|---|---|---|---|
| 133 | O | 3 | 1-F |
| 134 | O | 3 | 2-F |
| 135 | O | 3 | 3-F |
| 136 | O | 3 | 1-Cl |
| 137 | O | 3 | 2-Cl |
| 138 | O | 3 | 3-Cl |
| 139 | O | 3 | 1-Br |
| 140 | O | 3 | 2-Br |
| 141 | O | 3 | 3-Br |
| 142 | O | 3 | 1-I |
| 143 | O | 3 | 2-I |
| 144 | O | 3 | 3-I |
| 145 | O | 3 | 1-$CH_3$ |
| 146 | O | 3 | 2-$CH_3$ |
| 147 | O | 3 | 3-$CH_3$ |
| 148 | O | 3 | 1-$CH_3CH_2$ |
| 149 | O | 3 | 2-$CH_3CH_2$ |
| 150 | O | 3 | 3-$CH_3CH_2$ |
| 151 | O | 3 | 1-$CH_3CH_2CH_2$ |
| 152 | O | 3 | 2-$CH_3CH_2CH_2$ |
| 153 | O | 3 | 3-$CH_3CH_2CH_2$ |
| 154 | O | 3 | 1-$(CH_3)_2CH$ |
| 155 | O | 3 | 2-$(CH_3)_2CH$ |
| 156 | O | 3 | 3-$(CH_3)_2CH$ |
| 157 | O | 3 | 1-$CH_3(CH_2)_2CH_2$ |
| 158 | O | 3 | 2-$CH_3(CH_2)_2CH_2$ |
| 159 | O | 3 | 3-$CH_3(CH_2)_2CH_2$ |
| 160 | O | 3 | 1-$(CH_3)_2CHCH_2$ |
| 161 | O | 3 | 2-$(CH_3)_2CHCH_2$ |

| | | | |
|---|---|---|---|
| 162 | O | 3 | 3-$(CH_3)_2CHCH_2$ |
| 163 | O | 3 | 1-$CH_3CH_2CH(CH_3)$ |
| 164 | O | 3 | 2-$CH_3CH_2CH(CH_3)$ |
| 165 | O | 3 | 3-$CH_3CH_2CH(CH_3)$ |
| 166 | O | 3 | 1-$(CH_3)_3C$ |
| 167 | O | 3 | 2-$(CH_3)_3C$ |
| 168 | O | 3 | 3-$(CH_3)_3C$ |
| 169 | O | 3 | 1-$CH_3O$ |
| 170 | O | 3 | 2-$CH_3O$ |
| 171 | O | 3 | 3-$CH_3O$ |
| 172 | O | 3 | 1-$CH_3CH_2O$ |
| 173 | O | 3 | 2-$CH_3CH_2O$ |
| 174 | O | 3 | 3-$CH_3CH_2O$ |
| 175 | O | 3 | 1-$CH_3CH_2CH_2O$ |
| 176 | O | 3 | 2-$CH_3CH_2CH_2O$ |
| 177 | O | 3 | 3-$CH_3CH_2CH_2O$ |
| 178 | O | 3 | 1-$(CH_3)_2CHO$ |
| 179 | O | 3 | 2-$(CH_3)_2CHO$ |
| 180 | O | 3 | 3-$(CH_3)_2CHO$ |
| 181 | O | 3 | 1-$CH_3(CH_2)_2CH_2O$ |
| 182 | O | 3 | 2-$CH_3(CH_2)_2CH_2O$ |
| 183 | O | 3 | 3-$CH_3(CH_2)_2CH_2O$ |
| 184 | O | 3 | 1-$(CH_3)_2CHCH_2O$ |
| 185 | O | 3 | 2-$(CH_3)_2CHCH_2O$ |
| 186 | O | 3 | 3-$(CH_3)_2CHCH_2O$ |
| 187 | O | 3 | 1-$CH_3CH_2CH(CH_3)O$ |
| 188 | O | 3 | 2-$CH_3CH_2CH(CH_3)O$ |
| 189 | O | 3 | 3-$CH_3CH_2CH(CH_3)O$ |
| 190 | O | 3 | 1-$(CH_3)_3CO$ |

| | | | |
|---|---|---|---|
| 191 | O | 3 | 2 − $(CH_3)_3CO$ |
| 192 | O | 3 | 3 − $(CH_3)_3CO$ |

Table-9

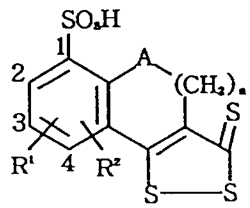

| Compound | A | n | $R^1$ | $R^2$ |
|---|---|---|---|---|
| 1 | $CH_2$ | 0 | 2 − OH | 3 − OH |
| 2 | $CH_2$ | 0 | 2 − OH | 4 − OH |
| 3 | $CH_2$ | 0 | 3 − OH | 4 − OH |
| 4 | $CH_2$ | 0 | 2 − $CH_3$ | 3 − $CH_3$ |
| 5 | $CH_2$ | 0 | 2 − $CH_3$ | 4 − $CH_3$ |
| 6 | $CH_2$ | 0 | 3 − $CH_3$ | 4 − $CH_3$ |
| 7 | $CH_2$ | 0 | 2 − $CH_3O$ | 3 − $CH_3O$ |
| 8 | $CH_2$ | 0 | 2 − $CH_3O$ | 4 − $CH_3O$ |
| 9 | $CH_2$ | 0 | 3 − $CH_3O$ | 4 − $CH_3O$ |
| 10 | $CH_2$ | 1 | 2 − OH | 3 − OH |
| 11 | $CH_2$ | 1 | 2 − OH | 4 − OH |
| 12 | $CH_2$ | 1 | 3 − OH | 4 − OH |

| | | | | |
|---|---|---|---|---|
| 13 | $CH_2$ | 1 | $2-CH_3$ | $3-CH_3$ |
| 14 | $CH_2$ | 1 | $2-CH_3$ | $4-CH_3$ |
| 15 | $CH_2$ | 1 | $3-CH_3$ | $4-CH_3$ |
| 16 | $CH_2$ | 1 | $2-CH_3O$ | $3-CH_3O$ |
| 17 | $CH_2$ | 1 | $2-CH_3O$ | $4-CH_3O$ |
| 18 | $CH_2$ | 1 | $3-CH_3O$ | $4-CH_3O$ |
| 19 | $CH_2$ | 2 | $2-OH$ | $3-OH$ |
| 20 | $CH_2$ | 2 | $2-OH$ | $4-OH$ |
| 21 | $CH_2$ | 2 | $3-OH$ | $4-OH$ |
| 22 | $CH_2$ | 2 | $2-CH_3$ | $3-CH_3$ |
| 23 | $CH_2$ | 2 | $2-CH_3$ | $4-CH_3$ |
| 24 | $CH_2$ | 2 | $3-CH_3$ | $4-CH_3$ |
| 25 | $CH_2$ | 2 | $2-CH_3O$ | $3-CH_3O$ |
| 26 | $CH_2$ | 2 | $2-CH_3O$ | $4-CH_3O$ |
| 27 | $CH_2$ | 2 | $3-CH_3O$ | $4-CH_3O$ |
| 28 | $CH_2$ | 3 | $2-OH$ | $3-OH$ |
| 29 | $CH_2$ | 3 | $2-OH$ | $4-OH$ |
| 30 | $CH_2$ | 3 | $3-OH$ | $4-OH$ |
| 31 | $CH_2$ | 3 | $2-CH_3$ | $3-CH_3$ |
| 32 | $CH_2$ | 3 | $2-CH_3$ | $4-CH_3$ |
| 33 | $CH_2$ | 3 | $3-CH_3$ | $4-CH_3$ |
| 34 | $CH_2$ | 3 | $2-CH_3O$ | $3-CH_3O$ |
| 35 | $CH_2$ | 3 | $2-CH_3O$ | $4-CH_3O$ |
| 36 | $CH_2$ | 3 | $3-CH_3O$ | $4-CH_3O$ |
| 37 | O | 1 | $2-OH$ | $3-OH$ |
| 38 | O | 1 | $2-OH$ | $4-OH$ |
| 39 | O | 1 | $3-OH$ | $4-OH$ |
| 40 | O | 1 | $2-CH_3$ | $3-CH_3$ |
| 41 | O | 1 | $2-CH_3$ | $4-CH_3$ |

| | | | | |
|---|---|---|---|---|
| 42 | O | 1 | $3-CH_3$ | $4-CH_3$ |
| 43 | O | 1 | $2-CH_3O$ | $3-CH_3O$ |
| 44 | O | 1 | $2-CH_3O$ | $4-CH_3O$ |
| 45 | O | 1 | $3-CH_3O$ | $4-CH_3O$ |
| 46 | O | 2 | $2-OH$ | $3-OH$ |
| 47 | O | 2 | $2-OH$ | $4-OH$ |
| 48 | O | 2 | $3-OH$ | $4-OH$ |
| 49 | O | 2 | $2-CH_3$ | $3-CH_3$ |
| 50 | O | 2 | $2-CH_3$ | $4-CH_3$ |
| 51 | O | 2 | $3-CH_3$ | $4-CH_3$ |
| 52 | O | 2 | $2-CH_3O$ | $3-CH_3O$ |
| 53 | O | 2 | $2-CH_3O$ | $4-CH_3O$ |
| 54 | O | 2 | $3-CH_3O$ | $4-CH_3O$ |
| 55 | O | 3 | $2-OH$ | $3-OH$ |
| 56 | O | 3 | $2-OH$ | $4-OH$ |
| 57 | O | 3 | $3-OH$ | $4-OH$ |
| 58 | O | 3 | $2-CH_3$ | $3-CH_3$ |
| 59 | O | 3 | $2-CH_3$ | $4-CH_3$ |
| 60 | O | 3 | $3-CH_3$ | $4-CH_3$ |
| 61 | O | 3 | $2-CH_3O$ | $3-CH_3O$ |
| 62 | O | 3 | $2-CH_3O$ | $4-CH_3O$ |
| 63 | O | 3 | $3-CH_3O$ | $4-CH_3O$ |

Table-10

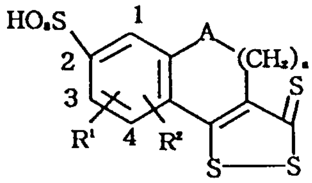

| Compound | A | n | $R^1$ | $R^2$ |
|---|---|---|---|---|
| 1 | $CH_2$ | 0 | 1−OH | 3−OH |
| 2 | $CH_2$ | 0 | 1−OH | 4−OH |
| 3 | $CH_2$ | 0 | 3−OH | 4−OH |
| 4 | $CH_2$ | 0 | 1−$CH_3$ | 3−$CH_3$ |
| 5 | $CH_2$ | 0 | 1−$CH_3$ | 4−$CH_3$ |
| 6 | $CH_2$ | 0 | 3−$CH_3$ | 4−$CH_3$ |
| 7 | $CH_2$ | 0 | 1−$CH_3O$ | 3−$CH_3O$ |
| 8 | $CH_2$ | 0 | 1−$CH_3O$ | 4−$CH_3O$ |
| 9 | $CH_2$ | 0 | 3−$CH_3O$ | 4−$CH_3O$ |
| 10 | $CH_2$ | 1 | 1−OH | 3−OH |
| 11 | $CH_2$ | 1 | 1−OH | 4−OH |
| 12 | $CH_2$ | 1 | 3−OH | 4−OH |
| 13 | $CH_2$ | 1 | 1−$CH_3$ | 3−$CH_3$ |
| 14 | $CH_2$ | 1 | 1−$CH_3$ | 4−$CH_3$ |
| 15 | $CH_2$ | 1 | 3−$CH_3$ | 4−$CH_3$ |
| 16 | $CH_2$ | 1 | 1−$CH_3O$ | 3−$CH_3O$ |
| 17 | $CH_2$ | 1 | 1−$CH_3O$ | 4−$CH_3O$ |
| 18 | $CH_2$ | 1 | 3−$CH_3O$ | 4−$CH_3O$ |
| 19 | $CH_2$ | 2 | 1−OH | 3−OH |
| 20 | $CH_2$ | 2 | 1−OH | 4−OH |

| | | | | |
|---|---|---|---|---|
| 21 | $CH_2$ | 2 | 3-OH | 4-OH |
| 22 | $CH_2$ | 2 | 1-$CH_3$ | 3-$CH_3$ |
| 23 | $CH_2$ | 2 | 1-$CH_3$ | 4-$CH_3$ |
| 24 | $CH_2$ | 2 | 3-$CH_3$ | 4-$CH_3$ |
| 25 | $CH_2$ | 2 | 1-$CH_3O$ | 3-$CH_3O$ |
| 26 | $CH_2$ | 2 | 1-$CH_3O$ | 4-$CH_3O$ |
| 27 | $CH_2$ | 2 | 3-$CH_3O$ | 4-$CH_3O$ |
| 28 | $CH_2$ | 3 | 1-OH | 3-OH |
| 29 | $CH_2$ | 3 | 1-OH | 4-OH |
| 30 | $CH_2$ | 3 | 3-OH | 4-OH |
| 31 | $CH_2$ | 3 | 1-$CH_3$ | 3-$CH_3$ |
| 32 | $CH_2$ | 3 | 1-$CH_3$ | 4-$CH_3$ |
| 33 | $CH_2$ | 3 | 3-$CH_3$ | 4-$CH_3$ |
| 34 | $CH_2$ | 3 | 1-$CH_3O$ | 3-$CH_3O$ |
| 35 | $CH_2$ | 3 | 1-$CH_3O$ | 4-$CH_3O$ |
| 36 | $CH_2$ | 3 | 3-$CH_3O$ | 4-$CH_3O$ |
| 37 | O | 1 | 1-OH | 3-OH |
| 38 | O | 1 | 1-OH | 4-OH |
| 39 | O | 1 | 3-OH | 4-OH |
| 40 | O | 1 | 1-$CH_3$ | 3-$CH_3$ |
| 41 | O | 1 | 1-$CH_3$ | 4-$CH_3$ |
| 42 | O | 1 | 3-$CH_3$ | 4-$CH_3$ |
| 43 | O | 1 | 1-$CH_3O$ | 3-$CH_3O$ |
| 44 | O | 1 | 1-$CH_3O$ | 4-$CH_3O$ |
| 45 | O | 1 | 3-$CH_3O$ | 4-$CH_3O$ |
| 46 | O | 2 | 1-OH | 3-OH |
| 47 | O | 2 | 1-OH | 4-OH |
| 48 | O | 2 | 3-OH | 4-OH |
| 49 | O | 2 | 1-$CH_3$ | 3-$CH_3$ |

| | | | | |
|---|---|---|---|---|
| 50 | O | 2 | 1-$CH_3$ | 4-$CH_3$ |
| 51 | O | 2 | 3-$CH_3$ | 4-$CH_3$ |
| 52 | O | 2 | 1-$CH_3O$ | 3-$CH_3O$ |
| 53 | O | 2 | 1-$CH_3O$ | 4-$CH_3O$ |
| 54 | O | 2 | 3-$CH_3O$ | 4-$CH_3O$ |
| 55 | O | 3 | 1-OH | 3-OH |
| 56 | O | 3 | 1-OH | 4-OH |
| 57 | O | 3 | 3-OH | 4-OH |
| 58 | O | 3 | 1-$CH_3$ | 3-$CH_3$ |
| 59 | O | 3 | 1-$CH_3$ | 4-$CH_3$ |
| 60 | O | 3 | 3-$CH_3$ | 4-$CH_3$ |
| 61 | O | 3 | 1-$CH_3O$ | 3-$CH_3O$ |
| 62 | O | 3 | 1-$CH_3O$ | 4-$CH_3O$ |
| 63 | O | 3 | 3-$CH_3O$ | 4-$CH_3O$ |

Table-11

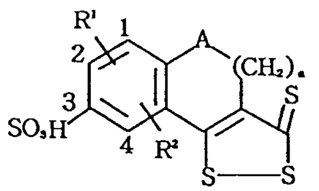

| Compound | A | n | $R^1$ | $R^2$ |
|---|---|---|---|---|

| | | | | |
|---|---|---|---|---|
| 1 | $CH_2$ | 0 | 1-OH | 2-OH |
| 2 | $CH_2$ | 0 | 1-OH | 4-OH |
| 3 | $CH_2$ | 0 | 2-OH | 4-OH |
| 4 | $CH_2$ | 0 | 1-$CH_3$ | 2-$CH_3$ |
| 5 | $CH_2$ | 0 | 1-$CH_3$ | 4-$CH_3$ |
| 6 | $CH_2$ | 0 | 2-$CH_3$ | 4-$CH_3$ |
| 7 | $CH_2$ | 0 | 1-$CH_3O$ | 2-$CH_3O$ |
| 8 | $CH_2$ | 0 | 1-$CH_3O$ | 4-$CH_3O$ |
| 9 | $CH_2$ | 0 | 2-$CH_3O$ | 4-$CH_3O$ |
| 10 | $CH_2$ | 1 | 1-OH | 2-OH |
| 11 | $CH_2$ | 1 | 1-OH | 4-OH |
| 12 | $CH_2$ | 1 | 2-OH | 4-OH |
| 13 | $CH_2$ | 1 | 1-$CH_3$ | 2-$CH_3$ |
| 14 | $CH_2$ | 1 | 1-$CH_3$ | 4-$CH_3$ |
| 15 | $CH_2$ | 1 | 2-$CH_3$ | 4-$CH_3$ |
| 16 | $CH_2$ | 1 | 1-$CH_3O$ | 2-$CH_3O$ |
| 17 | $CH_2$ | 1 | 1-$CH_3O$ | 4-$CH_3O$ |
| 18 | $CH_2$ | 1 | 2-$CH_3O$ | 4-$CH_3O$ |
| 19 | $CH_2$ | 2 | 1-OH | 2-OH |
| 20 | $CH_2$ | 2 | 1-OH | 4-OH |
| 21 | $CH_2$ | 2 | 2-OH | 4-OH |
| 22 | $CH_2$ | 2 | 1-$CH_3$ | 2-$CH_3$ |
| 23 | $CH_2$ | 2 | 1-$CH_3$ | 4-$CH_3$ |
| 24 | $CH_2$ | 2 | 2-$CH_3$ | 4-$CH_3$ |
| 25 | $CH_2$ | 2 | 1-$CH_3O$ | 2-$CH_3O$ |
| 26 | $CH_2$ | 2 | 1-$CH_3O$ | 4-$CH_3O$ |
| 27 | $CH_2$ | 2 | 2-$CH_3O$ | 4-$CH_3O$ |
| 28 | $CH_2$ | 3 | 1-OH | 2-OH |
| 29 | $CH_2$ | 3 | 1-OH | 4-OH |

| | | | | |
|---|---|---|---|---|
| 30 | $CH_2$ | 3 | 2-OH | 4-OH |
| 31 | $CH_2$ | 3 | 1-$CH_3$ | 2-$CH_3$ |
| 32 | $CH_2$ | 3 | 1-$CH_3$ | 4-$CH_3$ |
| 33 | $CH_2$ | 3 | 2-$CH_3$ | 4-$CH_3$ |
| 34 | $CH_2$ | 3 | 1-$CH_3O$ | 2-$CH_3O$ |
| 35 | $CH_2$ | 3 | 1-$CH_3O$ | 4-$CH_3O$ |
| 36 | $CH_2$ | 3 | 2-$CH_3O$ | 4-$CH_3O$ |
| 37 | O | 1 | 1-OH | 2-OH |
| 38 | O | 1 | 1-OH | 4-OH |
| 39 | O | 1 | 2-OH | 4-OH |
| 40 | O | 1 | 1-$CH_3$ | 2-$CH_3$ |
| 41 | O | 1 | 1-$CH_3$ | 4-$CH_3$ |
| 42 | O | 1 | 2-$CH_3$ | 4-$CH_3$ |
| 43 | O | 1 | 1-$CH_3O$ | 2-$CH_3O$ |
| 44 | O | 1 | 1-$CH_3O$ | 4-$CH_3O$ |
| 45 | O | 1 | 2-$CH_3O$ | 4-$CH_3O$ |
| 46 | O | 2 | 1-OH | 2-OH |
| 47 | O | 2 | 1-OH | 4-OH |
| 48 | O | 2 | 2-OH | 4-OH |
| 49 | O | 2 | 1-$CH_3$ | 2-$CH_3$ |
| 50 | O | 2 | 1-$CH_3$ | 4-$CH_3$ |
| 51 | O | 2 | 2-$CH_3$ | 4-$CH_3$ |
| 52 | O | 2 | 1-$CH_3O$ | 2-$CH_3O$ |
| 53 | O | 2 | 1-$CH_3O$ | 4-$CH_3O$ |
| 54 | O | 2 | 2-$CH_3O$ | 4-$CH_3O$ |
| 55 | O | 3 | 1-OH | 2-OH |
| 56 | O | 3 | 1-OH | 4-OH |
| 57 | O | 3 | 2-OH | 4-OH |
| 58 | O | 3 | 1-$CH_3$ | 2-$CH_3$ |

| | | | | |
|---|---|---|---|---|
| 59 | O | 3 | 1 − $CH_3$ | 4 − $CH_3$ |
| 60 | O | 3 | 2 − $CH_3$ | 4 − $CH_3$ |
| 61 | O | 3 | 1 − $CH_3O$ | 2 − $CH_3O$ |
| 62 | O | 3 | 1 − $CH_3O$ | 4 − $CH_3O$ |
| 63 | O | 3 | 2 − $CH_3O$ | 4 − $CH_3O$ |

Table-12

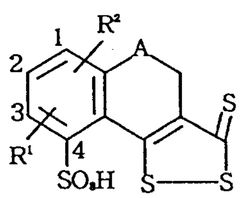

| Compound | A | n | $R^1$ | $R^2$ |
|---|---|---|---|---|
| 1 | $CH_2$ | 0 | 1 − OH | 2 − OH |
| 2 | $CH_2$ | 0 | 1 − OH | 3 − OH |
| 3 | $CH_2$ | 0 | 2 − OH | 3 − OH |
| 4 | $CH_2$ | 0 | 1 − $CH_3$ | 2 − $CH_3$ |
| 5 | $CH_2$ | 0 | 1 − $CH_3$ | 3 − $CH_3$ |
| 6 | $CH_2$ | 0 | 2 − $CH_3$ | 3 − $CH_3$ |
| 7 | $CH_2$ | 0 | 1 − $CH_3O$ | 2 − $CH_3O$ |
| 8 | $CH_2$ | 0 | 1 − $CH_3O$ | 3 − $CH_3O$ |
| 9 | $CH_2$ | 0 | 2 − $CH_3O$ | 3 − $CH_3O$ |

| | | | | |
|---|---|---|---|---|
| 10 | $CH_2$ | 1 | 1-OH | 2-OH |
| 11 | $CH_2$ | 1 | 1-OH | 3-OH |
| 12 | $CH_2$ | 1 | 2-OH | 3-OH |
| 13 | $CH_2$ | 1 | 1-$CH_3$ | 2-$CH_3$ |
| 14 | $CH_2$ | 1 | 1-$CH_3$ | 3-$CH_3$ |
| 15 | $CH_2$ | 1 | 2-$CH_3$ | 3-$CH_3$ |
| 16 | $CH_2$ | 1 | 1-$CH_3O$ | 2-$CH_3O$ |
| 17 | $CH_2$ | 1 | 1-$CH_3O$ | 3-$CH_3O$ |
| 18 | $CH_2$ | 1 | 2-$CH_3O$ | 3-$CH_3O$ |
| 19 | $CH_2$ | 2 | 1-OH | 2-OH |
| 20 | $CH_2$ | 2 | 1-OH | 3-OH |
| 21 | $CH_2$ | 2 | 2-OH | 3-OH |
| 22 | $CH_2$ | 2 | 1-$CH_3$ | 2-$CH_3$ |
| 23 | $CH_2$ | 2 | 1-$CH_3$ | 3-$CH_3$ |
| 24 | $CH_2$ | 2 | 2-$CH_3$ | 3-$CH_3$ |
| 25 | $CH_2$ | 2 | 1-$CH_3O$ | 2-$CH_3O$ |
| 26 | $CH_2$ | 2 | 1-$CH_3O$ | 3-$CH_3O$ |
| 27 | $CH_2$ | 2 | 2-$CH_3O$ | 3-$CH_3O$ |
| 28 | $CH_2$ | 3 | 1-OH | 2-OH |
| 29 | $CH_2$ | 3 | 1-OH | 3-OH |
| 30 | $CH_2$ | 3 | 2-OH | 3-OH |
| 31 | $CH_2$ | 3 | 1-$CH_3$ | 2-$CH_3$ |
| 32 | $CH_2$ | 3 | 1-$CH_3$ | 3-$CH_3$ |
| 33 | $CH_2$ | 3 | 2-$CH_3$ | 3-$CH_3$ |
| 34 | $CH_2$ | 3 | 1-$CH_3O$ | 2-$CH_3O$ |
| 35 | $CH_2$ | 3 | 1-$CH_3O$ | 3-$CH_3O$ |
| 36 | $CH_2$ | 3 | 2-$CH_3O$ | 3-$CH_3O$ |
| 37 | O | 1 | 1-OH | 2-OH |
| 38 | O | 1 | 1-OH | 3-OH |

| | | | | |
|---|---|---|---|---|
| 39 | O | 1 | 2-OH | 3-OH |
| 40 | O | 1 | 1-$CH_3$ | 2-$CH_3$ |
| 41 | O | 1 | 1-$CH_3$ | 3-$CH_3$ |
| 42 | O | 1 | 2-$CH_3$ | 3-$CH_3$ |
| 43 | O | 1 | 1-$CH_3O$ | 2-$CH_3O$ |
| 44 | O | 1 | 1-$CH_3O$ | 3-$CH_3O$ |
| 45 | O | 1 | 2-$CH_3O$ | 3-$CH_3O$ |
| 46 | O | 2 | 1-OH | 2-OH |
| 47 | O | 2 | 1-OH | 3-OH |
| 48 | O | 2 | 2-OH | 3-OH |
| 49 | O | 2 | 1-$CH_3$ | 2-$CH_3$ |
| 50 | O | 2 | 1-$CH_3$ | 3-$CH_3$ |
| 51 | O | 2 | 2-$CH_3$ | 3-$CH_3$ |
| 52 | O | 2 | 1-$CH_3O$ | 2-$CH_3O$ |
| 53 | O | 2 | 1-$CH_3O$ | 3-$CH_3O$ |
| 54 | O | 2 | 2-$CH_3O$ | 3-$CH_3O$ |
| 55 | O | 3 | 1-OH | 2-OH |
| 56 | O | 3 | 1-OH | 3-OH |
| 57 | O | 3 | 2-OH | 3-OH |
| 58 | O | 3 | 1-$CH_3$ | 2-$CH_3$ |
| 59 | O | 3 | 1-$CH_3$ | 3-$CH_3$ |
| 60 | O | 3 | 2-$CH_3$ | 3-$CH_3$ |
| 61 | O | 3 | 1-$CH_3O$ | 2-$CH_3O$ |
| 62 | O | 3 | 1-$CH_3O$ | 3-$CH_3O$ |
| 63 | O | 3 | 2-$CH_3O$ | 3-$CH_3O$ |

A typical process for producing the compound of general formula (1) is described below.

The compound of general formula (1) can be produced, for example, by sulfonating a compound represented by general formula (2)

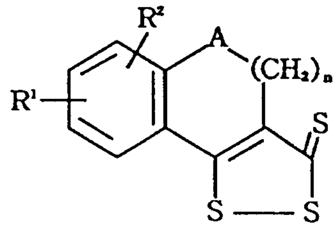

(2)

wherein A, $R^1$, $R^2$ and n are the same as defined above.

The sulfonating agent includes sulfuric acid, fuming sulfuric acid, sulfur trioxide, a sulfur trioxide complex, chlorosulfuric acid, fluorosulfuric acid, amidosulfuric acid, etc. The sulfur trioxide complex is a complex of sulfur trioxide with an ether, an amine or a Lewis acid (e.g. sulfide), and includes N,N-dimethylformamide-sulfur trioxide complex, dioxane-sulfur trioxide complex, pyridine-sulfur trioxide complex, triethylamine-sulfur trioxide complex, trimethylamine-sulfur trioxide complex, etc. The reaction is conducted in the presence or absence of a solvent. The solvent can be exemplified by chlorine-containing organic solvents (e.g. dichloromethane, chloroform, carbon tetrachloride, dichloroethane and tetrachloroethane), liquid sulfur dioxide, carbon disulfide, acetic acid, acetic anhydride, esters (e.g. ethyl acetate), ethers (e.g. diethyl ether), benzene, toluene, xylene, nitromethane, nitrobenzene, n-butyl alcohol, isobutyl alcohol, tert-butyl alcohol, acetonitrile, tetrahydrofuran, dioxane, dimethylformamide, dimethylacetamide and 1,3-dimethyl-2-imidazolidinone. The reaction temperature is -50 to 200 °C, and the reaction time is a few minutes to 10 hours. Incidentally, the compound of general formula (1) can also be produced by reacting the compound of general formula (2) with chlorosulfuric acid under the same conditions as above to form a sulfonyl chloride and then hydrolyzing it.

The compound of general formula (2) used as a raw material in the production of the compound of general formula (1), can be produced, for example, by Production Process 1 and Production Process 2 both shown below.

Production Process 1

A ketone represented by general formula (3)

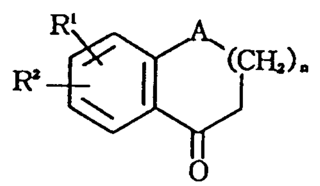

(3)

(wherein A, $R^1$, $R^2$ and n are the same as defined above) is reacted with carbon disulfide and a compound represented by general formula (4)

$R^3$-$W^1$ (4)

(wherein $R^3$ is a lower alkyl group of 1-4 carbon atoms, and $W^1$ is a halogen atom or a residue of a sulfuric acid or sulfonic acid ester) or general formula (5)

$W^1$-B-$W^1$ (5)

(wherein B is an alkylene group of 2-4 carbon atoms, and $W^1$ is the same as defined above) in the presence of an appropriate base to form a compound represented by general formula (6)

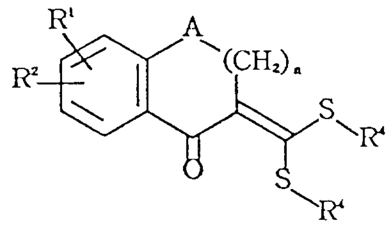

(6)

(wherein A, $R^1$, $R^2$ and n are the same as defined above, and two $R^4$s are each a lower alkyl group of 1-4 carbon atoms or bond to each other to form an alkylene group of 2-4 carbon atoms). Then, the compound of general formula (6) is reacted with phosphorus pentasulfide, whereby a compound of general formula (2) can be produced.

The first-step reaction is conducted generally in a solvent such as benzene, toluene, xylene, dimethyl sulfoxide or the like. The reaction temperature is -20 to 100 °C, preferably -10 °C to room temperature, and the reaction time is a few to 24 hours. The appropriate base includes, for example, sodium hydride, metal alcholates (e.g. sodium methoxide, sodium ethoxide and sodium tert-amyloxide) and hydroxides (e.g. sodium hydroxide, potassium hydroxide and ammonium hydroxide). The compound of general formula (6) can be produced also by the process described in Tetrahedron Letter, 43, 4207 (1973).

The second-step reaction is carried out by generally using an organic solvent inert to phosphorus pentasulfide, such as benzene, toluene, xylene, pyridine, chlorobenzene, dioxane or the like. The reaction temperature is room temperature to 200 °C and the reaction time is a few tens of minutes to 12 hours. Phosphorus pentasulfide is used in an amount of about 1-5 moles, preferably 1-3 moles per mole of the ketone.

Production Process 2

A 3-oxoester represented by general formula (7)

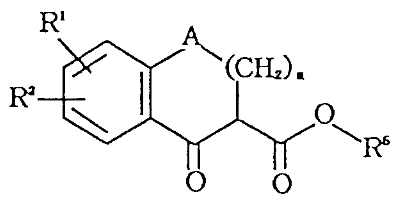

(7)

(wherein A, $R^1$, $R^2$ and n are the same as defined above, and $R^5$ is a lower alkyl group of 1-4 carbon atoms) is cyclized with a thionating agent, for example, phosphorus pentasulfide or the Lawson reagent [2,4-bis(4-methoxyphenyl)-1,3-dithia-2,4-diphosphethane-2,4-disulfide], whereby a compound of general formula (2) can be produced. In the reaction, the thionating agent is used in an amount of about 1-5 moles, preferably 1-3 moles per mole of the 3-oxoester.

Phosphorus pentasulfide and the Lawson reagent may simultaneously be added at any ratio, or powdery sulfur may be added to the reaction mixture. The amount of powdery sulfur added is about 0.5-5 moles per mole of the 3-oxoester. The solvent used in the reaction is an organic solvent inert to the thionating agent, such as benzene, toluene, xylene, pyridine, dioxane, chlorobenzene, carbon disulfide or the like. The reaction is carried out at 0-200 °C for a few tens of minutes to 12 hours.

The compound represented by general formula (7) can be produced from the compound represented by general formula (3), for example, by the process described in J. Am. Chem. Soc., 64, 94 (1942), J. Org. Chem., 33, 2457 (1968) or Chem. Pharm. Bull., 36, 401 (1988).

A compound of general formula (3) wherein A is an oxygen atom, can be produced, for example, by the process described in The Chemistry of Heterocyclic Compounds, A Series of Monographs, Vol. 31, Chromenes, Chromanones, and Chromones, Chap. V, 227 (1977), Indian J. Chem., 13, 1 (1975) or J. Med. Chem., 33, 445 (1990).

The compound represented by general formula (2) can also be produced via an intermediate of enamine form as described in J. Org. Chem., 56, 5360 (1991), etc., in the Production Process 1 or 2.

The thus obtained compound of general formula (1) can be isolated and purified by an ordinary method such as recrystallization, column chromatography or the like.

The compound of general formula (1) can be converted into its salt by treating said compound with a metal hydroxide (e.g. lithium hydroxide, sodium hydroxide, potassium hydroxide, calcium hydroxide or aluminum hydroxide), a metal carbonate (e.g. sodium carbonate or potassium carbonate), an organic amine (e.g. trimethylamine, triethylamine, piperidine or morpholine), an amino acid, ammonium hydroxide, ammonia or an ion exchange resin according to an ordinary method.

The second aspect of the present invention lies in a compound represented by general formula (11)

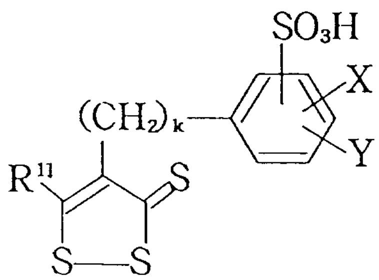

(11)

[wherein k is an integer of 0-5; X and Y are independently a hydrogen atom, a lower alkyl group or a lower alkoxy group; $R^{11}$ is an alkyl group or a group represented by general formula (12)

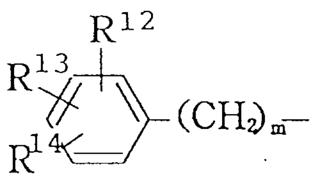

(12)

(wherein m is an integer of 0-4; and $R^{12}$, $R^{13}$ and $R^{14}$ are independently a hydrogen atom, a lower alkyl group or a lower alkoxy group; however, a case is excluded in which both k and m are zero, the sulfo group bonds to the 3-position, X is a 4-methoxy group, and $R^{12}$, $R^{13}$, $R^{14}$ and Y are each a hydrogen atom)], or a salt thereof; a therapeutic or preventive agent for diseases associated with peroxylipid, which contains said compound or salt as an active ingredient; and a process for producing said compound.

In Bull. Soc. Chim,, Fr., 84 (1955), there is a description on 4-(4-methoxy-3-sulfophenyl)-5-phenyl-1,2-dithiole-3-thione as a 1,2-dithiole-3-thione derivative having a sulfo group, simply for the interest of synthesis. However, the description makes no mention of the pharmacological activity of the compound.

Description is made below on the compound represented by general formula (11).

The lower alkyl group is, for example, an alkyl group of 1-4 carbon atoms and can be exemplified by methyl group, ethyl group, n-propyl group, isopropyl group, n-butyl group, isobutyl group, sec-butyl group and tert-butyl group.

The alkyl group is, for example, a straight-chain, branched-chain or cyclic alkyl group of 1-18 carbon atoms, preferably 3-18 carbon atoms and can be exemplified by the above-mentioned lower alkyl groups, n-pentyl group, isopentyl group, tert-pentyl group, neopentyl group, cyclopentyl group, hexyl group, cyclohexyl group, heptyl group, methylcyclohexyl group, octyl group, nonyl group, decyl group, undecyl group, dodecyl group, tridecyl group, tetradecyl group, pentadecyl group, hexadecyl group, heptadecyl group and octadecyl group.

The lower alkoxy group is, for example, an alkoxy group of 1-4 carbon atoms and can be exemplified by methoxy group, ethoxy group, n-propoxy group, isopropoxy group, allyloxy group, n-butoxy group, isobutoxy group, sec-butoxy group and tert-butoxy group.

The salt of the compound of general formula (11) can be any pharmacologically acceptable salt and can be exemplified by inorganic (e.g. lithium, sodium, potassium, calcium and aluminum) salts and salts with organic amines (e.g. ammonia, trimethylamine, triethylamine, piperidine and morpholine) or with amino acids.

A typical process for producing the compound of general formula (11) is described below.

The compound represented by general formula (11) can be produced, for example, by producing a compound represented by general formula (16)

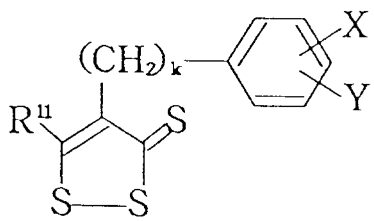

(16)

(wherein k, X, Y and $R^{11}$ are the same as defined above) by one of Production Processes 11, 12, 13, etc. and sulfonating the compound of general formula (16), for example, by Production Process 15.

Production Process 11

A ketone represented by general formula (18)

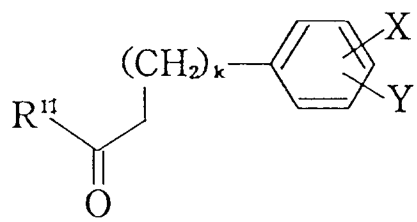

(18)

[wherein k is an integer of 0-5; X and Y are independently a hydrogen atom, a lower alkyl group or a lower alkoxy group; $R^{11}$ is an alkyl group or a group represented by general formula (12)

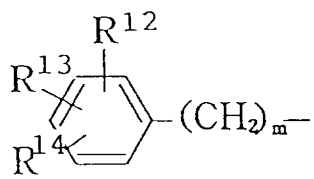

(12)

(wherein m is an integer of 0-4; and $R^{12}$, $R^{13}$ and $R^{14}$ are independently a hydrogen atom, a lower alkyl group or a lower alkoxy group; however, a case is excluded in which both k and m are zero, the sulfo group bonds to the 3-position, X is a 4-methoxy group, and $R^{12}$, $R^{13}$, $R^{14}$ and Y are each a hydrogen atom)] is reacted with carbon disulfide and a compound represented by general formula (20)

$R^{16}$-$W^1$ (20)

(wherein $R^{16}$ is a lower alkyl group of 1-4 carbon atoms, and $W^1$ is a halogen atom or a residue of a sulfuric acid or sulfonic acid ester) or general formula (21)

$W^1$-A-$W^1$ (21)

(wherein A is an alkylene group of 2-4 carbon atoms, and $W^1$ is the same as defined above) in the presence of an appropriate base to form a compound represented by general formula (22)

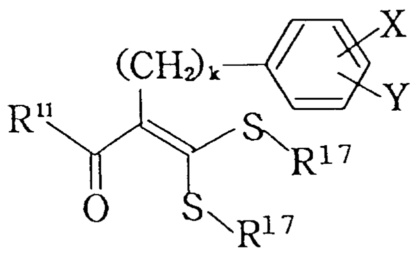

(22)

(wherein k, X, Y and $R^{11}$ are the same as defined above, and two $R^{17}$s are each a lower alkyl group of 1-4 carbon atoms or bond to each other to form an alkylene group of 2-4 carbon atoms). Then, the compound of general formula (22) is reacted with phosphorus pentasulfide, whereby a compound of general formula (16) can be produced.

The first-step reaction is conducted generally in a solvent such as benzene, toluene, xylene, dimethyl sulfoxide or the like. The reaction temperature is -20 to 100 °C and the reaction time is a few to 24 hours. The appropriate base includes, for example, sodium hydride, metal alcholates (e.g. sodium methoxide, sodium ethoxide and sodium tert-amyloxide), and hydroxides (e.g. sodium hydroxide, potassium hydroxide and ammonium hydroxide),

The second-step reaction is carried out by generally using an organic solvent inert to phosphorus pentasulfide, such as benzene, toluene, xylene, pyridine, chlorobenzene, dioxane or the like. The reaction temperature is room temperature to 200 °C and the reaction time is a few tens of minutes to 12 hours.

In the reaction, phosphorus pentasulfide is used in an amount of about 1-5 moles, preferably 1-3 moles per mole of the ketone.

The compound represented by general formula (18) can be produced, for example, by a reaction between an acid chloride and a Grignard reagent.

Production Process 12

A 3-oxoester represented by general formula (24)

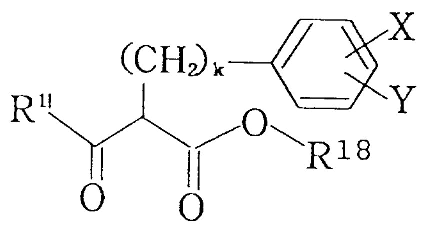

(24)

[wherein k, X, Y and $R^{11}$ are the same as defined with respect to general formula (18), and $R^{18}$ is a lower alkyl group of 1-4 carbon atoms] is cyclized with a thionating agent, for example, phosphorus pentasulfide or the Lawson reagent [2,4-bis(4-methoxyphenyl)-1,3-dithia-2,4-diphosphethane-2,4-disulfide], whereby a compound of general formula (16) can be produced. In the reaction, the thionating agent is used in an amount of about 1-5 moles, preferably 1-3 moles per mole of the 3-oxoester.

Phosphorus pentasulfide and the Lawson reagent may simultaneously be added at any ratio, or powdery sulfur may be added to the reaction mixture. The amount of powdery sulfur added is about 0.5-5 moles per mole of the 3-oxoester. The solvent used in the reaction is an organic solvent inert to the thionating agent, such as benzene, toluene, xylene, pyridine, chlorobenzene or the like. The reaction is carried out at 0-200 °C for a few tens of minutes to 12 hours.

The 3-oxoester of general formula (24) can be produced by reacting a 3-oxoester represented by general formula (26)

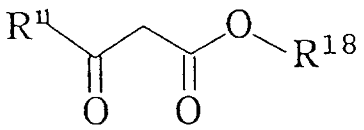

(26)

[wherein $R^{11}$ is the same as defined with respect to general formula (18) and $R^{18}$ is the same as defined with respect to general formula (24)] with a compound represented by general formula (28)

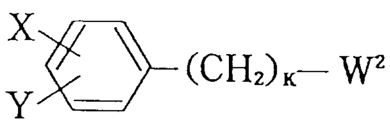

(28)

[wherein k, X and Y are the same as defined with respect to general formula (18); and $W^2$ is a halogen atom or a residue of sulfuric acid or sulfonic acid ester] in the presence of an appropriate base. The reaction is conducted in a solvent such as ether (e.g. acetone or diethyl ether), benzene, toluene, xylene, dimethylformamide, dimethyl sulfoxide, alcohol (e.g. methanol or ethanol) or the like. The reaction temperature is -5 °C to the boiling point of the solvent, and the reaction time is a few to 48 hours. The appropriate base includes sodium hydride; metal alcholates such as sodium methoxide, sodium ethoxide, magnesium methoxide, magnesium ethoxide and the like; alkali metal carbonates such as sodium carbonate, potassium carbonate and the like; and alkali metal iodides such as sodium iodide, potassium iodide and the like.

The 3-oxoester represented by general formula (26) can be produced, for example, by using the process described in J. Org. Chem., 43, 2087 (1978) or J. Chem Soc., 2407 (1963) or the conditions ordinarily used in the Claisen condensation.

Production Process 13

A compound of general formula (16) wherein $R^{11}$ is a compound of general formula (12) and both m and k are zero, can be produced, for example, by the process described in Bull. Soc. Chim. France, 84 (1955).

Production Process 15

The sulfonating agent used for the sulfonation of the compound represented by general formula (16) includes sulfuric acid, fuming sulfuric acid, sulfur trioxide, a sulfur trioxide complex, chlorosulfuric acid, fluorosulfuric acid, amidosulfuric acid, etc. The sulfur trioxide complex is a complex of sulfur trioxide with an ether, an amine or a Lewis acid (e.g. sulfide), and includes N,N-dimethylformamide-sulfur trioxide complex, dioxane-sulfur trioxide complex, pyridine-sulfur trioxide complex, triethylamine-sulfur trioxide complex, trimethylamine-sulfur trioxide complex, etc. The reaction is conducted in the presence or absence of a solvent. The solvent can be exemplified by chlorine-containing organic solvents (e.g. dichloromethane, chloroform, carbon tetrachloride, dichloroethane and tetrachloroethane), liquid sulfur dioxide, carbon disulfide, acetic acid, acetic anhydride, esters (e.g. ethyl acetate), ethers (e.g. diethyl ether), benzene, toluene, xylene, nitromethane, nitrobenzene, n-butyl alcohol, isobutyl alcohol, tert-butyl alcohol, acetonitrile, tetrahydrofuran, dioxane, dimethylformamide, dimethylacetamide and 1,3-dimethyl-2-imidazolidinone. The reaction temperature is -20 to 150 °C, and the reaction time is a few minutes to 10 hours. Incidentally, the compound of general formula (11) can also be produced by reacting the compound of general formula (16) with chlorosulfuric acid under the same conditions as above to form a sulfonyl chloride and then hydrolyzing it.

The thus obtained compound of general formula (11) can be isolated and purified by an ordinary method such as recrystallization, column chromatography or the like.

The compound of general formula (11) can be converted into its salt by treating said compound with a metal hydroxide (e.g. lithium hydroxide, sodium hydroxide, potassium hydroxide, calcium hydroxide or aluminum hydroxide), a metal carbonate (e.g. sodium carbonate or potassium carbonate), an organic amine (e.g. trimethylamine, triethylamine, piperidine or morpholine), ammonium hydroxide, ammonia or an ion exchange resin according to an ordinary method.

The compound represented by general formula (1) or (11) or the salt thereof can be made into pharmaceutical preparations by using a carrier, an excipient and other additives all ordinarily used in pharmaceutical preparations, and can be administered orally as tablets, granules, a powder, a suspension, capsules, a syrup, etc., or non-orally as an injection, suppositories, an isotonic solution for transfusion, etc.

For example, when said compound or salt thereof is made into tablets, there are used, as an adsorbent, crystalline cellulose, light silicic acid anhydride, etc. and, as an excipient, corn starch, lactose, calcium phosphate, magnesium stearate, etc. When an injection is made, there are used, as necessary, a pH-adjusting agent, a buffer solution, a stabilizing agent, a preservative, a solubilizing agent, etc.

The dose of the active ingredient [the compound of general formula (1) or (11) or the salt thereof] is appropriately determined depending upon, for example, the administration route and the weight, age and disease condition of patient. For example, in the case of a patient of myocardial infarction, intravenous injection is made continuously from right after urgent hospitalization to a few to several hours after the operation for the reopening of obstructed coronaria and, as necessary, individual administrations are preferably combined therewith. The active ingredient is administered at a dose of 0.1-500 mg per adult in each individual administration, and at a dose of 0.1-100 mg per minute per adult in continuous administration.

The present invention is hereinafter described in more detail with reference to Production Examples, Reference Examples, Evaluation Examples and Test Examples. However, the present invention is in no way restricted thereto.

Production Example 1

Production of 6-methoxy-7-sulfo-3H,4H-indeno[3,2-c]-1,2-dithiole-3-thione sodium salt (sodium salt of compound No. 42 in Table 3)

(1) Synthesis of 2-bis(methylthio)methylidene-5-methoxy-1-indanone

A mixture of 2.05 g of carbon disulfide and 4.38 g of 5-methoxy-1-indanone was dropwise added, at 5-10°C in 10 minutes, to a toluene (70 ml) solution containing sodium tert-amyloxide prepared from 4.76 g of tert-amyl alcohol and 2.17 g of sodium hydride (about 60%, in oil). The resulting mixture was stirred at room temperature for 1 hour. The reaction mixture was kept at 0-5°C, and 7.65 g of methyl iodide was dropwise added thereto in 20 minutes. The mixture was stirred at room temperature for 2.5 hours. To the reaction mixture was added 50 ml of water. The mixture was extracted with ethyl acetate. The organic layer was washed with a saturated aqueous sodium chloride solution, dried over anhydrous magnesium sulfate, and concentrated. The residue was purified by the use of a column to obtain 6.76 g of an intended product.

1H NMR ($CDCl_3$, $\delta$)

2.51 (3H, s), 2.56 (3H, s), 3.81 (2H, s), 3.88 (3H, s), 6.89-6.92 (2H, m), 7.75 (1H, d).

(2) Synthesis of 6-methoxy-4H,4H-indeno[3,2-c]-1,2-dithiole-3-thione

An o-xylene (20 ml) solution containing 6.50 g of 2-bis(methylthio)methylidene-5-methoxy-1-indanone was dropwise added, in 10 minutes, to an o-xylene (70 ml) suspension containing 8.66 g of phosphorus pentasulfide, being refluxed with heating. The mixture was stirred at the same temperature for 1 hour. Thereto was added chloroform. The mixture was washed with a saturated aqueous sodium chloride solution. The organic layer was dried over anhydrous sodium sulfate and concentrated. The residue was purified by the use of a column to obtain 1.63 g of an intended product.

1H NMR ($CDCl_3$, $\delta$)

3.72 (2H, s), 3.91 (3H, s), 6.99 (1H, dd), 7.11 (1H, d), 7.65 (1H, d).

(3) Synthesis of 6-methoxy-7-sulfo-3H,4H-indeno[3,2-c]-1,2-dithiole-3-thione sodium salt

4 ml of chlorosulfuric acid was added, at 0°C, to 1.01 g of 6-methoxy-3H,4H-indeno[3,2-c]-1,2-dithiole-3-thione. The mixture was stirred at the same temperature for 1 hour. The reaction mixture was poured into ice water. Thereto was added sodium carbonate to allow the mixture to have a pH of 10. Methanol was added thereto. The resulting precipitate (an inorganic salt) was removed by filtration. The filtrate was concentrated. The residue was purified by the use of a reversed-phase column to obtain 0.18 g of an intended product.

1H NMR ($DMSO-d_6$)

3.72 (2H, s), 3.88 (3H, s), 7.32 (1H, s), 8.17 (1H, s)

Production Example 2

Production of 6-methoxy-5-sulfo-3H,4H-indeno[3,2-c]-1,2-dithiole-3-thione sodium salt (sodium salt of compound No. 41 in Table 1)

4 ml of chlorosulfuric acid was added, at 0 ° C, to 1.01 g of 6-methoxy-3H,4H-indeno[3,2-c]-1,2-dithiole-3-thione. The mixture was stirred at the same temperature for 1 hour. The reaction mixture was poured into ice water. Thereto was added sodium carbonate to allow the mixture to have a pH of 10. Methanol was added thereto. The resulting precipitate (an inorganic salt) was removed by filtration. The filtrate was concentrated. The residue was purified by the use of a reversed-phase column to obtain 0.18 g of an intended product.

1H NMR (DMSO-$d_6$)

3.72 (2H, s), 3.88 (3H, s), 7.18 (1H, d), 8.01 (1H, d)

Production Example 3

Production of 4,5-dihydro-8-methoxy-7-sulfo-3H-naphtho-[1,2-c]-1,2-dithiole-3-thione sodium salt (sodium salt of compound No. 106 in Table 2)

(1) Synthesis of 2-ethoxycarbonyl-7-methoxy-1-tetralone

5.24 g of diethyl carbonate was added to a DMF (40 ml) suspension containing 2.00 g of sodium hydride (about 60%, in oil). The mixture was heated to 60 ° C. Thereto was dropwise added a DMF (10 ml) solution containing 4.40 g of 7-methoxy-1-tetralone. The mixture was stirred at the same temperature for 2 hours. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 5.63 g of an intended product.

1H NMR ($CDCl_3$, $\delta$)

1.35 (3H, t), 2.55 (2H, t), 2.75 (2H, t), 3.55-3.60 (1H, m), 3.84 (3H, s), 4.27 (2H, q), 6.89 (1H, dd), 7.09 (1H, d), 7.35 (1H, d).

(2) Synthesis of 4,5-dihydro-8-methoxy-3H-naphtho-[1,2-c]-1,2-dithiole-3-thione

A pyridine (10 ml) solution containing 5.50 g of 2-ethoxycarbonyl-7-methoxy-1-tetralone was dropwise added to a pyridine (60 ml) suspension containing 7.50 g of phosphorus pentasulfide, being refluxed with heating. The mixture was stirred at the same temperature for 2 hours. The reaction mixture was purified by the use of a Florisil® column, followed by sludging with ethyl acetate, whereby 2.24 g of an intended product was obtained.

1H NMR ($CDCl_3$, $\delta$)

2.88-2.95 (4H, m), 3.86 (3H, s), 7.01 (1H, dd), 7.15 (1H, d), 7.27 (1H, d).

(3) Synthesis of 4,5-dihydro-8-methoxy-7-sulfo-3H-naphtho[1,2-c]-1,2-dithiole-3-thione sodium salt

A concentrated sulfuric acid (4 ml) solution containing 0.92 g of 4,5-dihydro-8-methoxy-3H-naphtho-[1,2-c]-1,2-dithiole-3-thione was stirred at 100 ° C for 15 minutes. The reaction mixture was poured into ice water. Thereto was added sodium carbonate to allow the mixture to have a pH of 10. Methanol was added thereto. The resulting precipitate (an inorganic salt) was removed by filtration. The filtrate was concentrated. The residue was purified by the use of a reversed-phase column to obtain 0.36 g of an intended product.

1H NMR (DMSO-$d_6$)

2.76 (2H, t), 2.89 (2H, t), 3.87 (3H, s), 7.21 (1H, s), 7.74 (1H, s).

Production Example 4

Production of 4,5-dihydro-7-methoxy-8-sulfo-3H-naphtho[1,2-c]-1,2-dithiole-3-thione sodium salt (sodium salt of compound No. 106 in Table 3)

(1) Synthesis of 2-ethoxycarbonyl-6-methoxy-1-tetralone

6.29 g of diethyl carbonate was added to a DMF (50 ml) suspension containing 2.40 g of sodium hydride (about 60%, in oil). The mixture was heated to 60 °C. Thereto was dropwise added a DMF (10 ml) solution containing 5.28 g of 6-methoxy-1-tetralone. The mixture was stirred at the same temperature for 2 hours. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 6.80 g of an intended product.

1H NMR ($CDCl_3$, $\delta$)

1.30 (3H, t), 2.29-2.51 (2H, m), 2.95-3.02 (2H, m), 3.53-3.58 (1H, m), 3.84 (3H, s), 4.23-4.28 (2H, m), 6.69 (1H, d), 6.84 (1H, dd), 8.02 (1H, d).

(2) Synthesis of 4,5-dihydro-7-methoxy-3H-naphtho-[1,2-c]-1,2-dithiole-3-thione

A pyridine (10 ml) solution containing 6.60 g of 2-ethoxycarbonyl-6-methoxy-1-tetralone was dropwise added to a pyridine (60 ml) suspension containing 9.00 g of phosphorus pentasulfide, being heated under reflux. The mixture was stirred at the same temperature for 2 hours. The reaction mixture was purified by the use of a Florisil® column, followed by sludging with ethyl acetate, whereby 3.73 g of an intended product was obtained.

1H NMR ($CDCl_3$, $\delta$)

2.88-3.00 (4H, m), 3.88 (3H, s), 6.83-6.86 (2H, m), 7.59 (1H, d).

(3) Synthesis of 4,5-dihydro-7-methoxy-8-sulfo-3H-naphtho[1,2-c]-1,2-dithiole-3-thione sodium salt

A concentrated sulfuric acid (5 ml) solution containing 1.32 g of 4,5-dihydro-7-methoxy-3H-naphtho-[1,2-c]-1,2-dithiole-3-thione was stirred at 100 °C for 10 minutes. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 0.87 g of an intended product.

1H NMR (DMSO-$d_6$)

2.76 (2H, t), 3.00 (2H, t) 3.87 (3H, s), 7.11 (1H, s) 8.02 (1H, s).

The present compound can be synthesized also by the following process.

2.5 ml of chlorosulfuric acid was dropwise added, with ice-cooling, to a chloroform (15 ml) suspension containing 1.00 g of 4,5-dihydro-7-methoxy-3H-naphtho[1,2-c]-1,2-dithiole-3-thione. The mixture was stirred at the same temperature for 1 hour. The reaction mixture was poured into ice water. When ice was melted, chloroform was added. Layer separation was conducted, and sodium carbonate was added to the aqueous layer to allow the layer to have a pH of 10. Thereto was added methanol. The resulting precipitate (an inorganic salt) was removed by filtration. The filtrate was concentrated. The residue was purified by the use of a reversed-phase column to obtain 1.11 g of an intended product.

Production Example 5

Production of 4,5-dihydro-7-ethoxy-8-sulfo-3H-naphtho-[1,2-c]-1,2-dithiol-3-thione sodium salt (sodium salt of compound No. 109 in Table 3)

(1) Synthesis of 6-ethoxy-2-ethoxycarbonyl-1-tetralone

8.81 g of diethyl carbonate was added to a DMF (50 ml) suspension containing 3.36 g of sodium hydride (about 60%, in oil). The mixture was heated to 60 °C. Thereto was dropwise added a DMF (30 ml) solution containing 8.00 g of 6-ethoxy-1-tetralone. The mixture was stirred at the same temperature for 2 hours. The reaction mixture was subjected to distillation to remove DMF. To the residue was added diluted

hydrochloric acid. The mixture was extracted with ethyl acetate. The organic layer was dried over anhydrous magnesium sulfate and then concentrated. The residue was purified by the use of a column to obtain 10.77 g of an intended product.

1H NMR ($CDCl_3$, δ)

1.30 (3H, t), 1.43 (3H, t), 2.29-2.58 (2H, m), 2.93-2.99 (2H, m), 3.52-3.57 (1H, m), 4.09 (2H, q), 4.21-4.28 (2H, m), 6.68 (1H, d), 6.82 (1H, dd), 8.01 (1H, d).

(2) Synthesis of 4,5-dihydro-7-ethoxy-3H-naphtho-[1,2-c]-1,2-dithiole-3-thione

A pyridine (20 ml) solution containing 10.5 g of 6-ethoxy-2-ethoxycarbonyl-1-tetralone was dropwise added to a pyridine (80 ml) suspension containing 13.50 g of phosphorus pentasulfide, being refluxed with heating. The mixture was stirred at the same temperature for 2 hours. The reaction mixture was subjected to rough purification by the use of a Florisil® column and then to column purification, followed by sludging with ethyl acetate, whereby 5.75 g of an intended product was obtained.

1H NMR ($CDCl_3$, δ)

1.45 (3H, t), 2.86-3.00 (4H, m), 4.11 (2H, q), 6.80-6.84 (2H, m), 7.57 (1H, d).

(3) Synthesis of 4,5-dihydro-7-ethoxy-8-sulfo-3H-naphtho[1,2-c]-1,2-dithiole-3-thione sodium salt

2.0 ml of chlorosulfuric acid was dropwise added, with ice-cooling, to a chloroform (8 ml) suspension containing 1.12 g of 4,5-dihydro-7-ethoxy-3H-naphtho[1,2-c]-1,2-dithiole-3-thione. The mixture was stirred at room temperature for 1 hour. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 1.10 g of an intended product.

1H NMR (DMSO-$d_6$)

1.36 (3H, t), 2.75 (2H, t), 2.98 (2H, t), 4.19 (2H, q), 7.09 (1H, s), 8.04 (1H, s).

Production Example 6

Production of 4,5-dihydro-7-propoxy-8-sulfo-3H-naphtho-[1,2-c]-1,2-dithiole-3-thione sodium salt (sodium salt of compound No. 112 in Table 3)

(1) Synthesis of 6-hydroxy-1-tetralone

A mixture of 25.0 g of 6-methoxy-1-tetralone and 80.0 g of pyridine hydrochloride was stirred at 200 °C for 5 hours and then allowed to cool to room temperature. Water was added thereto. The mixture was extracted with ethyl acetate. The organic layer was dried over anhydrous magnesium sulfate and then concentrated. The residue was purified by the use of a column to obtain 17.6 g of an intended product.

1H NMR ($CDCl_3$, δ)

2.06-2.19 (2H, m), 2.63 (2H, t), 2.91 (2H, t), 6.71-6.80 (3H, m), 7.98 (1H, d).

(2) Synthesis of 6-propoxy-1-tetralone

10.00 g of n-propyl bromide was dropwise added to a DMF (70 ml) solution containing 8.11 g of 6-hydroxy-1-tetralone and 8.29 g of potassium carbonate. The mixture was stirred at 90 °C for 1 hour. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 10.15 g of an intended product.

1H NMR ($CDCl_3$, δ)

1.05 (3H, t), 1.76-1.89 (2H, m), 2.06-2.16 (2H, m), 2.61 (2H, t), 2.92 (2H, t), 3.97 (2H, t), 6.70 (1H, d), 6.82 (1H, dd), 8.00 (1H, d).

(3) Synthesis of 2-ethoxycarbonyl-6-propoxy-1-tetralone

8.81 g of diethyl carbonate was added to a DMF (50 ml) suspension containing 3.36 g of sodium hydride (about 60%, in oil). The mixture was heated to 60 °C. Thereto was dropwise added a DMF (30 ml)

solution containing 8.59 g of 6-propoxy-1-tetralone. The mixture was stirred at the same temperature for 2 hours. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 10.66 g of an intended product.

1H NMR ($CDCl_3$, δ)

1.04 (3H, t), 1.25-1.36 (3H, m), 1.79-1.86 (2H, m), 2.42-2.52 (2H, m), 2.97 (2H, m), 3.52-3.58 (1H, m), 3.97 (2H, t), 4.23-4.28 (2H, m), 6.68 (1H, d), 6.83 (1H, dd), 8.02 (1H, d).

(4) Synthesis of 4,5-dihydro-7-propoxy-3H-naphtho-[1,2-c]-1,2-dithiole-3-thione

A pyridine (20 ml) solution containing 10.0 g of 2-ethoxycarbonyl-6-propoxy-1-tetralone was dropwise added to a pyridine (80 ml) suspension containing 13.00 g of phosphorus pentasulfide, being refluxed with heating. The mixture was stirred at the same temperature for 2 hours. The reaction mixture was subjected to rough purification by the use of a Florisil® column and further to column purification, followed by sludging with ethyl acetate, whereby 5.70 g of an intended product was obtained.

1H NMR ($CDCl_3$, δ)

1.06 (3H, t), 1.84 (2H, qt), 2.86-3.00 (4H, m), 3.99 (2H, t), 6.81-6.85 (2H, m), 7.58 (1H, d).

(5) Synthesis of 4,5-dihydro-7-propoxy-8-sulfo-3H-naphtho[1,2-c]-1,2-dithiole-3-thione sodium salt

2.0 ml of chlorosulfuric acid was dropwise added, with ice-cooling, to a chloroform (8 ml) suspension containing 1.12 g of 4,5-dihydro-7-propoxy-3H-naphtho[1,2-c]-1,2-dithiole-3-thione. The mixture was stirred at the same temperature for 1.5 hours. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 1.24 g of an intended product.

1H NMR (DMSO-$d_6$)

1.02 (3H, t), 1.74 (2H, q), 2.75 (2H, t), 2.98 (2H, t), 4.05 (2H, t), 7.08 (1H, s), 8.03 (1H, s).

Production Example 7

Production of 4,5-dihydro-6-methoxy-9-sulfo-3H-naptho-[1,2-c]-1,2-dithiole-3-thione sodium salt (sodium salt of compound No. 105 in Table 4)

The above compound was produced in the same manner as in Production Example 4.

1H NMR (DMSO-$d_6$)

2.67-2.78 (4H, m), 3.88 (3H, s), 7.18 (1H, d), 7.98 (1H, d).

Production Example 8

Production of 4,5-dihydro-6-hydroxy-9-sulfo-3H-naphtho-[1,2-c]-1,2-dithiole-3-thione disodium salt (sodium salt of compound No. 66 in Table 4)

A concentrated sulfuric acid (5 ml) solution containing 1.10 g of 4,5-dihydro-6-methoxy-3H-naphtho-[1,2-c]-1,2-dithiole-3-thione was stirred at 100 °C for 10 minutes. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 0.16 g of an intended product.

1H NMR (DMSO-$d_6$)

2.73 (2H, t), 2.89 (2H, t) 7.05 (1H, d) 7.46 (1H, d).

Production Example 9

Production of 5,6-dihydro-sulfo-3H,4H-benzocyclohepteno[9,8-c]-1,2-dithiole-3-thione sodium salt

(1) Synthesis of 2-bis(methylthio)methylidene-1-benzosuberone

A mixture of 2.38 g of carbon disulfide and 5.00 g of 1-benzosuberone was dropwise added, at 5-10 °C in 30 minutes, to a benzene (100 ml) solution containing sodium tert-amyloxide prepared from 5.50 g of tert-amyl alcohol and 2.50 g of sodium hydride (about 60%, in oil). The mixture was stirred at room temperature for 2 hours. The reaction mixture was kept at 0-5 °C. Thereto was dropwise added 9.04 g of methyl iodide in 20 minutes. The mixture was stirred at room temperature for 2 hours. To the reaction mixture was added water. The mixture was extracted with ethyl acetate. The organic layer was dried over anhydrous magnesium sulfate and then concentrated. The residue was purified by the use of a column to obtain 6.53 g of an intended product.

1H NMR ($CDCl_3$, $\delta$)

1.93-2.00 (2H, m), 2.42 (3H, s) 2.44 (3H, s), 2.72-2.78 (4H, m), 7.15-7.18 (1H, m), 7.31-7.37 (1H, m), 7.41-7.46 (1H, m), 7.87-7.91 (1H, m).

(2) Synthesis of 5,6-dihydro-3H,4H-benzocyclohepteno-[9.8-c]-1,2-dithiole-3-thione

An o-xylene (200 ml) solution containing 6.53 g of 2-bis(methylthio)methylidene-1-benzosuberone was dropwise added to an o-xylene (150 ml) suspension containing 9.88 g of phosphorus pentasulfide, being refluxed with heating. The mixture was refluxed with heating, for 30 minutes. The reaction mixture was washed with water. The organic layer was dried over anhydrous magnesium sulfate and then concentrated. The residue was purified by the use of a column to obtain 5.96 g of an intended product.

1H NMR ($CDCl_3$, $\delta$)

2.20-2.31 (2H, m), 2.57-2.71 (4H, m), 7.36-7.57 (3H, m), 7.58-7.60 (1H, m).

(3) Synthesis of 5,6-dihydro-chlorosulfonyl-3H,4H-benzocycloheptano[9.8-c]-1,2-dithiole-3-thione

2.3 ml of chlorosulfuric acid was dropwise added, with ice-cooling, to a chloroform (10 ml) solution containing 800 mg of 5,6-dihydro-3H,4H-benzocyclohepteno[9,8-c]-1,2-dithiole-3-thione. The mixture was stirred at the same temperature for 15 minutes and then refluxed for 1.5 hours with heating. The reaction mixture was poured into ice water. When ice was melted, chloroform was added. Layer separation was conducted. The organic layer was dried over anhydrous magnesium sulfate and then concentrated to dryness to obtain 930 mg of an intended product.

1H NMR ($CDCl_3$, $\delta$)

2.30-2.38 (2H, m), 2.67-2.76 (4H, m) 7.66 (1H, d), 8.12 (1H, dd) 8.22 (1H, d).

(4) Synthesis of 5,6-dihydro-sulfo-3H,4H-benzocyclohepteno[9.8-c]-1,2-dithiole-3-thione sodium salt

Sodium carbonate was added to an aqueous solution containing 2.90 g of 5,6-dihydro-chlorosulfonyl-3H,4H-benzocyclohepteno[9,8-c]-1,2-dithiole-3-thione. The mixture was refluxed with heating, for 1 hour. To the reaction mixture was added methanol. The resulting precipitate (an inorganic salt) was removed by filtration. The filtrate was concentrated. The residue was purified by the use of a reversed-phase column to obtain 290 mg of an intended product.

1H NMR (DMSO-$d_6$)

2.11-2.18 (2H, m), 2.53-2.58 (4H, m), 7.47 (1H, d), 7.75 (1H, d), 7.82 (1H, s).

Production Example 10

Production of 7-methoxy-8-sulfo-3H,4H-benzopyrano-[4,3-c]-1,2-dithiole-3-thione sodium salt (sodium salt of compound No. 42 in Table 7)

(1) Synthesis of 3-methoxyphenyl-2-cyanoethyl ether

A mixture of 28 g of 3-methoxyphenol, 80 ml of acrylonitrile and 2 ml of Triton B (a 40% aqueous solution) was refluxed with heating, in a nitrogen stream for 20 hours. After the reaction mixture was allowed

to cool, 500 ml of ether was added thereto. The mixture was washed with 2 N aqueous sodium hydroxide, diluted hydrochloric acid and water in this order. The organic layer was dried over anhydrous magnesium sulfate and concentrated to dryness to obtain 34.8 g of an intended product.

1H NMR ($CDCl_3$, $\delta$)

2.82 (2H, t), 3.79 (3H, s), 4.18 (2H, t), 6.46-6.58 (3H, m), 7.20 (1H, t).

(2) Synthesis of 3-(3-methoxyphenoxy)propionic acid

80 ml of concentrated hydrochloric acid was added to 34 g of 3-methoxyphenyl-2-cyanoethylether. The mixture was refluxed with heating, for 5 hours. The reaction mixture was allowed to cool and then cooled in an ice water bath. The resulting solid precipitate was collected by filtration. The solid was washed with water and then made into a chloroform solution. The solution was dried over anhydrous magnesium sulfate and then concentrated. The residue was subjected to sludging with hexane to obtain 22.8 g of an intended product.

1H NMR ($CDCl_3$, $\delta$)

2.85 (2H, t), 3.79 (3H, s), 4.24 (2H, t), 6.46-6.54 (3H, m), 7.18 (1H, t).

(3) Synthesis of 7-methoxy-2H-1-benzopyran-4-one

20 ml of thionyl chloride was dropwise added to a toluene (120 ml) solution containing 22 g of 3-(3-methoxyphenoxy)propionicacid. The mixture was refluxed with heating, for 1.5 hours. The reaction mixture was concentrated. The residue was dissolved in 100 ml of chloroform. To the solution was dropwise added 10.5 ml of trifluoromethanesulfonic acid at -65 °C. After the completion of the dropwise addition, the mixture was returned to room temperature. The organic layer was washed with water and an aqueous sodium hydrogen-carbonate solution in this order, dried over anhydrous magnesium sulfate, and concentrated. The residue was purified by the use of a column to obtain 8.0 g of an intended product.

1H NMR ($CDCl_3$, $\delta$)

2.76 (2H, t), 3.84 (3H, s), 4.52 (2H, t), 6.41 (1H, d), 6.58 (1H, dd), 7.84 (1H, d).

(4) Synthesis of 3-bis(methylthio)methylidene-7-methoxy-2H-1-benzopyran-4-one

2.6 ml of dimethylacetamide was dropwise added, in 10 minutes, to a benzene (20 ml) suspension containing 2 g of 7-methoxy-2H-1-benzopyran-4-one, 0.9 g of sodium hydride (about 60%, in oil), 1 ml of carbon disulfide and 2 ml of methyl iodide. The mixture was stirred at room temperature for 2 hours. Thereto was added ice, and the mixture was extracted with ethyl acetate. The organic layer was dried over anhydrous magnesium sulfate and then concentrated. The residue was purified by the use of a column to obtain 2.33 g of an intended product.

1H NMR ($CDCl_3$, $\delta$)

2.43 (3H, s), 2.48 (3H, s), 3.84 (3H, s), 5.36 (2H, s), 6.39 (1H, d), 6.60 (1H, dd). 7.92 (1H, d).

(5) Synthesis of 7-methoxy-3H,4H-benzopyrano[4,3-c]-1,2-dithiole-3-thione

An o-xylene (20 ml) solution containing 3.4 g of 3-bis(methylthio)methylidene-7-methoxy-2H-1-benzopyran-4-one was dropwise added to an o-xylene (20 ml) suspension containing 3.2 g of phosphorus pentasulfide, being refluxed with heating. The mixture was refluxed with heating, for 1 hour. To the reaction mixture was added ethyl acetate. The mixture was washed with water. The organic layer was dried over anhydrous magnesium sulfate and concentrated. The residue was purified by the use of a column to obtain 1.07 g of an intended product.

1H NMR ($CDCl_3$, $\delta$)

3.84 (3H, s) 5.15 (2H, s), 6.66-6.73 (2H, m), 7.67 (1H, d).

(6) Synthesis of 7-methoxy-8-sulfo-3H,4H-benzopyrano-[4,3-c]-1,2-dithiole-3-thione sodium salt

A concentrated sulfuric acid (4 ml) solution containing 1.00 g of 7-methoxy-3H,4H-benzopyrano[4,3-c]-1,2-dithiole-3-thione was stirred at room temperature for 4 hours. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 1.18 g of an intended

product.
1H NMR (DMSO-$d_6$)
3.84 (3H, s), 5.18 (2H, s), 6.70 (1H, s), 7.88 (1H, s).

Production Example 11

Production of 8-ethoxy-7-sulfo-3H,4H-benzopyrano-[4,3-c]-1,2-dithiole-3-thione sodium salt (sodium salt of compound No. 45 in Table 6)

(1) Synthesis of 6-hydroxy-4-chromanone

A mixture of 24.0 g of 6-methoxy-4-chromanone and 75.0 g of pyridine hydrochloride was stirred at 200°C for 5 hours. The reaction mixture was allowed to cool to room temperature. Thereto was added water. The mixture was extracted with ethyl acetate. The organic layer was dried over anhydrous magnesium sulfate and concentrated. The residue was purified by the use of a column to obtain 10.64 g of an intended product.
1H NMR ($CDCl_3$, δ)
2.81 (2H, t), 4.49 (2H, t), 6.10 (1H, bs), 6.90 (1H, d), 7.08 (1H, dd), 7.40 (1H, d).

(2) Synthesis of 6-ethoxy-4-chromanone

5.23 g of ethyl bromide was dropwise added to a DMF (40 ml) solution containing 4.92 g of 6-hydroxy-4-chromanone and 4.98 g of potassium carbonate. The mixture was stirred at 90°C for 1.5 hours. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 5.34 g of an intended product.
1H NMR ($CDCl_3$, δ)
1.40 (3H, t), 2.79 (2H, t), 4.02 (2H, q), 4.49 (2H, t), 6.90 (1H, d), 7.09 (1H, dd), 7.31 (1H, d).

(3) Synthesis of 3-bis(methylthio)methylidene-6-ethoxy-4-chromanone

A mixture of 2.05 g of carbon disulfide and 5.19 g of 6-ethoxy-4-chromanone was dropwise added, at 5-10°C in 10 minutes, to a toluene (70 ml) solution containing sodium tert-amyloxide prepared from 4.76 g of tert-amyl alcohol and 2.17 g of sodium hydride (about 60%, in oil). The mixture was stirred at room temperature for 1 hour. The reaction mixture was kept at 0-5°C. Thereto was dropwise added 7.65 g of methyl iodide in 20 minutes. The mixture was stirred at room temperature for 2.5 hours. To the reaction mixture was added water. The mixture was extracted with ethyl acetate. The organic layer was washed with a saturated aqueous sodium chloride solution, dried over anhydrous magnesium sulfate, and concentrated. The residue was purified by the use of a column to obtain 4.01 g of an intended product.
1H NMR ($CDCl_3$, δ)
1.41 (3H, t), 2.44 (3H, s), 2.50 (3H, s), 4.04 (2H, q), 5.33 (2H, s), 6.87 (1H, d), 7.05 (1H, dd), 7.41 (1H, d).

(4) Synthesis of 8-ethoxy-3H,4H-benzopyrano[4,3-c]-1,2-dithiole-3-thione

An o-xylene (20 ml) solution containing 4.00 g of 3-bis(methylthio)methylidene-6-ethoxy-4-chromanone was dropwise added, in 10 minutes, to an o-xylene (40 ml) suspension containing 4.80 g of phosphorus pentasulfide, being refluxed wth heating. The mixture was refluxed with heating, for 1 hour. To the reaction mixture was added chloroform. The mixture was washed with a saturated aqueous sodium chloride solution. The organic layer was dried over anhydrous magnesium sulfate and then concentrated. The residue was purified by the use of a column to obtain 2.53 g of an intended product.
1H NMR ($CDCl_3$, δ)
1.43 (3H, t), 4.04 (2H, q), 5.18 (2H, s), 6.94-7.03 (3H, m).

(5) Synthesis of 8-ethoxy-7-sulfo-3H,4H-benzopyrano-[4,3-c]-1,2-dithiole-3-thione sodium salt

1.0 ml of chlorosulfuric acid was dropwise added, with ice-cooling, to a chloroform (5 ml) suspension containing 1.00 g of 8-ethoxy-3H,4H-benzopyrano-[4,3-c]-1,2-dithiole-3-thione. The mixture was stirred at the same temperature for 2 hours. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 0.083 g of an intended product.
1H NMR (DMSO-$d_6$)
1.32 (3H, t), 4.11 (2H, q), 5.10 (2H, s), 7.18 (1H, s), 7.37 (1H, s).

Production Example 12

Production of 8-methoxy-7-sulfo-3H,4H-benzopyrano-[4,3-c]-1,2-dithiole-3-thione sodium salt (sodium salt of compound No. 42 in Table 6)

The above compound was produced in the same manner as in Production Example 10.
1H NMR (DMSO-$d_6$)
3.83 (3H, s), 5.10 (2H, s), 7.16 (1H, s), 7.36 (1H, s).

Production Example 13

Production of 8-sulfo-3H,4H-benzopyrano[4,3-c]-1,2-dithiole-3-thione sodium salt (sodium salt of compound No. 1 in Table 7)

The above compound was produced in the same manner as in Production Example 10.
1H NMR (DMSO-$d_6$)
5.19 (2H, s), 7.05 (1H, d), 7.71 (1H, d), 7.76 (1H, dd).

Reference Example 1

Production of 4,5-dihydro-7-(1-methyl)ethoxy-3H-naphtho[1,2-c]-1,2-dithiole-3-thione

(1) Synthesis of 6-(1-methyl)ethoxy-1-tetralone

10.00 g of isopropyl iodide was dropwise added to a DMF (70 ml) solution containing 6.30 g of 6-hydroxy-1-tetralone and 6.00 g of potassium carbonate. The mixture was stirred at 90 °C for 0.5 hours. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 6.65 g of an intended product.
1H NMR ($CDCl_3$, $\delta$)
1.36 (6H, d), 2.08-2.15 (2H, m), 2.60 (2H, t), 2.91 (1H, t), 4.59-4.68 (1H, m), 6.67 (1H, d), 6.79 (1H, dd), 7.99 (1H, d).

(2) Synthesis of 2-ethoxycarbonyl-6-(1-methyl)ethoxy-1-tetralone

8.81 g of diethyl carbonate was added to a DMF (50 ml) suspension containing 3.36 g of sodium hydride (about 60%, in oil). The mixture was heated to 60 °C. Thereto was dropwise added a DMF (30 ml) solution containing 8.59 g of 6-(1-methyl)ethoxy-1-tetralone. The mixture was stirred at the same temperature for 2 hours. The reaction mixture was subjected to distillation to remove DMF. To the residue was added diluted hydrochloric acid. The mixture was extracted with ethyl acetate. The organic layer was dried over anhydrous magnesium sulfate and then concentrated. The residue was purified by the use of a column to obtain 10.50 g of an intended product.
1H NMR ($CDCl_3$, $\delta$)
1.32 (3H, t), 1.35 (3H, s), 1.37 (3H, s), 2.45-2.55 (2H, m), 2.92-3.02 (2H, m), 3.52-3.58 (1H, m), 4.24 (2H, q), 4.61-4.66 (1H, m), 6.66 (1H, d), 6.80 (1H, d), 8.01 (1H, d).

(3) Synthesis of 4,5-dihydro-7-(1-methyl)ethoxy-3H-naphtho[1.2-c]-1,2-dithiole-3-thione

A pyridine (20 ml) solution containing 10.0 g of 2-ethoxycarbonyl-6-(1-methyl)ethoxy-1-tetralone was dropwise added to a pyridine (80 ml) suspension containing 12.9 g of phosphorus pentasulfide, being refluxed with heating. The mixture was stirred at the same temperature for 2 hours. The reaction mixture was subjected to purification by chromatography on a Florisil® column and a silica gel column, followed by sludging with ethyl acetate, whereby 5.62 g of an intended product was obtained.

1H NMR ($CDCl_3$, $\delta$)

1.38 (6H, d), 2.88-3.00 (4H, m), 4.64 (1H, qq), 6.80-6.83 (2H, m), 7.57 (1H, d).

Reference Example 2

Production of 4,5-dihydro-9-methoxy-6(3H)-benzoxepino-[5,4-c]-1,2-dithiole-3-thione

(1) Synthesis of 2,3-dihydro-7-methoxy-4-bis(methylthio)methylidene-1-benzoxepin-5-one

A mixture of 1.80 g of carbon disulfide and 4.55 g of 3,4-dihydro-7-methoxy-1(2H)-benzoxepin-5-one synthesized by the process described in Indian J. Chem., 13, 1 (1975) was dropwise added, at 5-10 °C in 10 minutes, to a toluene (60 ml) solution containing sodium tert-amyloxide prepared from 4.18 g of tert-amyl alcohol and 1.90 g of sodium hydride (about 60%, in oil). The mixture was stirred at room temperature for 1 hour. The reaction mixture was kept at 0-5 °C. Thereto was dropwise added 6.72 g of methyl iodide in 20 minutes. The mixture was stirred at room temperature for 2.5 hours. To the reaction mixture was added water. The mixture was extracted with ethyl acetate. The organic layer was washed with a saturated aqueous sodium chloride solution, dried over anhydrous magnesium sulfate, and concentrated. The residue was purified by the use of a column to obtain 4.06 g of an intended product.

1H NMR ($CDCl_3$, $\delta$)

2.40 (3H, s), 2.42 (3H, s), 3.11 (2H, t), 3.83 (3H, s), 4.24 (2H, t), 6.95-7.02 (2H, m), 7.33 (1H, d).

(2) Synthesis of 4,5-dihydro-9-methoxy-6-benzoxepino-[5,4-c]-1,2-dithiole-3-thione

An o-xylene (20 ml) solution containing 4.00 g of 2,3-dihydro-7-methoxy-4-bis(methylthio)methylidene-1-benzoxepin-5-one was dropwise added, in 10 minutes, to an o-xylene (40 ml) suspension containing 4.80 g of phosphorus pentasulfide, being refluxed with heating. The mixture was refluxed with heating, for 1 hour. To the reaction mixture was added chloroform. The mixture was washed with a saturated aqueous sodium chloride solution. The organic layer was dried over anhydrous magnesium sulfate and then concentrated. The residue was purified by the use of a column to obtain 2.30 g of an intended product.

1H NMR ($CDCl_3$, $\delta$)

3.09 (2H, t), 3.85 (3H, s), 4.46 (2H, t), 7.02 (1H, dd), 7.12 (1H, d), 7.18 (1H, d).

Then, description is made on Production Examples of the present compound represented by general formula (11).

Production Example 21

Production of 5-hexyl-4-(4-methoxy-3-sulfophenyl)-3H-1,2-dithiole-3-thione sodium salt

(1) Synthesis of 4-methoxyphenyl-2-octanone

A THF (30 ml) solution containing 10.10 g of hexyl magnesium bromide was dropwise added, at -78 °C in 1.2 hours, to a THF (50 ml) solution containing 12.40 g of 4-methoxyphenylacetyl chloride. The mixture was stirred at the same temperature for 3 hours. Thereto was added water, followed by extraction with ethyl acetate. The organic layer was dried over anhdyrous magnesium sulfate and then concentrated. The residue was purified by the use of a column to obtain 3.48 g of an intended product.

1H NMR (CDCl3, $\delta$)

0.86 (3H, t), 1.24 (6H, m), 1.53 (2H, t), 2.42 (2H, t), 3.61 (2H, s), 3.80 (3H, s), 6.86 (2H, d), 7.11 (2H, d)

(2) Synthesis of 2-(4-methoxyphenyl)-1,1-bis(methylthio)-1-nonen-3-one

A benzene (20 ml) solution containing 1.13 g of carbon disulfide and 3.46 g of 4-methoxyphenyl-2-octanone was dropwise added, at 5-10°C in 15 minutes, to a benzene (50 ml) solution containing sodium tert-amyloxide prepared from 2.62 g of tert-amyl alcohol and 1.19 g of sodium hydride (about 60%, in oil). The mixture was stirred at room temperature for 2 hours. The reaction mixture was kept at 0-5°C. Thereto was dropwise added 4.20 g of methyl iodide. The mixture was stirred at room temperature for 1 hour. Thereto was added 200 ml of water, followed by extraction with ethyl acetate. The organic layer was washed with water, dried over anhydrous magnesium sulfate, and concentrated. The residue was purified by the use of a column to obtain 3.33 g of an intended product.

1H NMR (CDCl3, δ)

0.84 (3H, t), 1.24 (6H, m), 1.55 (2H, m), 2.21 (3H, s), 2.38 (3H, s), 2.51 (2H, t), 3.82 (3H, s), 6.89 (2H, d), 7.23 (2H, d)

(3) Synthesis of 5-hexyl-4-(4-methoxyphenyl)-3H-1,2-dithiole-3-thione

An o-xylene (15 ml) of solution containing 3.33 g of 2-(4-methoxyphenyl)-1,1-bis(methylthio)-1-nonen-3-one was dropwise added to an o-xylene (35 ml) suspension containing 3.99 g of phosphorus pentasulfide, being refluxed with heating. The mixture was refluxed for 60 minutes and then cooled to room temperature. Thereto was added 50 ml of ethyl acetate. The mixture was washed with a 5% aqueous sodium hydrogencarbonate solution and a saturated aqueous sodium chloride solution. The organic layer was dried over anhydrous magnesium sulfate and then concentrated. The resiude was purified by the use of a column to obtain 2.23 g of an intended product.

1H NMR (CDCl3, δ)

0.85 (3H, t), 1.22 (6H, m), 1.60 (2H, m), 2.68 (2H, t), 3.85 (3H, s), 7.01 (2H, d), 7.11 (2H, d)

(4) Synthesis of 5-hexyl-4-(4-methoxy-3-sulfophenyl)-3H-1,2-dithiole-3-thione sodium salt

A concentrated sulfuric acid (10 ml) solution containing 1.00 g of 4-(4-methoxyphenyl)-5-hexyl-3H-1,2-dithiole-3-thione was stirred at 100°C for 10 minutes. The reaction mixture was poured into ice water. Thereto was added sodium carbonate until it was not soluble anymore. Thereto was added methanol. The resulting precipitate (an inorganic salt) was removed by filtration. The filtrate was concentrated. The residue was purified by a Florisil® column, followed by recrystallization from hexane/ethyl acetate, to obtain 1.02 g of an intended product.

1H NMR (CD3OD, δ)

0.83 (3H, t), 1.21 (6H, m), 1.80 (2H, m), 2.18 (2H, m), 3.96 (3H, s), 7.16 (1H, d), 7.27 (1H, d), 7.69 (1H, s)

Production Example 22

Production of 5-dodecyl-4-(4-methoxy-3-sulfophenyl)-3H-1,2-dithiole-3-thione sodium salt

The above compound was produced in the same manner as in Production Example 21.

1H NMR (CD3OD, δ)

0.89 (3H, t), 1.25 (18H, m), 1.64 (2H, t), 2.73 (2H, t), 3.98 (3H, s), 7.14 (1H, d), 7.26 (1H, dd), 7.71 (1H, d)

Production Example 23

Production of 5-methyl-4-(4-sulfobenzyl)-3H-1,2-dithiole-3-thione sodium salt

(1) Synthesis of 1,1-bis(methylthio)-2-benzyl-1-buten-3-one

A mixture of 7.61 g of carbon disulfide and 14.82 g of 4-phenyl-2-butanone was dropwise added, at 5-10°C in 50 minutes, to a benzene (100 ml) solution containing sodium tert-amyloxide prepared from 17.63 g of tert-amyl alcohol and 8.00 g of sodium hydride (about 60%, in oil). The mixture was stirred at room temperature for 5 hours. The reaction mixture was kept at 0-5°C. Thereto was dropwise added 28.97 g of methyl iodide in 1 hour. The mixture was stirred at room temperature for 3.5 hours. Thereto was added 200

ml of water. The mixture was extracted with ethyl acetate. The organic layer was washed with water, dried over anhydrous magnesium sulfate, and concentrated. The residue was purified by the use of a column to obtain 17.31 g of an intended product.

1H NMR (CDCl3, δ)

2.14 (3H, s), 2.33 (3H, s), 2.36 (3H, s), 3.98 (2H, s), 7.15-7.30 (5H, m)

(2) Synthesis of 5-methyl-4-benzyl-3H-1,2-dithiole-3-thione

An o-xylene (100 ml) solution containing 17.31 g of 1,1-bis(methylthio)-2-benzyl-1-buten-3-one was dropwise added to an o-xylene (300 ml) suspension containing 27.45 g of phosphorus pentasulfide, being refluxed with heating. The mixture was refluxed for 30 minutes and then cooled to room temperature. Thereto was added 300 ml of diethyl ether. Filtration under reduced pressure was conducted to remove insolubles. The filtrate was washed with water, a 1% aqueous sodium hydroxide solution and a saturated aqueous sodium chloride solution in this order. The organic layer was dried over anhydrous magnesium sulfate and then concentrated. The residue was purified by the use of a column to obtain 14.35 g of an intended product.

1H NMR (CDCl3, δ)

2.44 (3H, s), 4.11 (2H, s), 7.18-7.28 (5H, m)

(3) Synthesis of 5-methyl-4-(4-sulfobenzyl)-3H-1,2-dithiole-3-thione sodium salt

1.5 ml of chlorosulfuric acid was dropwise added, with ice-cooling, to a chloroform (7 ml) solution cntaining 600 mg of 5-methyl-4-benzyl-3H-1,2-dithiole-3-thione. The mixture was stirred at the same temperature for 5 minutes and then at room temperature for 50 minutes. The reaction mixture was poured into ice water. When the ice was melted, chloroform was added thereto. Layer separation was conducted. To the aqueous layer was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 330 mg of an intended product.

1H NMR (DMSO-d6, δ)

2.49 (3H, s), 4.04 (2H, s), 7.11 (2H, d), 7.49 (2H, d)

Production Example 24

Production of 4-(4-methoxy-3-sulfophenyl)-5-methyl-3H-1,2-dithiole-3-thione

(1) Synthesis of 4-(4-methoxyphenyl)-5-methyl-3H-1,2-dithiole-3-thione

The above compound was synthesized in the same manner as in the item (2) of Production Example 23.

1H NMR (CDCl3, δ)

2.37 (3H, s), 3.85 (3H, s), 7.01 (2H, d), 7.13 (2H, d)

(2) Synthesis of 4-(4-methoxy-3-sulfophenyl)-5-methyl-3H-1,2-dithiole-3-thione

A concentrated sulfuric acid (10 ml) solution containing 1.00 g of 4-(4-methoxyphenyl)-5-methyl-3H-1,2-dithiole-3-thione was stirred at 100 °C for 15 minutes. The reaction mixture was poured into ice water. Sodium carbonate was added thereto until it was not soluble anymore. The supernatant liquid was allowed to stand. The resulting precipitate (solid) was purified by the use of a column to obtain 680 mg of an intended product.

1H NMR (DMSO-d6, δ)

2.40 (3H, s), 3.98 (3H, s), 7.16 (1H, d), 7.31 (1H, d), 7.75 (1H, s)

Production Example 25

Production of 4-(4-methoxy-3-sulfobenzyl)-5-methyl-3H-1,2-dithiole-3-thione sodium salt

(1) Synthesis of 1,1-bis(methylthio)-2-(4-methoxybenzyl)-1-buten-3-one

A mixture of 4.27 g of carbon disulfide and 10.00 g of 4-(4-methoxyphenyl)-2-butanone was dropwise added, at 5-10 °C in 1.2 hours, to a benzene (60 ml) solution containing sodium tert-amyloxide prepared from 9.90 g of tert-amyl alcohol and 4.49 g of sodium hydride (about 60%, in oil). The mixture was stirred at room temperature for 5 hours. The reaction mixture was kept at 0-5 °C. Thereto was dropwise added 16.24 g of methyl iodide in 1 hour. The mixture was stirred at room temperature for 2.5 hours. Thereto was added 100 ml of water. The mixture was extracted with ethyl acetate. The organic layer was washed with water, dried over anhydrous magnesium sulfate, and concentrated. The residue was purified by the use of a column to obtain 11.37 g of an intended product.

1H NMR (CDCl3, δ)

2.14 (3H, s), 2.32 (3H, s), 2.36 (3H, s), 3.78 (3H, s), 3.90 (2H, s), 6.82 (2H, d), 7.07 (2H, d)

(2) Synthesis of 4-(4-methoxybenzyl)-5-methyl-3H-1,2-dithiole-3-thione

An o-xylene (12 ml) solution containing 4.37 g of 1,1-bis(methylthio)-2-(4-methoxybenzyl)-1-buten-3-one was dropwise added to an o-xylene (80 ml) suspension containing 8.06 g of phosphorus pentasulfide, being refluxed with heating. The mixture was refluxed for 15 minutes and then cooled to room temperature. Thereto was added 50 ml of diethyl ether. Filtration under reduced pressure was conducted to remove insolubles. The filtrate was washed with water, a 1% aqueous sodium hydroxide solution solution and a saturated aqueous sodium chloride solution in this order. The organic layer was dried over magnesium sulfate and then concentrated. The residue was purified by the use of a column to obtain 3.17 g of an intended product.

1H NMR (CDCl3, δ)

2.45 (3H, s), 3.77 (3H, s), 4.04 (2H, s), 6.80 (2H, d), 7.12 (2H, d)

(3) Synthesis of 4-(4-methoxy-3-sulfobenzyl)-5-methyl-3H-1,2-dithiole-3-thione

A concentrated sulfuric acid (10 ml) solution containing 1.00 g of 4-(4-methoxybenzyl)-5-methyl-1,2-dithiole-3-thione was stirred at 100 °C for 5 minutes. The reaction mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 260 mg of an intended product.

1H NMR (DMSO-d6, δ)

2.49 (3H, s), 3.72 (3H, s), 3.97 (2H, s), 6.84 (1H, d), 7.09 (1H, dd), 7.54 (1H, s)

The above compound can be produced also by the following process.

1.5 ml of chlorosulfuric acid was dropwise added, with ice-cooling, to a chloroform (7 ml) solution containing 500 mg of 4-(4-methoxybenzyl)-5-methyl-3H-1,2-dithiole-3-thione. The mixture was stirred at the same temperature for 5 minutes and then at room temperature for 15 minutes. The reaction mixture was poured into ice. When ice was melted, chloroform was added thereto. Layer separation was conducted. The organic layer was dried over anhydrous magnesium sulfate and then concentrated to dryness to obtain 4-(3-chlorosulfonyl-4-methoxybenzyl)-5-methyl-3H-1,2-dithiole-3-thione. This compound was refluxed with heating, in an aqueous sodium carbonate solution. The resulting solution was combined with the aqueous layer obtained by the above layer separation. Thereto was added sodium carbonate to allow the mixture to have a pH of 10. Thereto was added methanol. The resulting precipitate (an inorganic salt) was removed by filtration. The filtrate was concentrated. The residue was purified by the use of a reversed-phase column to obtain 305 mg of an intended product.

Production Example 26

Production of 5-hexyl-4-(4-methoxy-3-sulfobenzyl)-3H-1,2-dithiole-3-thione sodium salt

(1) Synthesis of ethyl 3-oxononanoate

Synthesis was conducted as follows by the process described in J. Org. Chem., 43, 2087 (1978).

To a methylene chloride (25 ml) solution containing 22.49 g of Meldrum's acid were dropwise added, at 0 °C, 25.00 g of enanthic acid chloride in 5 minutes and 24.19 g of pyridine in 30 minutes. The mixture was stirred at the same temperature for 1 hour and then at room temperature for 2 hours. The reaction mixture was washed with 0.5 N hydrochloric acid and water in this order. The organic layer was dried over anhydrous magnesium sulfate and then concentrated to dryness. The residue was dissolved in 350 ml of ethanol. The ethanol solution was refluxed for 3 hours. The reaction mixture was concentrated. The residue was purified by the use of a column to obtain 21.44 g of an intended product.

1H NMR (CDCl3, δ)

0.88 (3H, t), 1.24-1.36 (9H, m), 1.56-1.68 (2H, m), 2.53 (2H, t), 3.43 (2H, s), 4.20 (2H, q)

(2) Synthesis of ethyl 2-(4-methoxybenzyl)-3-oxononanoate

A DMF (50 ml) solution containing 18.02 g of ethyl 3-oxononanoate was dropwise added, with ice-cooling, to a DMF (200 ml) suspension containing 4.00 g of sodium hydride (about 60%, in oil). The mixture was stirred at room temperature for 40 minutes. Thereto was dropwise added a DMF (20 ml) solution containing 14.09 g of 4-methoxybenzyl chloride, at the same temperature. The mixture was stirred at 80 °C for 6 hours. The reaction mixture was subjected to distillation to remove DMF. The residue was dissolved in chloroform. The chloroform solution was washed with water, dried over anhydrous magnesium sulfate, and concentrated. The residue was purified by the use of a column to obtain 12.12 g of an intended product.

1H NMR (CDCl3, δ)

0.86 (3H, t), 1.12-1.28 (11H, m), 2.19 (1H, t), 3.14 (2H, t) 3.75 (2H, d), 3.77 (3H, s), 4.13 (2H, q), 6.78 (2H, d), 7.02 (2H, d)

(3) Synthesis of 5-hexyl-4-(4-methoxybenzyl)-3H-1,2-dithiole-3-thione

A pyridine (10 ml) solution containing 12.12 g of ethyl 2-(4-methoxybenzyl)-3-oxononanoate was dropwise added to a pyridine (20 ml) suspension containing 12.60 g of phosphorus pentasulfide, being refluxed. The mixture was stirred at the same temperature for 2 hours. The reaction mixture was returned to room temperature and poured into water. The mixture was extracted with chloroform. The organic layer was dried over anhydrous magnesium sulfate and then concentrated. The residue was purified by the use of a column to obtain 2.65 g of an intended product.

1H NMR (CDCl3, δ)

0.87 (3H, t), 1.21-1.36 (6H, m), 1.57-1.64 (2H, m), 2.79 (2H, t), 3.77 (3H, s), 4.06 (2H, s), 6.79 (2H, d), 7.10 (2H, d)

(4) Synthesis of 5-hexyl-4-(4-methoxy-3-sulfobenzyl)-3H-1,2-dithiole-3-thione sodium salt

A concentrated sulfuric acid (6 ml) solution containing 600 mg of 5-hexyl-4-(4-methoxybenzyl)-3H-1,2-dithiole-3-thione was stirred at 100 °C for 5 minutes. The reaction mixture was poured into ice water. Thereto was added sodium carbonate to allow the mixture to have a pH of 10. Methanol was added thereto. The resulting precipitate (an inorganic salt) was removed by filtration. The filtrate was concentrated. The residue was purified by the use of a revesed-phase column to obtain 468 mg of an intended product.

1H NMR (DMSO-d6, δ)

0.84 (3H, t), 1.19-1.32 (6H, m), 1.52-1.59 (2H, m), 2.85 (2H, t), 3.70 (3H, s), 3.98 (2H, s), 6.83 (1H, d), 7.04 (1H, dd), 7.50 (1H, d)

Production Example 27

Production of 5-butyl-4-(4-sulfobenzyl)-3H-1,2-dithiole-3-thione sodium salt

The above compound was produced in the same manner as in Production Example 26.
1H NMR (DMSO-d6, δ)
0.83 (3H, t), 1.33 (2H, m), 1.52 (2H, m), 2.86 (2H, t), 4.07 (2H, s), 7.11 (2H, d), 7.50 (2H, d)

Production Example 28

Production of 5-hexyl-4-(4-sulfobenzyl)-3H-1,2-dithiole-3-thione sodium salt

The above compound was produced in the same manner as in Production Example 26.
1H NMR (DMSO-d6, δ)
0.85 (3H, m), 1.23 (6H, m), 1.53 (2H, m), 2.85 (2H, m), 4.07 (2H, s), 7.10 (2H, d), 7.48 (2H, d)

Production Example 29

Production of 5-octyl-4-(4-sulfobenzyl)-3H-1,2-dithiole-3-thione sodium salt

The above compound was produced in the same manner as in Production Example 26.
1H NMR (DMSO-d6, δ)
0.85 (3H, m), 1.23 (10H, m), 1.64 (2H, m), 2.50 (2H, m), 4.02 (2H, s), 6.96 (2H, d), 7.54 (2H, d)

Production Example 30

Production of 5-butyl-4-(4-methoxy-3-sulfobenzyl)-3H-1,2-dithiole-3-thione sodium salt

The above compound was produced in the same manner as in Production Example 26.
1H NMR (DMSO-d6, δ)
0.85 (3H, t), 1.34 (2H, m), 1.57 (2H, m), 2.85 (2H, t) 3.70 (3H, s), 3.99 (2H, s), 6.83 (1H, d), 7.06 (1H, d), 7.50 (1H, s)

Production Example 31

Prodution of 5-pentyl-4-(4-methoxy-3-sulfobenzyl)-3H-1,2-dithiole-3-thione sodium salt

The above compound was produced in the same manner as in Production Example 26.
1H NMR (DMSO-d6, δ)
0.83 (3H, m), 1.24 (4H, m), 1.56 (2H, m), 2.84 (2H, m), 3.71 (3H, s), 3.99 (2H, s), 6.85 (1H, m), 7.02 (1H, m), 7.50 (1H, m)

Production Example 32

Production of 5-heptyl-4-(4-methoxy-3-sulfobenzyl)-3H-1,2-dithiole-3-thione sodium salt

The above compound was produced in the same manner as in Production Example 26.
1H NMR (DMSO-d6, δ)
0.83 (3H, m), 1.25 (8H, m), 1.55 (2H, m), 2.87 (2H, m), 3.69 (3H, s), 3.98 (2H, s), 6.84 (1H, d), 7.03 (1H, d), 7.52 (1H, s)

Production Example 33

Production of 5-octyl-4-(4-methoxy-3-sulfobenzyl)-3H-1,2-dithiole-3-thione sodium salt

The above compound was produced in the same manner as in Production Example 26.
1H NMR (DMSO-d6, δ)
0.85 (3H, t), 1.22 (10H, s), 1.55 (2H, m), 2.85 (2H, t), 3.69 (3H, s), 3.98 (2H, s), 6.82 (1H, d), 7.03 (1H,

d), 7.49 (1H, s)

Production Example 34

Production of 4-(4-methoxy-3-sulfophenyl)-5-(p-tolyl)-3H-1,2-dithiole-3-thione sodium salt

(1) Synthesis of 4-methoxy-α-methyl-4'-methylstilbene

A diethyl ether (50 ml) solution containing 9.00 g of 4-methoxyacetophenone was dropwise added, at such a rate as to give rise to mild refluxing, to a diethyl ether (150 ml) solution containing a Grignard reagent prepared from 10.03 g of 4-methylbenzyl chloride and 1.70 g of magnesium. The mixture was stirred at room temperature for 3 hours. The reaction mixture was added to 2 N aqueous hydrochloric acid solution. The mixture was extracted with diethyl ether. The organic layer was washed with water and a saturated aqueous sodium chloride solution, dried over anhydrous magnesium sulfate, and concentrated. To the residue was added 250 ml of 6 N aquous sulfuric acid solution. The mixture was refluxed for 90 minutes. The reaction mixture was extracted with diethyl ether. The organic layer was washed with water and a saturated aqueous sodium chloride solution, dried over anhydrous magnesium sulfate, and concentrated. The residue was purified by the use of a column to obtain 15.30 g of an intended product.
Melting point: 59-61 ° C

(2) Synthesis of 4-(4-methoxyphenyl)-5-(p-tolyl)-3H-1,2-dithiole-3-thione

8.00 g of 4-methoxy-α-methyl-4'-methylstilbene and 3.30 g of sulfur were reacted in the absence of any solvent at 240 ° C for 1 hour. Thereto was added ethyl acetate. The resulting precipitate (a solid) was collected by filtration, washed with ethyl acetate, dried, and recrystallized from benzene to obtain 3.20 g of an intended product.

1H NMR (CDCl3, δ)

2.33 (3H, s), 3.80 (3H, s), 6.87 (2H, d), 7.07 (2H, d), 7.10 (2H, d), 7.14 (2H, d)

(3) Synthesis of 4-(4-methoxy-3-sulfophenyl)-5-(p-tolyl)-3H-1,2-dithiole-3-thione sodium salt

A concentrated sulfuric acid (5 ml) solution containing 4-(4-methoxyphenyl)-5-(p-tolyl)-3H-1,2-dithiole-3-thione was stirred at 100 ° C for 5 minutes. The reacton mixture was poured into ice water. Thereto was added sodium carbonate to allow the mixture to have a pH of 10. Thereto was added methanol. The resulting precipitate (an inorganic salt) was removed by filtration. The filtrate was concentrated. The residue was washed with water to obtain 324 mg of an intended product.

1H NMR (DMSO-d6, δ)

2.30 (3H, s), 3.74 (3H, s), 6.92 (1H, d), 7.02 (1H, dd), 7.19 (2H, d), 7.23 (2H, d), 7.41 (1H, d)

Production Example 35 (reference example)

Production of 4-(4-methoxy-3-sulfophenyl)-5-phenyl-3H-1,2-dithiole-3-thione

The above compound was produced in the same manner as in Production Example 34.

1H NMR (CDCl3-CD30D, δ)

3.92 (3H, s), 6.97 (1H, d), 7.14 (1H, dd), 7.29-7.41 (5H, m), 7.62 (1H, d)

Production Example 36

Production of 4-(4-methoxy-3-sulfophenyl)-5-(o-tolyl)-3H-1,2-dithiole-3-thione sodium salt

The above compound was produced in the same manner as in Production Example 34.

1H NMR (DMSO-d6, δ)

2.05 (3H, s), 3.68 (3H, s), 6.79 (1H, d, J = 8.8Hz), 6.92 (1H, dd), 7.18-7.41 (4H, m), 7.53 (1H, d)

Production Example 37

Production of 4-(4-methoxy-3-sulfophenyl)-5-(m-tolyl)-3H-1,2-dithiole-3-thione sodium salt

The above compound was produced in the same manner as in Production Example 34.
1H NMR (DMSO-d6, δ)
2.28 (3H, s), 3.74 (3H, s), 6.90 (1H, d), 7.02 (1H, dd), 7.20-7.27 (4H, m), 7.43 (1H, d)

Production Example 38

Production of 5-(4-ethylphenyl)-4-(4-methoxy-3-sulfophenyl)-3H-1,2-dithiole-3-thione sodium salt

The above compound was produced in the same manner as in Production Example 34.
1H NMR (DMSO-d6, δ)
1.15 (3H, t), 3.77 (3H, s), 6.59 (2H, q), 6.94 (1H, d), 7.03 (1H, d), 7.20-7.27 (4H, m), 7.46 (1H, d)

Production Example 39

Production of 5-phenyl-4-(4-sulfobenzyl)-3H-1,2-dithiole-3-thione sodium salt

(1) Synthesis of 4-benzyl-5-phenyl-3H-1,2-dithiole-3-thione

A pyridine (50 ml) solution containing 12.00 g of ethyl 2-benzylbenzoylacetate was dropwise added to a pyridine (150 ml) suspension containing 10.00 g of phosphorus pentasulfide, being refluxed. The mixture was stirred at the same temperature for 4 hours. The reaction mixture was returned to room temperature and poured into water. The mixture was extracted with chloroform. The organic layer was dried over anhydrous magnesium sulfate and then concentrated. The residue was purified by the use of a column to obtain 5.28 g of an intended product.
1H NMR (CDCl3, δ)
4.06 (2H, s), 6.98-7.00 (2H, m), 7.14-7.21 (3H, m), 7.33-7.35 (2H, m), 7.42-7.51 (3H, m)

(2) Synthesis of 5-phenyl-4-(4-sulfobenzyl)-3H-1,2-dithiole-3-thione sodium salt

A concentrated sulfuric acid (10 ml) solution containing 1 g of 4-benzyl-5-phenyl-3H-1,2-dithiole-3-thione was stirred at 100°C for 10 minutes. The reaction mixture was poured into ice water. The resulting precipitate (a solid) was collected by filtration. The solid was dissolved in a small amount of water. Thereto was added sodium carbonate for neutralization. The resulting precipitate (a solid) was collected by filtration and washed with water and acetone to obtain 636 mg of an intended product.
1H NMR (DMSO-d6, δ)
3.99 (2H, s), 6.90 (2H, d), 7.44-7.56 (7H, m)

Proudction Example 40

Production of 4-(4-methoxy-3-sulfobenzyl)-5-phenyl-3H-1,2-dithiole-3-thione sodium salt

(1) Synthesis of ethyl 2-(4-methoxybenzyl)benzoylacetate

10.00 g of ethyl benzoylacetate and 7 ml of 4-methoxybenzyl chloride were added to a DMF (50 ml) solution containing 2.00 g of sodium hydride (about 60%, in oil), with ice-cooling. The mixture was stirred at room temperature for 3 hours. The reaction mixture was extracted with ethyl acetate. The extract was dried over anhydrous magnesium sulfate and then concentrated. The residue was purified by the use of a column to obtain 14.00 g of an intended product.
1H NMR (CDCl3, δ)
1.12 (3H, t), 3.25-3.28 (2H, m), 3.75 (3H, s) 4.06-4.13 (2H, m) 4.58 (1H, t), 6.79 (2H, d), 7.14 (2H, d), 7.42-7.46 (2H, m), 7.54-7.57 (1H, m), 7.94-7.96 (2H, m)

(2) Synthesis of 4-(4-methoxybenzyl)-5-phenyl-3H-1,2-dithiole-3-thione

14.00 g of ethyl 2-(4-methoxybenzyl)benzoylacetate was dropwise added to a pyridine (200 ml) suspension containing 14.00 g of phosphorus pentasulfide, being refluxed. The mixture was stirred at the same temperature for 6 hours. The reaction mixture was subjected to preliminary pyrification by the use of a column. The resulting oily substance was dissolved in ethyl acetate. The solution was washed with 2 N aqueous hydrochloric acid solution and water. The organic layer was dried over anhydrous magnesium sulfate and concentrated. The residue was purified by the use of a column to obtain 6.20 g of an intended product.

1H NMR (CDCl3, δ)

3.75 (3H, s), 4.00 (2H, s), 6.72 (2H, d), 6.91 (2H, d), 7.34-7.37 (2H, m), 7.43-7.53 (3H, m)

(3) Synthesis of 4-(4-methoxy-3-sulfobenzyl)-5-phenyl-3H-1,2-dithiole-3-thione sodium salt

A concentrated sulfuric acid (7 ml) solution containing 1 g of 4-methoxybenzyl-5-phenyl-3H-1,2-dithiole-3-thione was stirred at 100 °C for 10 minutes. The reaction mixture was poured into ice water. The resulting precipitate (an oily substance) was washed with a small amount of water and then dissolved in 40 ml of water. To the aqueous solution was added a saturated aqueous sodium carbonate solution for neutralization. The resulting precipitate (a solid) was collected by filtration and washed with water and acetone to obtain 360 mg of an intended product.

1H NMR (DMSO-d6, δ)

3.70 (3H, s), 3.90 (2H, s), 6.78 (1H, d), 6.81 (1H, dd), 7.41 (1H, d), 7.49-7.60 (5H, m)

Production Example 41

Production of 4-(4-methyl-3-sulfobenzyl)-5-phenyl-3H-1,2-dithiole-3-thione sodium salt

The above compound was produced in the same manner as in Production Example 40.

1H NMR (DMSO-d6, δ)

2.44 (3H, s), 3.93 (2H, s), 6.71 (1H, dd), 6.93 (1H, d), 7.47 (1H, d), 7.48-7.58 (5H, m)

Production Example 42

Production of 4-(4-sulfobenzyl)-5-(o-tolyl)-3H-1,2-dithiole-3-thione sodium salt

The above compound was produced in the same manner as in Production Example 40.

1H NMR (DMSO-d6, δ)

2.00 (3H, s), 3.75 (2H, s), 6.78 (2H, d), 7.26-7.49 (6H, m)

Production Example 43

Production of 4-(4-sulfobenzyl)-5-(m-tolyl)-3H-1,2-dithiole-3-thione sodium salt

The above compound was produced in the same manner as in Production Example 40.

1H NMR (DMSO-d6, δ)

2.33 (3H, s), 3.98 (2H, s), 6.89 (2H, d), 7.25-7.30 (2H, m), 7.40-7.45 (2H, m), 7.44 (2H, d)

Production Example 44

Production of 4-(4-sulfobenzyl)-5-(p-tolyl)-3H-1,2-dithiole-3-thione sodium salt

The above compound was produced in the same manner as in Production Example 40.

1H NMR (DMSO-d6, δ)

2.36 (3H, s), 3.99 (2H, s), 6.90 (2H, d), 7.34 (2H, d), 7.39 (2H, d), 7.44 (2H, d)

Production Example 45

Production of 5-(3,4-dimethylphenyl)-4-(4-sulfobenzyl)-3H-1,2-dithiole-3-thione sodium salt

The above compound was produced in the same manner as in Production Example 40.
1H NMR (DMSO-d6, δ)
2.23 (3H, s), 2.27 (3H, s), 4.00 (2H, s), 6.93 (2H, d), 7.17-7.18 (1H, m), 7.21-7.31 (2H, m), 7.45 (2H, d)

Production Example 46

Production of 5-(4-ethylphenyl)-4-(4-sulfobenzyl)-3H-1,2-dithiole-3-thione sodium salt

The above compound was produced in the same manner as in Production Example 40.
1H NMR (DMSO-d6, δ)
1.19 (3H, t), 2.65 (2H, q), 3.99 (2H, s), 6.90 (2H, d), 7.38 (2H, d), 7.43 (2H, d), 7.44 (2H, d)

Production Example 47

Production of 5-phenyl-4-[2-(4-sulfophenyl)ethyl]-3H-1,2-dithiole-3-thione sodium salt

The above compound was produced in the same manner as in Production Example 40.
1H NMR (DMSO-d6, δ)
2.71-2.78 (4H, m) 6.89 (2H, d), 7.34-7.38 (2H, m), 7.45 (2H, d), 7.51-7.59 (3H, m)

Production Example 48

Production of 4-[2-(4-sulfophenyl)ethyl]-5-(p-tolyl)-3H-1,2-dithiole-3-thione sodium salt

The above compound was produced in the same manner as in Production Example 40.
1H NMR (DMSO-d6, δ)
2.39 (3H, s), 2.77 (4H, s), 6.93 (2H, d), 7.28 (2H, d), 7.36 (2H, d), 7.47 (2H, d)

Production Example 49

Production of 4-[3-(4-sulfophenyl)propyl]-5-phenyl-3H-1,2-dithiole-3-thione sodium salt

The above compound was produced in the same manner as in Production Example 40.
1H NMR (DMSO-d6, δ)
1.67-1.78 (2H, m), 2.45-2.59 (4H, m), 6.97 (2H, d), 7.45-7.61 (7H, m)

Production Example 50

Production of 4-[5-(4-sulfophenyl)pentyl]-5-phenyl-3H-1,2-dithiole-3-thione sodium salt

The above compound was produced in the same manner as in Production Example 40.
1H NMR (DMSO-d6, δ)
1.09-1.18 (2H, m), 1.35-1.45 (4H, m), 2.22-2.58 (4H, m), 7.03 (2H, d, J = 8.1Hz), 7.49 (2H, d), 7.55-7.61 (5H, m)

Production Example 51

Production of 5-phenyl-4-(3-methoxy-6-sulfobenzyl)-3H-1,2-dithiole-3-thione sodium salt

The above compound was produced in the same manner as in Production Example 40.
1H NMR (DMSO-d6, δ)
3.67 (3H, s), 4.49 (2H, s), 6.12 (1H, d), 6.68 (1H, dd), 7.42-7.56 (5H, m), 7.71 (1H, d)

Production Example 52

Production of 4-(3-methoxy-6-sulfobenzyl)-5-(p-tolyl)-3H-1,2-dithiole-3-thione sodium salt

The above compound was produced in the same manner as in Production Example 40.
1H NMR (DMSO-d6, δ)
2.32 (3H, s), 3.66 (3H, s), 4.50 (2H, s), 6.11 (1H, d), 6.69 (1H, dd), 7.25 (2H, d), 7.32 (2H, d), 7.73 (1H, d)

Production Example 53

Production of 5-phenyl-4-(2-methoxy-5-sulfobenzyl)-3H-1,2-dithiole-3-thione sodium salt

The above compound was produced in the same manner as in Production Example 40.
1H NMR (DMSO-d6, δ)
3.70 (3H, s), 3.84 (2H, s), 6.84 (1H, d), 7.06 (1H, d), 7.39-7.57 (6H, m)

Evaluation Example 1

Inhibitory action for formation of peroxylipid

Method

Male SD rats of 7-10 week age were anesthetized with pentobarbital and then subjected to dehematization. Thereafter, their brains were enucleated. The brain tissues were homogenized with a phosphate buffer solution with ice-cooling. Each of the 5% homogenates was incubated at 37 °C for 60 minutes to form malondialdehyde (MDA). The amount of MDA was determined by measuring the absorbance at 535 nm according to the thiobarbituric acid method. Using this MDA amount (a) and a control value (b) (a MDA amount when no test compound was added), the inhibition (%) for formation of peroxylipid, of test compound was calculated from the following formula.

Inhibition (%) for formation of peroxylipid = [(b-a)/b]x100

Each test compound was added to the homogenate before the incubation.

Result

The inhibitory action for formation of peroxylipid, of each test compound in rat brain homogenate is shown in Table 13. In Table 13, each test compound shows a high inhibition for formation of peroxylipid.

Table 13

| Inhibitory action for formation of peroxylipid, of each test compound in rat brain homogenate | |
|---|---|
| Test compound | Inhibition (%) |
| Production Example 3 | 100 |
| Production Example 4 | 100 |
| Production Example 5 | 100 |
| Production Example 12 | 100 |
| Production Example 13 | 100 |

Incidentally, the concentration of each test compound was $10^{-4}$ M, and the number of rats used for each test compound was n = 2.

Evaluation Example 2

Inhibitiory action for reperfusion arrhythmia

Method

Male SD rats of 8-12 week age were anesthetized with pentobarbital and subjected to thoracotomy under artificial respiration. The left anterior descending coronary artery of each rat was ligated for 5 minutes and then subjected to reperfusion for 10 minutes. An electrocardiograph in standard bipolar limb lead II was recorded for each rat to examine the occurrence of ventricular arrhythmia. A test compound was intravenously administered 5 minutes before the obstruction of coronary artery. For comparison, the same evaluation was conducted also on the 5-(4-methoxy-3-sulfophenyl)-3H-1,2-dithiole-3-thione sodium salt described in Eur. J. Med. Chem. 15, 405 (1980).

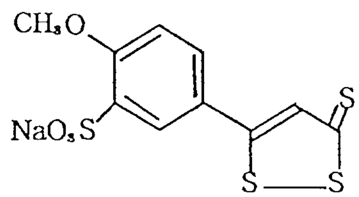

Result

Arrhythmia such as ventricular extrasystole, ventricular tachicardia (VT), ventricular fibrillation (VF) and the like appear after the ligation of left anterior descending coronary artery and subsequent reperfusion. In Table 14 are shown the ventricular fibrillation incidences and mortalities of control group and test compound-administered group. The 5-(4-methoxy-3-sulfophenyl)-3H-1,2-dithiole-3-thione sodium salt described in the above literature shows no significant reduction for ventricular fibrillation incidence or mortality, while the compound of the present invention shows significant reductions for both ventricular fibrillation incidence and mortality.

Table 14

| Effect on ventricular arrhythmia in obstruction of rat coronary artery and subsequent reperfusion | | | |
|---|---|---|---|
| Test compound | Amount used mg/kg) | VF incidence (VF rats)/(tested rats) (%) | Mortality (%) |
| Control | | 5/6 (83.3) | 4/6 (66.7) |
| Production Example 4 | 1.0 | 2/5 (40.0) | 2/5 (40.0) |
| Compound in literature | 3.0 | 6/7 (85.7) | 6/7 (85.7) |

Evaluation Example 3

Inhibitory action for disappearance of enzyme in myocardial cells after ischemia and reperfusion

Method

Male SD rats of 7-12 week age were anesthetized with pentobarbital and subjected to thoracotomy under artificial respiration. The left anterior descending coronary artery of each rat was ligated for 20 minutes and then subjected to reperfusion for 2 hours. Then, the heart of each rat was enucleated and the left ventricle was separated. The left ventricle was homogenized to measure the activity of creatine phosphokinase (CPK) remaining in the ventricle. A test compound was intravenously administered 5

minutes before the obstruction of coronary artery. In Table 15 are shown the differences in CPK activity between non-ischemia territory and ischemia and reperfusion territory of left ventricle, of test compound and control.

Result

As seen in Table 15, the test compound of the present invention inhibits the disapperance of creatine phosphokinase from myocardial cells, caused by ischemia and reperfusion.

Table 15

| Test Compound | Amount used (mg/kg) | CPK activity (U/mg protein) (Non-ischemia territory)-(ischemia and reperfusion territory) |
|---|---|---|
| Control | | 2.72 ± 0.63 (n = 8) |
| Production Example 4 | 1.0 | 1.36 ± 0.34 (n = 7) |

In Table 15, CPK activity indicates (average value ± standard error), and n indicates the number of tested rats.

Evaluation Example 4

Inhibitory action for formation of peroxylipid

Method

Male SD rats of 7-10 week age were anesthetized with pentobarbital and then subjected to dehematization. Thereafter, their brains were enucleated. The brain tissues were homogenized with a phosphate buffer solution with ice-cooling. Each of the 5% homogenates was incubated at 37 °C for 60 minutes to form malondialdehyde (MDA). The amount of MDA was determined by measuring the absorbance at 535 nm according to the thiobarbituric acid method. Using this MDA amount (a) and a control value (b) (a MDA amount when no test compound was added), the inhibition (%) for formation of peroxylipid, of test compound was calculated from the following formula.

Inhibition (%) for formation of peroxylipid = [(b-a)/b]x100

Each test compound was added to the homogenate before the incubation.

Result

The inhibitory action for formation of peroxylipid, of each test compound in rat brain homogenate is shown in Table 16. In Table 16, each test compound shows a high inhibition for formation of peroxylipid.

Table 16

| Inhibitory action for formation of peroxylipid, of each test compound in rat brain homogenate | |
|---|---|
| Test compound | Inhibition (%) |
| Production Example 26 | 100 |
| Production Example 34 | 100 |
| Production Example 39 | 100 |

Incidentally, the concentration of each test compound was $10^{-4}$ M, and the number of rats used for each test compound was n = 2.

Evaluation Example 5

Inhibitiory action of reperfusion arrhythmia

Method

Male SD rats of 8-12 week age were anesthetized with pentobarbital and subjected to thoracotomy under artificial respiration. The left anterior descending coronary artery of each rat was ligated for 5 minutes and then subjected to reperfusion for 10 minutes. An electrocardiograph in standard bipolar limb lead II was recorded for each rat to examine the occurrence of ventricular arrhythmia. Each test compound was intravenously administered 5 minutes before the obstruction of coronary artery. For comparison, the same evaluation was conducted also on the 5-(4-methoxy-3-sulfophenyl)-3H-1,2-dithiole-3-thione sodium salt described in Eur. J. Med. Chem. 15, 405 (1980).

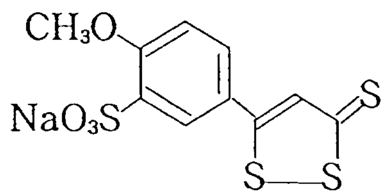

(32)

Result

Arrhythmia such as ventricular extrasystole, ventricular tachicardia (VT), ventricular fibrillation (VF) and the like appear after the ligation of left anterior descending coronary artery and subsequent reperfusion. In Table 17 are shown the ventricular fibrillation incidences and mortalities of control group and test compound-administered groups. The 5-(4-methoxy-3-sulfophenyl)-3H-1,2-dithiole-3-thione sodium salt described in the above literature shows no significant reduction for ventricular fibrillation incidence or mortality, while the tested compounds of the present invention show significant reductions for both ventricular fibrillation incidence and mortality.

Table 17

| Effect on ventricular arrhythmia in obstruction of rat coronary artery and subsequent reperfusion | | | |
|---|---|---|---|
| Test compound | Amount used mg/kg) | VF incidence (VF rats)/(tested rats) (%) | Mortality (%) |
| Control | | 63/93 (67.7) | 57/93 (61.3) |
| Production Example 26 | 1.0 | 3/6 (50.0) | 2/6 (33.3) |
| Production Example 34 | 1.0 | 3/7 (42.9) | 2/7 (28.6) |
| Production Example 39 | 1.0 | 1/4 (25.0) | 0/4 (0.0) |
| Compound in literature | 3.0 | 6/7 (85.7) | 6/7 (85.7) |

Evaluation Example 6

Inhibitory action for disappearance of enzyme in myocardial cells after ischemia and reperfusion

Method

Male SD rats of 7-12 week age were anesthetized with pentobarbital and subjected to thoracotomy under artificial respiration. The left anterior descending coronary artery of each rat was ligated for 20 minutes and then subjected to reperfusion for 2 hours. Then, the heart of each rat was enucleated and the left ventricle was separated. The left ventricle was homogenized to measure the activity of creatine phosphokinase (CPK) remaining in the ventricle. Each test compound was intravenously administered 5

minutes before the obstruction of coronary artery. For comparison, the same evaluation was conducted also on the 4-(4-methoxy-3-sulfophenyl)-5-phenyl-3H-1,2-dithiole-3-thione sodium salt described in Bull. Soc. Chim. Fr., 84 (1955). In Table 18 are shown the differences in CPK activity between non-ischemia territory and ischemia and reperfusion territory of left ventricle, of test compounds and control.

Result

As seen in Table 18, all of the test compounds of the present invention inhibit the disapperance of creatine phosphokinase from myocardial cells, caused by ischemia and reperfusion, and reduce the rat mortality. The 4-(4-methoxy-3-sulfophenyl)-5-phenyl-3H-1,2-dithiole-3-thione sodium salt described in the above literature shows no significant effect.

Table 18

| Test Compound | Amount used (mg/kg) | CPK activity (U/mg protein) (Non-ischemia territory)-(ischemia and-reperfusion territory) | Mortality (%) |
|---|---|---|---|
| Control | | 1.43 ± 0.18 (n = 34) | 54.7 |
| Production Example 26 | 1.0 | 0.70 ± 0.23 (n = 9) | 33.3 |
| Production Example 34 | 1.0 | 0.81 ± 0.43 (n = 10) | 40.0 |
| Production Example 39 | 1.0 | 0.84 ± 0.18 (n = 8) | 12.5 |
| Compound in literature | 1.0 | 1.66 ± 0.23 (n = 4) | 50.0 |

In Table 18, CPK activity indicates (average value ± standard error), and n indicates the number of tested rats.

Test Example 1 Solubility in water

Method

Solubility in 5% aqueous glucose solution was measured.

Result

The results are shown in Table 19. In Table 19, all of the test compounds of the present invention show a high water solubility.

Table 19

| Test compound | Solubility |
|---|---|
| Production Example 3 | 10 mg/ml or more |
| Production Example 4 | 10 mg/ml or more |
| Production Example 5 | 10 mg/ml or more |
| Production Example 12 | 10 mg/ml or more |
| Production Example 13 | 10 mg/ml or more |

Test Example 2 Solubility in water

Method

Solubility in 5% aqueous glucose solution was measured.

Result

The results are shown in Table 20. In Table 20, all of the test compounds of the present invention show a high water solubility.

Table 20

| Test compound | Solubility |
|---|---|
| Production Example 26 | 10 mg/ml or more |
| Production Example 34 | 10 mg/ml or more |

The compound represented by general formula (1) or (11), or salt thereof according to the present invention is highly soluble in water and has an inhibitory action for formation of peroxylipid, and accordingly is useful as a therapeutic or preventive agent for diseases associated with peroxylipid. The present invention also provides a useful process for producing said compound or salt.

**Claims**

**1.** A compound represented by general formula (1)

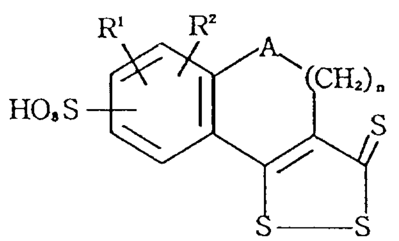

(1)

(wherein A is a methylene group or an oxygen atom; $R^1$ and $R^2$ are independently a hydrogen atom, a hydroxyl group, a halogen atom, a lower alkyl group or a lower alkoxy group; and n is an integer of 0-3 when A is a methylene group, and an integer of 1-3 when A is an oxygen atom); or a salt thereof.

**2.** A compound of general formula (1) according to Claim 1 wherein A is a methylene group and $R^2$ is a hydrogen atom; or a salt thereof.

**3.** A compound of general formula (1) according to Claim 2 wherein $R^1$ is a hydrogen atom, a hydroxyl group or a lower alkoxy group; or a salt thereof.

**4.** A compound of general formula (1) according to Claim 3 wherein $R^1$ is a lower alkoxy group; or a salt thereof.

**5.** A compound of general formula (1) according to Claim 1 wherein A is an oxygen atom and $R^2$ is a hydrogen atom; or a salt thereof.

**6.** A compound of general formula (1) according to Claim 5 wherein $R^1$ is a hydrogen atom, a hydroxyl group or a lower alkoxy group; or a salt thereof.

**7.** A compound of general formula (1) according to Claim 1, which is any of the following compounds (a) to (k); or a salt thereof.

(a) 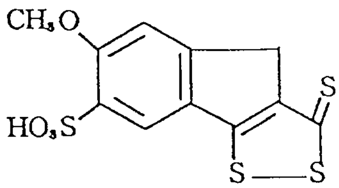

(b) 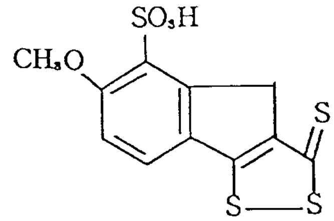

(c) 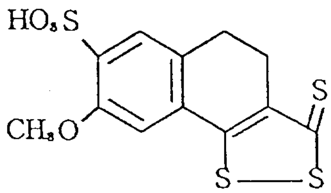

(d) 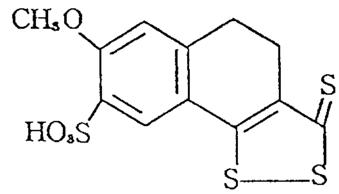

(e) 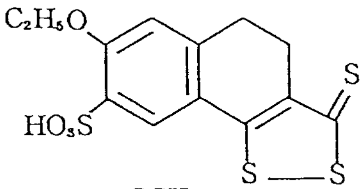

(f) 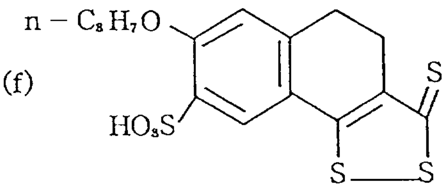

(g) 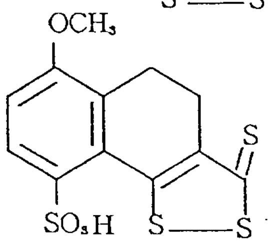

(h) 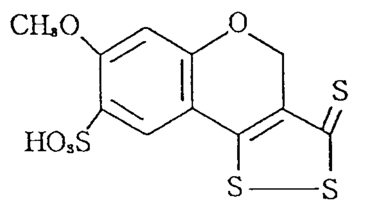

(i) 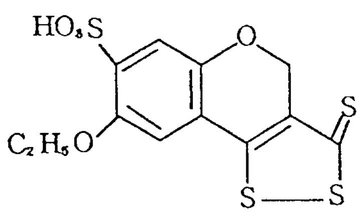

(j) 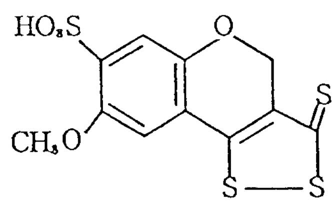

(k) 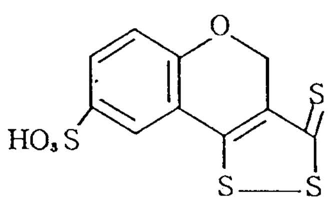

**8.** A process for producing a compound represented by general formula (1)

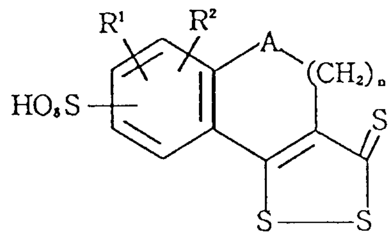

(1)

(wherein A is a methylene group or an oxygen atom; $R^1$ and $R^2$ are independently a hydrogen atom, a hydroxyl group, a halogen atom, a lower alkyl group or a lower alkoxy group; and n is an integer of 0-3 when A is a methylene group, and an integer of 1-3 when A is an oxygen atom), which process comprises treating a compound represented by general formula (2)

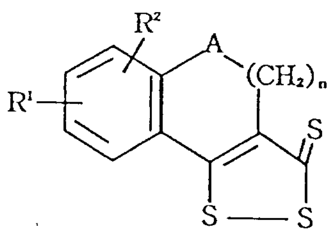

(2)

(wherein A, $R^1$, $R^2$ and n are the same as defined above) with a sulfonating agent.

**9.** A compound represented by general formula (11)

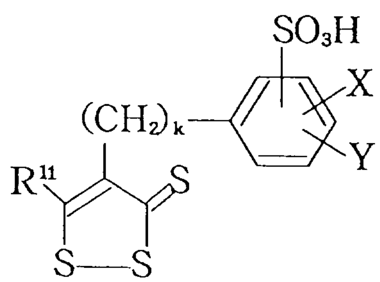

(11)

[wherein k is an integer of 0-5; X and Y are independently a hydrogen atom, a lower alkyl group or a lower alkoxy group; $R^{11}$ is an alkyl group or a group represented by general formula (12)

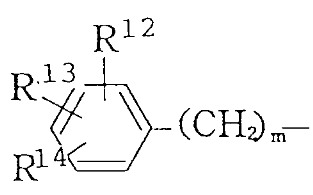

(12)

(wherein m is an integer of 0-4; and $R^{12}$, $R^{13}$ and $R^{14}$ are independently a hydrogen atom, a lower alkyl group or a lower alkoxy group; however, a case is excluded in which both k and m are zero, the sulfo group bonds to the 3-position, X is a 4-methoxy group, and $R^{12}$, $R^{13}$, $R^{14}$ and Y are each a hydrogen atom)]; or a salt thereof.

**10.** A compound of general formula (11) according to Claim 9 wherein $R^{11}$ is an alkyl group; or a salt thereof.

**11.** A compound of general formula (11) according to Claim 9, wherein $R^{11}$ is a group represented by general formula (12)

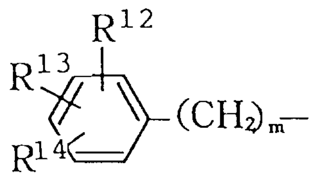

(12)

(wherein m, $R^{12}$, $R^{13}$ and $R^{14}$ are the same as defined above; however, a case is excluded in which both k and m are zero, the sulfo group bonds to the 3-position, X is a 4-methoxy group, and $R^{12}$, $R^{13}$, $R^{14}$ and Y are each a hydrogen atom); or a salt thereof.

**12.** A compound of general formula (11) according to Claim 11, wherein both k and m are zero but a case is excluded in which the sulfo group bonds to the 3-position, X is a 4-methoxy group, and $R^{12}$, $R^{13}$, $R^{14}$ and Y are each a hydrogen atom; or a salt thereof.

**13.** A compound of general formula (11) according to Claim 9, which is any of the following compounds; or a salt thereof.
5-Hexyl-4-(4-methoxy-3-sulfobenzyl)-3H-1,2-dithiole-3-thione
4-(4-Methoxy-3-sulfophenyl)-5-(p-tolyl)-3H-1,2-dithiole-3-thione

**14.** A therapeutic or preventive agent for diseases associated with peroxylipid, which contains, as the active ingredient, a compound or a salt thereof selected from those set forth in Claims 1-7 and Claims 9-13.

European Patent Office

# EUROPEAN SEARCH REPORT

Application Number
EP 94 11 3804

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | CHEMICAL ABSTRACTS, vol. 54, no. 22, 1960, Columbus, Ohio, US; abstract no. 24660b,, U.SCHMIDT ET AL. 'TRITHIONES.XII.' * abstract * | 1 | C07D339/04<br>C07D495/04<br>A61K31/385 |
| A | & ANN., vol.631, 1960, WEINHEIM pages 129 - 138 | 1 | |
| A | BE-A-642 497 (K/SERHO) * page 1 - page 3; claims 4,18 * | 9-11 | |
| D,P,A | CHEMICAL ABSTRACTS, vol. 120, no. 17, 1994, Columbus, Ohio, US; abstract no. 217648y, page 1079 ; * abstract * | 1,9-14 | |
| D,P,A | & JP-A-05 301 868 (MITSUI TOATSU) | 1,9-14 | |
| | | | TECHNICAL FIELDS SEARCHED (Int.Cl.6) |
| | | | C07D |

The present search report has been drawn up for all claims

| Place of search | Date of completion of the search | Examiner |
|---|---|---|
| THE HAGUE | 24 November 1994 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)